# EUROPEAN PATENT APPLICATION

(11) **EP 4 529 888 A2**
(43) Date of publication of application: **02.04.2025**
(21) Application number: 24223793.1
(22) Date of filing: 16.09.2022
(51) Int. Cl.: A61F 2/14

(54) **INTRACORNEAL OPTICAL IMPLANT**

(30) Priority: 17.09.2021 US 202163245384 P; 13.06.2022 US 202263351480 P
(62) Divisional of application: 22783489.2
(71) Applicant: INTRA-KER S.r.l., 47122 Forli FC (IT)
(72) Inventor: Busin, Massimo, 47122 Forlì (IT); ELIACHAR, Eliahu, 3437113 Haifa (IL); YU, Angeli Christy, 6000 Banilad Cebu City (PH); LILACH, Nir, 3658200 Kfar Yehoshua (IL); LAVAN, Ron Michael, 3432520 Haifa (IL)
(74) Representative: Papa, Elisabetta

(57) **Abstract**

A device (120) comprising a corneal implant for implantation within the cornea, the implant defining a planar body comprising: (i) an inner platform portion (222) located about an axis of circular symmetry of the implant, (ii) a peripheral portion comprising one or more angularly-spaced arms (204) that extend laterally from a peripheral edge of the inner platform, and (iii) a peripheral skirt portion which defines an outer circular perimeter of the implant, wherein the one or more angularly-spaced arms connect between the inner platform and the peripheral skirt portion; a wireless communication circuit configured for receiving an image data signal from an extraocular unit; an image projection controller configured to process the received image data signal to generate an image; and an image generator mounted to the inner platform portion, configured to project an image projection light beam, based on the generated image, in a posterior direction onto the retina of the eye.

## Description

### CROSS-REFERENCE TO RELATED APPLICATIONS

This application claims the benefit of priority from U.S. Provisional Patent Application No. 63/245,384, filed September 17, 2021, entitled, "INTRACORNEAL IMPLANT FOR PROJECTING IMAGES ONTO THE RETINA," and from U.S. Provisional Patent Application No. 63/351,480, filed June 13, 2022, entitled "INTRACORNEAL OPTICAL IMPLANT," the contents of both of which are hereby incorporated herein by reference in their entirety.

### FIELD OF THE INVENTION

The invention relates to the field of ocular devices and methods.

### BACKGROUND

The cornea is the transparent anterior portion of the eyeball that covers the iris, pupil, and anterior chamber. A healthy cornea acts as a transparent window that allows entry of light, which passes through the crystalline lens, and allows formation of images onto the retina.

Opacities of the cornea can lead to irreversible loss of its transparency, which may result in severe visual impairment. Corneal opacities can occur due to scarring from various causes such as mechanical trauma, chemical injuries, infections, and several other diseases of the cornea and the ocular surface.

Importantly, corneal opacities may cause blindness in an otherwise healthy eye. Despite the presence of complete corneal opacification, eyes with corneal blindness can retain an intact intraocular visual pathway through a functional retina and optic nerve. Even with severe visual impairment secondary to corneal disease, patients may still have significant potential visual acuity.

Corneal transplantation is traditionally performed to replace an irreversibly opaque cornea with a corneal allograft. However, a considerable number of cases are deemed ineligible for corneal transplantation due to the presence of high-risk features for allograft rejection and graft failure. While alternative surgical treatments such as artificial corneal implants or keratoprosthesis have been developed to obviate the risk of allograft rejection, such procedures can be associated with several complications including device extrusion, retroprosthetic membrane formation, retinal detachment and endophthalmitis. In patients with substantial corneal damage secondary to ocular trauma or burns, ocular infections, cicatricial keratoconjunctivitis, multiple failed corneal grafts or immunologic and ocular surface diseases, there is still no safe and effective surgical treatment for longterm visual rehabilitation.

The foregoing examples of the related art and limitations related therewith are intended to be illustrative and not exclusive. Other limitations of the related art will become apparent to those of skill in the art upon a reading of the specification and a study of the figures.

### SUMMARY

The following embodiments and aspects thereof are described and illustrated in conjunction with systems, tools and methods which are meant to be exemplary and illustrative, not limiting in scope.

There is provided, in an embodiment, a device comprising: an intracorneal implant dimensioned and adapted for implantation within the cornea of an eye, the intracorneal implant defining a planar body having a convexity which substantially conforms to the convexity of the cornea, the intracorneal implant comprising: (i) a central optical part comprising an optical stem having an anterior end and extending axially through the cornea to a posterior end, and (ii) a peripheral haptic part comprising two or more angularly-spaced haptics that extend laterally from the central optical part, wherein, when the intracorneal implant is implanted within the cornea, the anterior end is located substantially at the anterior surface of the eye, and the posterior end abuts or is substantially in contact engagement with a thin layer of corneal tissue located posteriorly to the posterior end.

There is also provided, in an embodiment, a method comprising: providing an intracorneal implant dimensioned and adapted for implantation within the cornea of an eye, the intracorneal implant defining a planar body having a convexity which substantially conforms to the convexity of the cornea, the intracorneal implant comprising: (i) a central optical part comprising an optical stem having an anterior end and extending axially through the cornea to a posterior end, and (ii) a peripheral haptic part comprising two or more angularly-spaced haptics that extend laterally from the central optical part; and implanting the intracorneal implant within the cornea, such that the anterior end is located substantially at the anterior surface of the eye, and the posterior end is in contact engagement with a thin layer of corneal tissue located posteriorly to the posterior end.

In some embodiments, the optical stem is configured to project an image in a posterior direction, through the thin layer of corneal tissue, and onto the retina of the eye.

In some embodiments, the anterior end is a light-receiving end, wherein the optical stem defines a light-transparent pathway oriented for transmitting light received at the anterior end from an extraocular light source through the optical stem and through the thin layer of corneal tissue, onto the retina of the eye.

In some embodiments, the optical stem includes an image generator configured to receive an image data signal from an extraocular source, and to project an image in a posterior direction, through the thin layer of corneal tissue, onto the retina of the eye.

In some embodiments, the method further comprises receiving, by the image generator, the image data signal from the extraocular source, and projecting the image in the posterior direction, through the thin layer of corneal tissue, and onto the retina of the eye.

In some embodiments, the optical stem comprises at least one optical lens.

In some embodiments, the thin layer of corneal tissue is substantially transparent.

In some embodiments, the thin layer of corneal tissue has a thickness of approximately 10-50µm.

In some embodiments, the thin layer of corneal tissue comprises, in at least a central portion thereof, the pre-Descemet layer and the Descemet membrane of the cornea.

In some embodiments, the thin layer of corneal tissue further comprises, in at least the central portion thereof, the Descemet membrane of the cornea.

In some embodiments, the thin layer of corneal tissue further comprises, in at least the central portion thereof, the endothelial layer of the cornea.

In some embodiments, the thin layer of corneal tissue is part of a corneal tissue scaffold configured for surgical implantation in the eye within a through-recess formed by a full-thickness corneal trephination in the eye.

In some embodiments, the thin layer of corneal tissue forms a central, substantially transparent, region of the corneal tissue scaffold.

In some embodiments, the corneal tissue scaffold is preprocessed using at least one of the following techniques: dehydration, lyophilization, plasticization, gelling, dehydrothermal treatment, mechanical processing, compressing, and sterilization.

In some embodiments, the corneal tissue scaffold is preprocessed using at least one of the following techniques: dehydration, lyophilization, plasticization, gelling, dehydrothermal treatment, mechanical processing, compressing, decontamination, and sterilization.

In some embodiments, each of the haptics comprises at least one opening configured to facilitate fixation and adaptation of the device or the intracorneal implant with the corneal tissue through the at least one opening, when the device is implanted within the cornea.

In some embodiments, when the intracorneal implant is implanted within the cornea, at least a portion of the anterior end is exposed through the anterior surface of the eye.

In some embodiments, the anterior end is configured for mounting at least one external optical element.

In some embodiments, the at least one external optical element is removably mounted to the anterior end.

There is further provided, in an embodiment, an implantable corneal tissue scaffold device, comprising: a central portion defining a recess having a posterior end comprising a thin layer of corneal tissue, wherein the thin layer of corneal tissue comprises at least the pre-Descemet layer and the Descemet membrane, or at least the pre-Descemet layer; and a peripheral portion of corneal tissue which surrounds the recess, comprising one or more of: posterior layers of the stroma of the cornea, the pre-Descemet layer, the Descemet membrane, and the endothelial layer, wherein the implantable corneal tissue scaffold is prepared by: (i) performing, in a cornea, a deep lamellar dissection which separates between the deep posterior stroma of the cornea and the pre-Descemet layer or between the pre-Descemet layer and the Descemet membrane; (ii) performing, in the cornea, a partial trephination and removal of anterior layers of the stroma of the cornea; (iii) removing a central portion of deep posterior stroma of the cornea to create the recess, and (iv) performing a full-thickness trephination to remove the entire implantable corneal tissue scaffold device comprising the central portion and the peripheral portion, from the cornea.

There is further provided, in an embodiment, a method comprising: preparing an implantable corneal tissue scaffold comprising a central portion defining a recess having a posterior end comprising a thin layer of corneal tissue, wherein the thin layer of corneal tissue comprises at least the pre-Descemet layer and the Descemet membrane, or at least the pre-Descemet layer, and a peripheral portion of corneal tissue which surrounds the recess, comprising one or more of: posterior layers of the stroma of the cornea, the pre-Descemet layer, the Descemet membrane, and the endothelial layer, by: (i) performing, in a cornea, a deep lamellar dissection which separates between the deep posterior stroma of the cornea and the pre-Descemet layer or between the pre-Descemet layer and the Descemet membrane; (ii) performing, in the cornea, a partial trephination and removal of anterior layers of the stroma of the cornea; (iii) removing a central portion of deep posterior stroma of the cornea to create the recess, and (iv) performing a full-thickness trephination to remove the entire implantable corneal tissue scaffold device comprising the central portion and the peripheral portion, from the cornea.

In some embodiments, the corneal tissue scaffold device has a diameter of 5- 11mm.

In some embodiments, the thin layer of corneal tissue has a thickness of between 10-50µm, wherein the peripheral portion has a thickness of between 100-500µm.

In some embodiments, the thin layer of corneal tissue is substantially transparent.

In some embodiments, the cornea is a donor cornea.

In some embodiments, the recess has a diameter of between 3-7mm.

In some embodiments, the deep lamellar dissection is performed using one or more of: pneumatic dissection, hydro-assisted dissection, viscoelastic-assisted dissection, and manual dissection.

In some embodiments, the removed anterior layers of the stroma of the cornea have a thickness of between 100-400 µm.

In some embodiments, the corneal tissue scaffold device is configured for implanting in an eye within a through-recess formed in the eye by a full-thickness trephination.

In some embodiments, the method further comprises implanting the corneal tissue scaffold device in an eye within the through-recess formed in the eye by the full-thickness trephination.

In some embodiments, the corneal tissue scaffold device is preprocessed using at least one of the following techniques: dehydration, lyophilization, plasticization, gelling, dehydrothermal treatment, mechanical processing, compressing, decontamination, and sterilization.

In some embodiments, the method further comprises preprocessing the corneal tissue scaffold device using at least one of the following techniques: dehydration, lyophilization, plasticization, gelling, dehydrothermal treatment, mechanical processing, compressing, decontamination, and sterilization.

There is further provided, in an embodiment, a system comprising: an implantable corneal tissue scaffold comprising a central portion defining a recess having a posterior end comprising a thin layer of corneal tissue, wherein the thin layer of corneal tissue comprises at least the pre-Descemet layer and the Descemet membrane, or at least the pre-Descemet layer, and a peripheral portion of corneal tissue which surrounds the recess, comprising one or more of: posterior layers of the stroma of the cornea, the pre-Descemet layer, the Descemet membrane, and the endothelial layer; and an intracorneal implant defining a planar body having a convexity which substantially conforms to the convexity of the cornea, the intracorneal implant comprising: (i) a central optical part comprising an optical stem having an anterior end and extending axially through the cornea to a posterior end, and (ii) a peripheral haptic part comprising two or more angularly-spaced haptics that extend laterally from the central optical part, wherein the intracorneal implant is mounted to the implantable corneal tissue scaffold device such that the posterior end abuts or is in contact engagement with the thin layer of corneal tissue.

In some embodiments, the system further comprises an anterior corneal lamella layer sutured in place over the intracorneal implant.

In some embodiments, the system is intended for surgical implantation within a through-recess formed by a full-thickness corneal trephination in an eye.

There is further provided, in an embodiment, a device comprising: a corneal implant dimensioned and adapted for implantation within the cornea of an eye, the implant defining a planar body comprising: (i) an inner platform portion located about an axis of circular symmetry of the implant, and (ii) a peripheral portion comprising one or more angularly-spaced arms that extend laterally from a peripheral edge of the inner platform; a wireless communication circuit configured for receiving an image data signal from an extraocular unit; an image projection controller configured to process the received image data signal to generate an image; and an image generator mounted to the inner platform portion, configured to project an image projection light beam, based on the generated image, in a posterior direction onto the retina of the eye.

There is further provided, in an embodiment, a method comprising: providing a device comprising a corneal implant dimensioned and adapted for implantation within the cornea of an eye, the implant defining a planar body comprising: (i) an inner platform portion located about an axis of circular symmetry of the implant, and (ii) a peripheral portion comprising one or more angularly-spaced arms that extend laterally from a peripheral edge of the inner platform; a wireless communication circuit configured for receiving an image data signal from an extraocular unit; an image projection controller configured to process the received image data signal to generate an image; and an image generator mounted to the inner platform portion, configured to project an image projection light beam, based on the generated image, in a posterior direction onto the retina of the eye; implanting the device in the cornea of an eye, such that transparent corneal tissue or no corneal tissue is present posterior to the image generator of the device; receiving, by the wireless communication circuit, the image data signal; processing, by the image projection controller, the received image data signal, to generate the image; and projecting, by the image generator, the image in a posterior direction onto the retina of the eye.

In some embodiments, the device further comprises a peripheral skirt portion which defines an outer circular perimeter of the implant, wherein the one or more angularly- spaced arms connect between the inner platform and the peripheral skirt portion.

In some embodiments, the wireless communication circuit comprises a reception inductive coil.

In some embodiments, the reception inductive coil is further configured for receiving power inductively from the extraocular unit.

In some embodiments, the wireless communication circuit comprises a photovoltaic cell configured to receive a light beam encoding the image data signal.

In some embodiments, the photovoltaic cell is further configured for receiving power from the light beam.

In some embodiments, the device further comprises a memory storage for storing the received image data signal.

In some embodiments, the image generator comprises a liquid crystal display device having an illuminator element optically coupled thereto.

In some embodiments, the method further comprises generating, wherein the generating comprises generating by the liquid crystal display device a display representing the image; and wherein the projecting comprises operating the illuminator element to illuminate the liquid crystal display device to project the display onto the retina of the eye.

In some embodiments, the image generator comprises a liquid crystal display device configured for being illuminated by an extraocular illumination source.

In some embodiments, the method further comprises generating, wherein the generating comprises generating by the liquid crystal display device a display representing the image; and wherein the projecting comprises operating the extraocular illumination source to illuminate the liquid crystal display device to project the display onto the retina of the eye.

In some embodiments, the peripheral skirt portion, inner platform portion, and at least one arm, define at least one opening in the planar body, wherein the at least one opening is configured to facilitate fixation and adaptation of the device with the corneal tissue through the at least one opening, when the device is implanted within the cornea.

In some embodiments, the device further comprises an optical assembly mounted posteriorly of the image generator and optically coupled therewith, configured to focus the projected image onto the retina.

In some embodiments, the device further comprises at least one haptic that extends outwardly from the outer circular perimeter of the implant, wherein the at least one haptic comprises a proximal end which is connected to the outer circular perimeter, and a projecting segment culminating in a distal end.

There is further provided, in an embodiment, a system comprising: a device comprising a corneal implant dimensioned and adapted for implantation within the cornea of an eye, the implant defining a planar body comprising: (i) an inner platform portion located about an axis of circular symmetry of the implant, and (ii) a peripheral portion comprising one or more angularly-spaced arms that extend laterally from a peripheral edge of the inner platform, a wireless communication circuit configured for receiving an image data signal from an extraocular unit, an image projection controller configured to process the received image data signal to generate an image, and an image generator mounted to the inner platform portion, configured to project an image projection light beam, based on the generated image, in a posterior direction onto the retina of the eye; and an extraocular unit comprising: at least one imaging device for capturing visual images, an image processing module for processing and storing the captured visual images as digital image data, and a wireless communication module for transmitting the digital image data to the device through the wireless communication circuit.

There is further provided, in an embodiment, a method comprising: providing a system comprising a device comprising a corneal implant dimensioned and adapted for implantation within the cornea of an eye, the implant defining a planar body comprising: (i) an inner platform portion located about an axis of circular symmetry of the implant, and (ii) a peripheral portion comprising one or more angularly-spaced arms that extend laterally from a peripheral edge of the inner platform, a wireless communication circuit configured for receiving an image data signal from an extraocular unit; an image projection controller configured to process the received image data signal to generate an image; and an image generator mounted to the inner platform portion, configured to project an image projection light beam, based on the generated image, in a posterior direction onto the retina of the eye, and an extraocular unit comprising: at least one imaging device for capturing visual images, an image processing module for processing the captured visual images to generate digital image data, and a wireless communication module for transmitting the digital image data to the device through the wireless communication circuit; implanting the device in the cornea of an eye, such that transparent corneal tissue or no corneal tissue is present posterior to the image generator of the device; capturing, by the at least one imaging device of the extraocular unit, at least one visual image; processing, by the image processing module, the at least one captured visual image, to generate the digital image data, transmitting, by the wireless communication module, the image data signal, receiving, by the wireless communication circuit, the image data signal; processing, by the image projection controller, the received image data signal, to generate the image; and projecting, by the image generator, the image in a posterior direction onto the retina of the eye.

In some embodiments, the device further comprises a peripheral skirt portion which defines an outer circular perimeter of the implant, wherein the one or more angularly- spaced arms connect between the inner platform and the peripheral skirt portion.

In some embodiments, the wireless communication circuit comprises a reception inductive coil, wherein the wireless communication module comprises a transmission inductive coil.

In some embodiments, the reception inductive coil is further configured for receiving power inductively from the transmission inductive coil.

In some embodiments, the wireless communication module comprises a light beam source.

In some embodiments, the wireless communication circuit comprises a photovoltaic cell configured to receive a light beam encoding the image data signal, wherein the light beam is emitted from the light beam source.

In some embodiments, the photovoltaic cell is further configured for receiving power from the light beam.

In some embodiments, the device comprises a memory storage for storing the received image data signal.

In some embodiments, the image generator comprises a liquid crystal display device having an illuminator element optically coupled thereto.

In some embodiments, the method further comprises generating, wherein the generating comprises generating by the liquid crystal display device a display representing the image; and wherein the projecting comprises operating the illuminator element to illuminate the liquid crystal display device to project the display onto the retina of the eye.

In some embodiments, the image generator comprises a liquid crystal display device, wherein the extraocular unit comprises an illumination source configured to provide illumination to the liquid crystal display device.

In some embodiments, the method further comprises generating, wherein the generating comprises generating by the liquid crystal display device a display representing the image; and wherein the projecting comprises operating the illumination source to illuminate the liquid crystal display device to project the display onto the retina of the eye.

In some embodiments, the illumination source is configured to emit sufficient illumination so as to traverse impaired or opaque layers and to reach the device, when it is implanted within the cornea.

In some embodiments, the peripheral skirt portion, inner platform portion, and at least one arm, define at least one opening in the planar body, wherein the at least one opening is configured to facilitate fixation and adaptation of the device with the corneal tissue through the at least one opening, when the device is implanted within the cornea.

In some embodiments, the device comprises an optical assembly mounted posteriorly of the image generator and optically coupled therewith, configured to focus the projected image onto the retina.

In some embodiments, the device comprises one or more focusing optics mounted posteriorly of the image generator and optically coupled therewith, configured to focus the projected image onto the retina.

In some embodiments, the device comprises at least one haptic that extends outwardly from the outer circular perimeter of the implant, wherein the at least one haptic comprises a proximal end which is connected to the outer circular perimeter, and a projecting segment culminating in a distal end.

In some embodiments, the implanting comprises adjusting a placement of the device within the cornea by manipulating the at least one haptic.

In some embodiments, the components of the extraocular unit are mounted to one of: a spectacle frame, headwear, a helmet, and a headband.

In addition to the exemplary aspects and embodiments described above, further aspects and embodiments will become apparent by reference to the figures and by study of the following detailed description.

### BRIEF DESCRIPTION OF THE FIGURES

Exemplary embodiments are illustrated in referenced figures. Dimensions of components and features shown in the figures are generally chosen for convenience and clarity of presentation and are not necessarily shown to scale. The figures are listed below.

Figs. 1A-1C are schematic illustrations, in perspective (Fig. 1A), perspective cross-section (Fig. 1B), a side cross-section (Fig 1C) views, of an exemplary device of the present disclosure which provides a pathway for transmitting external light through the eye and onto the retina, to replace impaired or opaque layers of the cornea that is otherwise fully or partially impermeable to visible light, according to some embodiments of the present disclosure;
Figs. 1D-1E illustrate a device of the present disclosure as implanted within an eye, according to some embodiments of the present disclosure;
Figs. 2A-2E show an exemplary intracorneal implant of the device of the present disclosure in top perspective (Fig. 2A), bottom perspective (Fig. 2B), side (Fig. 2C), top (Fig. 2D), and bottom (Fig. 2E) views, according to some embodiments of the present disclosure;
Figs. 3A-3C are illustrations, in cross-sectional (Fig. 3A), exploded perspective (Fig. 3B) and exploded side (Fig. 3C) views, of an exemplary optical stem of the device of the present disclosure, configured to be implanted within the cornea of the eye, and to transmit light from an ambient environment and/or an extraocular light source, through the eye, along an optical path and onto the retina, according to some embodiments of the present disclosure;
Figs. 4A-4B show, in perspective (Fig. 4A) and side (Fig. 4B) views, an exemplary inner optical lens of the present disclosure, such as may be used in conjunction with the device of the present disclosure, according to some embodiments of the present disclosure;
Figs. 5A-5D show, in front perspective (Fig. 5A), back perspective (Fig. 5B), side (Fig. 5C), and top (Fig. 5D) views an exemplary optical stem of the present disclosure, such as may be used in conjunction with the device of the present disclosure, according to some embodiments of the present disclosure;
Fig. 6 is an exploded perspective view of an exemplary device of the present disclosure, which further comprises optional external optical elements, according to some embodiments of the present disclosure;
Figs. 7A-7D show front perspective (Fig. 7A), back perspective (Fig. 7B), side (Fig. 7C), and top (Fig. 7E) views of an exemplary optional external optical elements (e.g. external anterior end plate and/or external optical lens) which may be further comprised in the device of the present disclosure, according to some embodiments of the present disclosure;
Figs. 8A-8B show an external optical lens of the present disclosure, according to some embodiments of the present disclosure;
Figs. 9A-9D show exploded perspective (Fig. 9A), exploded side (Fig. 9B) and cross-sectional (Figs. 9C and 9D) views of an exemplary embodiment of a device of the present disclosure, according to some embodiments of the present disclosure;
Figs. 10A-10C show exploded perspective (Fig. 10A), exploded side (Fig. 10B) and cross-sectional (Fig. 10C) views of another exemplary embodiment of a device of the present disclosure, according to some embodiments of the present disclosure;
Figs. 11A-11D show exploded perspective (Fig. HA), exploded side (Fig. 11B) and cross-sectional (Figs. 11C and HD) views of another exemplary embodiment of a device of the present disclosure, according to some embodiments of the present disclosure;
Figs. 11E-11G show exemplary variations of an optical stem comprising a magnetic coupling system configured for magnetically removably attaching an optional external end plate to the anterior end of the device of the present disclosure, according to some embodiments of the present disclosure;
Figs. 12A-12C show exploded perspective (Fig. 12A), exploded side (Fig. 12B) and cross-sectional (Fig. 12C) views of another exemplary embodiment of a device of the present disclosure, according to some embodiments of the present disclosure;
Figs. 13A-13F illustrate the functional steps in a process for the preparation of an exemplary pre-processed corneal tissue scaffold derived from a donor cornea, according to some embodiments of the present disclosure;
Figs. 14A-14D illustrate an exemplary process for producing a pre-assembled intracorneal system including the corneal tissue scaffold and the device of the present disclosure, according to some embodiments of the present disclosure;
Figs. 15A-15F show the functional steps in an exemplary process for surgical implantation of a preassembled intracorneal system including the corneal tissue scaffold and the device of the present disclosure, according to some embodiments of the present disclosure;
Figs. 16A-16G show the functional steps in an exemplary process for surgical implantation of a pre-processed corneal tissue scaffold separately, followed by an implantation of an intracorneal implant of the present disclosure, according to some embodiments of the present disclosure;
Figs. 17A-17J show the functional steps in an exemplary process for surgical implantation of an intracorneal implant of the present disclosure, according to some embodiments of the present disclosure;
Figs. 18A-18C are schematic illustrations of exemplary system for intracorneal projecting of images onto a retina, according to some embodiments of the present disclosure;
Fig. 18D illustrates an exemplary extraocular unit, according to some embodiments of the present disclosure;
Fig. 18E shows an exemplary illumination source, according to some embodiments of the present disclosure;
Figs. 19A-19D show front perspective, posterior, anterior, and side views of an exemplary intracorneal implant, according to some embodiments of the present disclosure;
Fig. 20 is a schematic illustration of an exemplary intracorneal implant comprising a plurality of haptics, according to some embodiments of the present disclosure;
Figs. 21A-21D illustrate steps in the process for surgical implantation of an intracorneal implant of the present disclosure within the cornea, according to some embodiments of the present disclosure;

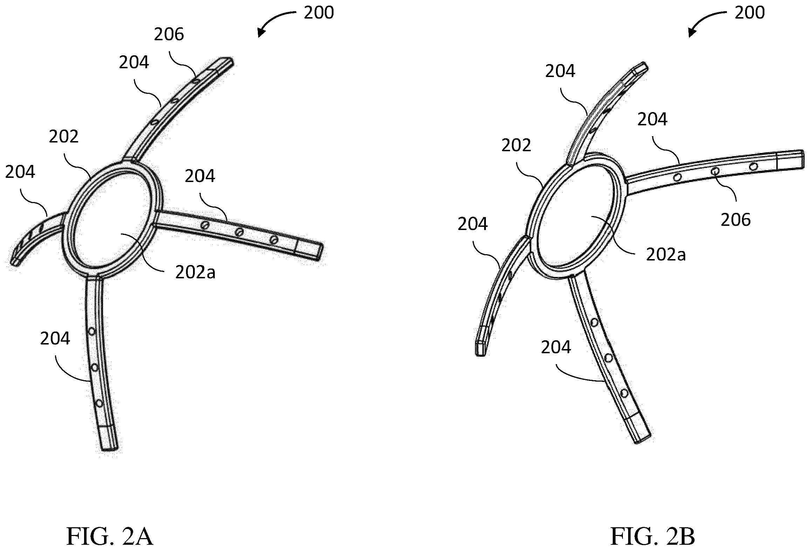

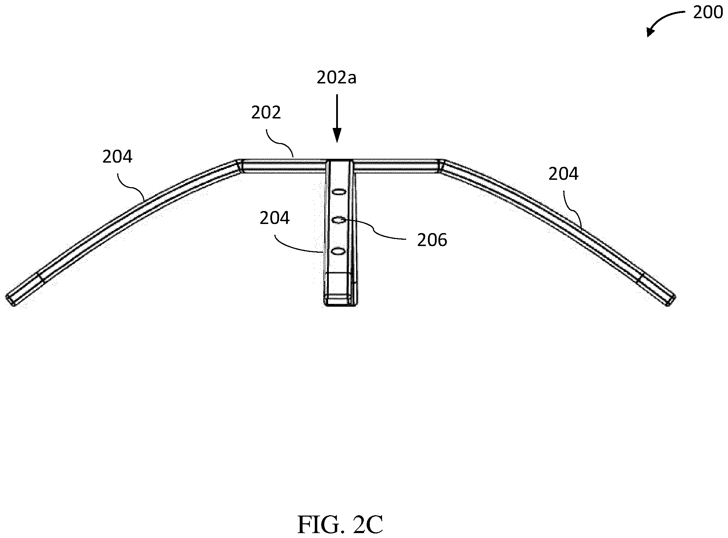

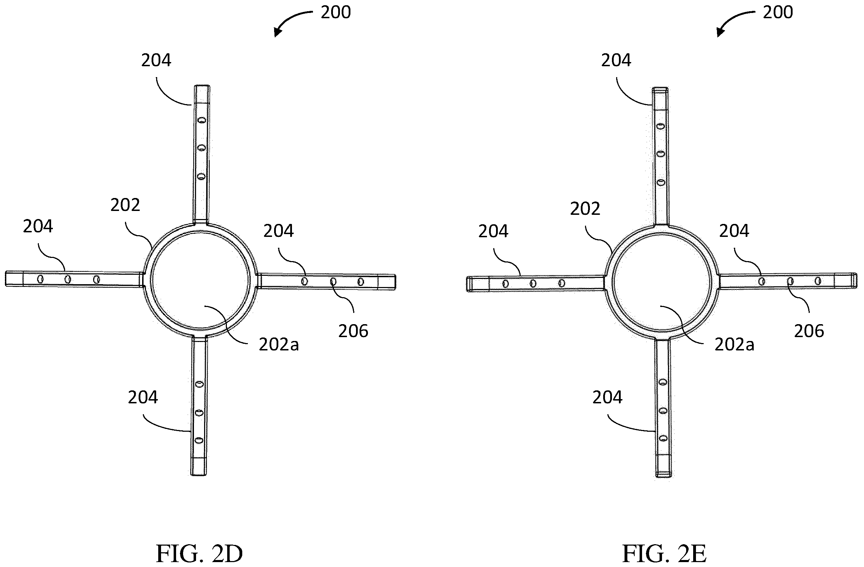

Fig. 2 IE shows an optional optical assembly, according to some embodiments of the present disclosure; and

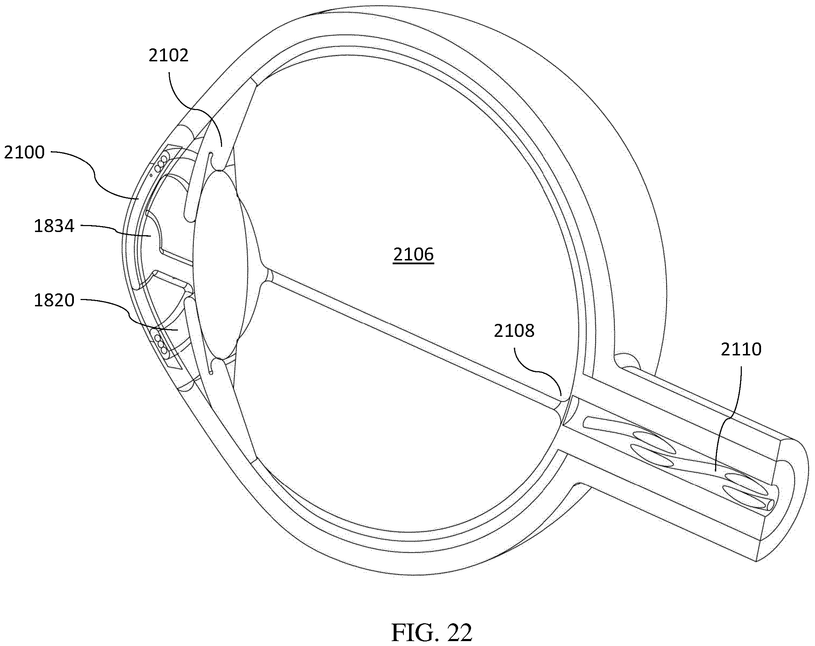

Fig. 22 shows a cross-sectional view of an eye having an intracorneal implant implanted within the cornea, according to some embodiments of the present disclosure.

### DETAILED DESCRIPTION

Disclosed herein are systems, devices and methods which provide for intracorneal implants configured for implantation within the eye, and having a light-projecting posterior port configured to project images onto the retina of the eye.

As used herein, the term 'intracorneal implant' may be used to refer to any device configured to be implanted or deployed inside the cornea of the eye.

In some embodiments, the intracorneal implants of the present disclosure are configured to be implanted within the eye such that only corneal tissue that is substantially transparent is present posteriorly to the light-projecting posterior port of the device, wherein the light -projecting posterior port of the device is configured to project an image projection light beam which traverses the substantially transparent corneal tissue present posteriorly to the light-projecting posterior port, to reach the retina of the eye.

In other embodiments, the intracorneal implants of the present disclosure are configured to be implanted within the eye such that a layer of corneal tissue that is partially transparent, partially semi-transparent, and/or partially opaque, is present posteriorly to the light-projecting posterior port of the device, wherein the light-projecting posterior port of the device is configured to project an image projection light beam which traverses the corneal tissue present posteriorly to the light-projecting posterior port, to reach the retina of the eye.

In accordance with some aspects of the present invention, a device of the present disclosure provides an optical pathway for transmitting external light through the eye and onto the retina, wherein the pathway traverses or completely replaces impaired or opaque layers of the cornea that are otherwise fully or partially impermeable to visible light.

In one aspect of the present disclosure, the device is provided comprising an intracorneal implant configured for surgical implantation within the eye. In some embodiments, the device of the present disclosure may be dimensioned and configured for surgical implantation within the eye, traversing or completely replacing any opaque tissue layers of the cornea, and wherein only substantially transparent corneal tissue is present posterior to the device. In some embodiments, the device of the present disclosure may be configured for surgical implantation within the eye, such that a light-projecting posterior port of the device abuts or is substantially in contact engagement with a very thin layer of corneal tissue (e.g., having a thickness of approximately 10-50µm) that is substantially transparent to light, located posteriorly to the light-projecting posterior port of the device. In some embodiments, the very thin layer comprises at least the pre-Descemet layer. In some embodiments, the very thin layer comprises the pre-Descemet layer in addition to the Descemet membrane. In some embodiments, the very thin layer comprises the pre-Descemet layer, the Descemet membrane, and the endothelium. In some embodiments, the very thin layer of corneal tissue acts as a barrier such that the device does not extend into the anterior chamber, thereby avoiding potential complications including device luxation, hypotony, glaucoma, retroprosthetic membrane formation, or intraocular infection.

In some embodiments, the device of the present disclosure comprises an intracorneal implant configured for surgical implantation within the cornea of the eye, wherein the intracorneal implant comprises an optical stem configured to transmit light from an ambient environment and/or an extraocular light source, through the eye, along an optical path and through the image projection posterior port, onto the retina. In some embodiments, the intracorneal implant of the present disclosure may be configured for surgical implantation within the cornea of the eye, such that a light-projecting posterior port of the device abuts or is substantially in contact engagement with a very thin layer of corneal tissue (e.g., having a thickness of approximately 10-50µm) that is substantially transparent to light, located posteriorly to the light-projecting posterior port of the device. In some embodiments, the very thin layer comprises at least the pre-Descemet layer. In some embodiments, the very thin layer comprises the pre-Descemet layer in addition to the Descemet membrane. In some embodiments, the very thin layer comprises the pre- Descemet layer, the Descemet membrane, and the endothelium.

In some embodiments, the device of the present disclosure comprises an intracorneal implant configured for surgical implantation within the cornea of the eye, wherein the intracorneal implant comprises an optical stem configured to transmit light from an ambient environment and/or an external light source, through the eye, along an optical path, through the image projection port, and onto the retina. In some embodiments, the optical stem defines a light-transparent pathway for transmitting light from the ambient environment and/or an extraocular light source through the eye along an optical path, wherein the pathway defined by the optical stem may comprise at least one optical element, such as an optical lens.

In some embodiments, the optical stem of the present disclosure comprises an optical device that projects an image beyond its light-projecting posterior port (e.g., when the device of the present disclosure is implanted in the eye, the image is projected on the retina), based on light entering the anterior end of the optical stem. In some embodiments, the optical stem has a light-receiving anterior port at an anterior end of the optical stem, located close to the eye's anterior surface. The optical stem extends through the cornea to a light-projecting posterior port of the optical stem, closer to the posterior surface of the cornea. In some embodiments, the anterior end of the optical stem lies just beneath, is substantially at the level of, is flush with, or is slightly proud of, the eye's anterior surface. In some embodiments, the anterior end of the optical stem sits proximately to the eye's surface, fully or partially covered by a thin conjunctival flap layer. In some embodiments, at least a portion of the anterior end of the optical stem is exposed through the eye's anterior surface and forms a means for accepting external optical elements of the device of the present disclosure, as shall be further described below. In some embodiments, the anterior end of the optical stem may comprise an anterior surface which acts as an external optical lens or other optical element. In some embodiments, the anterior end of the optical stem may be configured to receive one or more external optical elements. In some embodiments, the anterior end of the optical stem may comprise a facility configured for removably and/or adjustably mounting one or more optional external elements. In some embodiments, the external elements may be colored or feature a design configured for cosmesis and/or to impart a natural appearance to the eye (e.g. the external optical elements may be colored to resemble/mimic the natural color of the iris).

In some embodiments, the optical stem is a transparent optical path that allows external light or imaging components to pass through the optical stem and to project through the light-projecting posterior port, to form an image on the retina. In some embodiments, the optical stem includes inner optical and/or imaging components along the optical path, such as lenses, which assist in providing a focused image on the retina.

In other embodiments, the optical stem includes an image generator incorporating any suitable image display and/or projection technology, such as liquid crystal display (LCD) element that has an illuminator element optically coupled thereto, a LED projector, a laser diode projector, a LED array, and the like. In some embodiments, the image generator is configured to receive an image data signal from an extraocular source (such as an extraocular imaging device), and to generate an optical image signal that is projected in a posterior direction through the light-projecting posterior port, onto the retina of the eye.

In some embodiments, the device of the present disclosure comprises: (i) an intracorneal implant configured for surgical implantation within the cornea of the eye, and (ii) an optical stem configured to transmit light from an ambient environment and/or an extraocular light source, through the eye, along an optical path, and through the light projecting posterior port, onto the retina.

In some embodiments, an intracorneal implant of the present disclosure generally defines a convex body having a convexity which substantially conforms to the convexity of the cornea. In some embodiments, the implant comprises a central optical part, configured to receive and retain the optical stem of the present device, and a peripheral haptic part. In some embodiments, the central optical part comprises a circumferentially-continuous annular rim, which defines a central aperture configured to receive and retain the optical stem of the present device therein. In some embodiments, the peripheral haptic part of the implant comprises two or more angularly-spaced haptics that extend laterally from a peripheral edge of the annular body member.

In another aspect of the present disclosure, a method is disclosed which provides a pathway for transmitting external light through the eye and onto the retina, traversing or completely replacing any opaque layers of the cornea that are otherwise fully or partially impermeable to visible light. In some embodiments, the method comprises providing a device comprising an intracorneal implant, and surgically implanting the intracorneal implant within the cornea of the eye, at a position where only substantially transparent corneal tissue is present posterior to a light -projecting posterior port of the device. In some embodiments, the intracorneal implant of the present disclosure may be configured for surgical implantation within the cornea of the eye, such that a light-projecting posterior port of the device abuts or is substantially in contact engagement with a very thin layer of corneal tissue (e.g., having a thickness of approximately 10-50µm) that is substantially transparent to light, located posteriorly to the light-projecting posterior port of the device. In some embodiments, the very thin layer comprises at least the pre-Descemet layer. In some embodiments, the very thin layer comprises the pre-Descemet layer in addition to the Descemet membrane. In some embodiments, the very thin layer comprises the pre- Descemet layer, the Descemet membrane, and the endothelium.

In some embodiments, the device of the present disclosure comprises an intracorneal implant configured for surgical implantation within the cornea of the eye, wherein the intracorneal implant comprises an optical stem configured to transmit light from an ambient environment and/or an extraocular light source, through the eye, along an optical path and onto the retina. In some embodiments, the intracorneal implant of the present disclosure may be configured for surgical implantation within the cornea of the eye, such that a light-projecting posterior port of the device abuts or is substantially in contact engagement with a very thin layer of corneal tissue (e.g., having a thickness of approximately 10-50µm) that is substantially transparent to light, located posteriorly to the light-projecting posterior port of the device. In some embodiments, the very thin layer comprises at least the pre-Descemet layer. In some embodiments, the very thin layer comprises the pre-Descemet layer in addition to the Descemet membrane. In some embodiments, the very thin layer comprises the pre-Descemet layer, the Descemet membrane, and the endothelium. In some embodiments, the very thin layer of corneal tissue may comprise a part of the optical path.

In some embodiments, the optical stem defines a light-transparent pathway for transmitting light from the ambient environment and/or an extraocular light source through the eye along an optical path, wherein the pathway defined by the optical stem may comprise one or more optional optical elements, such as optical lenses. In some embodiments, the optical stem of the present disclosure comprises an optical device that projects an image beyond its light-projecting posterior port (e.g., when the device of the present disclosure is implanted in the eye, the image is projected on the retina) based on light entering a light receiving anterior port located at an anterior end of the optical stem. In some embodiments, the optical stem has a light-receiving anterior port located at an anterior end of the optical stem, close to the eye's anterior surface. In some embodiments, the optical stem extends through the cornea to a light-projecting posterior port of the optical stem, located closer to the posterior surface of the cornea. In some embodiments, the anterior end of the optical stem lies just beneath, is substantially at the level of, is flush with, or is slightly proud of, the eye's anterior surface. In some embodiments, the anterior end of the optical stem sits proximately to the eye's surface, fully or partially underneath a thin conjunctival flap layer. In some embodiments, the anterior end of the optical stem may comprise a curved anterior surface which acts as an anterior-end focusing optical element. In some embodiments, the anterior end of the optical stem may be configured to receive one or more external optical elements. In some embodiments, the optical stem includes inner optical and/or imaging components, such as lenses, which assist in providing a focused image on the retina. In other embodiments, the optical stem is simply a transparent path that allows external light or imaging components to form the image on the retina. In some embodiments, the external optical elements may be removable, replaceable, adjustable, and/or may be colored or feature a design configured for cosmesis and/or to impart a natural appearance to the eye (e.g. the external optical elements may be colored to resemble/mimic the natural color of the iris).

In other embodiments, the optical stem includes an image generator incorporating any suitable image display and/or projection technology, such as liquid crystal display (LCD) element that has an illuminator element optically coupled thereto, a LED projector, a laser diode projector, a LED array, and the like. In some embodiments, the image generator is configured to receive an image data signal from an extraocular source (such as an extraocular imaging device), and to generate an optical image signal that is projected in a posterior direction through the light-projecting posterior port, onto the retina of the eye.

In some embodiments, the intracorneal implant of the present disclosure generally defines a convex body having a convexity which substantially conforms to the convexity of the cornea. In some embodiments, the implant comprises a central optical part, configured to receive and retain an optical stem of the present device therein, and a peripheral haptic part. In some embodiments, the central optical part comprises a circumferentially- continuous annular body member configured to receive and retain an optical stem of the present device therein. In some embodiments, the peripheral haptic part of the implant comprises two or more angularly-spaced haptics that extend laterally from a peripheral edge of the annular body member.

In some embodiments, the method comprises surgically implanting a device of the present disclosure in a recipient, by first performing deep lamellar dissection (e.g. pneumatic, hydro-assisted, viscoelastic-assisted and/or manual dissection) in the recipient cornea which separates between the deep posterior stroma and the pre-Descemet layer (alternatively, between the pre-Descemet layer and the Descemet membrane). A partial thickness trephination and anterior keratectomy is performed to remove the anterior corneal lamella of the recipient cornea. The deep posterior stroma (e.g. the bubble roof) is removed to create a recess, leaving the layers posterior thereto, i.e., pre-Descemet layer, the Descemet membrane, and/or the endothelium, intact. The device of the present disclosure is placed and sutured within the created recess. The previously-removed recipient anterior corneal lamella, or a donor anterior corneal lamella, is sutured. A conjunctival flap may be placed to temporarily cover the cornea. The conjunctival flap can then be partially removed, to create a central aperture corresponding to the anterior end of the device, which may be configured to receive one or more external optical elements. As used herein, 'recess' may refer to any opening, dissection, separation, niche or any other opening within the cornea in any shape that can host the intracorneal implant.

In yet another aspect of the present disclosure, there is provided a pre-processed corneal tissue scaffold derived from a donor cornea and comprising at least some of stromal layers, pre-Descemet layer, the Descemet membrane, and/or the endothelium.

In some embodiments, a corneal tissue scaffold of the present disclosure may comprise various compositions of corneal layers at different regions thereof. For example, the corneal tissue scaffold may comprise, at a central, substantially transparent, region thereof, the pre-Descemet layer, the Descemet membrane, and/or the endothelium. In some embodiments, the central region of the corneal tissue scaffold is thus configured to be substantially transparent to light. In some embodiments, regions of the corneal tissue scaffold surrounding the central, substantially transparent, region, may have a different composition of corneal layers, comprising at least some of the stromal layer, pre-Descemet layer, the Descemet membrane, and/or the endothelium.

In some embodiments, the pre-processed corneal tissue scaffold of the present system comprises corneal tissue derived from a donor human cornea. In some embodiments, initially, the corneal tissue is collected from a donor. The collected corneal tissue may then be preserved and transported in conventional tissue preservation manner to a suitable processing location or facility for evaluation, preparation, processing and storage.

In some embodiments, the corneal tissue is then prepared by performing deep lamellar dissection (e.g. pneumatic, hydro-assisted, viscoelastic-assisted and/or manual dissection) which separates (i) between the posterior stroma and the pre-Descemet layer, or, alternatively, (ii) between the pre-Descemet layer and the Descemet membrane. A partial thickness trephination and anterior keratectomy is performed to remove the donor anterior corneal lamella. The deep posterior stroma (e.g. the bubble roof) is removed to create a recess, leaving the layers posterior thereto, i.e., pre-Descemet layer, the Descemet membrane, and/or the endothelium, intact. A full-thickness trephination is then performed to obtain the corneal tissue scaffold.

The obtained corneal tissue scaffold may then be decontaminated, dehydrated, sterilized, and packaged for use by surgeons and other medical professionals in appropriate surgical procedures as further detailed herein.

In some embodiments, the corneal tissue scaffold of the present system may further be pre-processed using any one or more suitable preprocessing methods and techniques, to stabilize and protect the corneal tissue scaffold during preparation, storage, and transportation. In some embodiments, preprocessing methods and techniques according to the present invention may include, but are not limited to: dehydration, lyophilization, plasticization, gelling, dehydrothermal treatment, mechanical processing, compressing, decontamination, sterilization, and the like.

In some embodiments, the preprocessing prevents the corneal tissue scaffold from decomposing, putrefying, becoming infected, and/or fracturing during storage and transportation, and allows the corneal tissue scaffold to be stored at room temperature or at a temperature lower than room temperature. In some embodiments, the preprocessing also helps maintain the corneal tissue scaffold so that it has similar material properties to those of live or hydrated tissue or treated tissue. In some embodiments, the preprocessing allows for direct implantation of the corneal tissue scaffold into a recipient subject without further processing or with minimal processing after removal from the packaging.

In some embodiments, the pre-processed donor corneal tissue scaffold of the present disclosure is configured for implantation in a recipient subject, to replace at least some of the stromal layers, pre-Descemet layer, the Descemet membrane, and/or the endothelial layer in the subject, in preparation for further implantation of a device of the present disclosure, as further detailed herein. In such embodiments, the pre-processed corneal tissue scaffold of the present disclosure is configured for implantation in conjunction with a full-thickness transplant procedure, in which a full-thickness resection of the patient's cornea is performed, followed by replacement with an implant and/or system comprising at least of the corneal tissue scaffold of the present disclosure.

In yet another aspect of the present disclosure, a system is provided comprising (i) a device configured for surgical implantation within the eye, and (ii) a pre-processed corneal tissue scaffold derived from a donor cornea and comprising at least some of the stromal layers, pre-Descemet layer, the Descemet membrane, and/or the endothelial layer.

In some embodiments, the device of the present system comprises an intracorneal implant with an optical stem. In some embodiments, the optical stem is configured to transmit light from an ambient environment and/or an extraocular light source, through the eye, along an optical path, through a light-projecting posterior port, and onto the retina. In some embodiments, the optical stem defines a light-transparent pathway for transmitting light from the ambient environment and/or an extraocular light source through the eye along an optical path, wherein the pathway defined by the optical stem may comprise one or more optional external optical elements, such as optical lenses.

In some embodiments, the optical stem comprises an optical device that forms an image beyond its light-projecting posterior port (e.g., when the device of the present system is implanted in the eye, the image is projected on the retina) based on light entering a light receiving anterior port located at an anterior end of the optical stem. In some embodiments, the optical stem has a light-receiving anterior port close to the anterior surface, extending axially through the cornea to a light-projecting posterior port of the optical stem, closer to the posterior surface of the cornea. In some embodiments, the anterior end of the optical stem lies just beneath, is substantially at the level of, is flush with, or is slightly proud of, the eye's anterior surface. In some embodiments, the anterior end of the optical stem sits proximately to the eye's surface, fully or partially underneath a thin conjunctival flap layer. In some embodiments, the anterior end of the optical stem may comprise a curved anterior surface which acts as an anterior-end focusing optical element, and/or one or more separately-formed external optical elements. In some embodiments, the anterior end of the optical stem may be configured to receive one or more external optical elements. In some embodiments, the anterior end of the optical stem may comprise a facility configured for a removably and/or adjustably mounting one or more external optical elements. In some embodiments, the external elements may be colored or feature a design configured for cosmesis and, or to impart a natural appearance to the eye (e.g. the external optical elements may be colored to resemble/mimic the natural color of the iris).

In some embodiments, the optical stem includes inner optical and/or imaging components, such as lenses, which assist in providing a focused image on the retina. In other embodiments, the optical stem is simply a transparent path that allows external light or imaging components to form the image on the retina.

In other embodiments, the optical stem includes an image generator incorporating any suitable image display and/or projection technology, such as liquid crystal display (LCD) element that has an illuminator element optically coupled thereto, a LED projector, a laser diode projector, a LED array, and the like. In some embodiments, the image generator is configured to receive an image data signal from an extraocular source (such as an extraocular imaging device), and to generate an optical image signal that is projected in a posterior direction through the light-projecting posterior port, onto the retina of the eye.

In some embodiments, the intracorneal implant of the present system generally defines a convex body having a convexity which substantially follows the convexity of the cornea. In some embodiments, the implant comprises a central optical part and a peripheral haptic part. In some embodiments, the central optical part comprises a circumferentially-continuous annular body member configured to receive and retain an optical stem of the present device therein. In some embodiments, the peripheral haptic part of the implant comprises two or more angularly-spaced haptics that extend laterally from a peripheral edge of the annular body member.

In some embodiments, a corneal tissue scaffold of the present system may comprise various compositions of corneal layers at different regions thereof. For example, the corneal tissue scaffold may comprise, at a central, substantially transparent, region thereof, the pre-Descemet layer, the Descemet membrane, and/or the endothelium. In some embodiments, the central region of the corneal tissue scaffold is thus configured to be substantially transparent to light. In some embodiments, regions of the corneal tissue scaffold surrounding the central, substantially transparent region, may have a different composition of corneal layers, comprising at least some of the stromal layer, pre-Descemet layer, the Descemet membrane, and/or the endothelium.

In some embodiments, the pre-processed corneal tissue scaffold of the present system comprises corneal tissue derived from a donor human cornea. In some embodiments, initially, the corneal tissue is collected from a donor. The collected corneal tissue may then be preserved and transported in conventional tissue preservation manner to a suitable processing location or facility for evaluation, preparation, processing and storage.

In some embodiments, the donor corneal tissue is then prepared by performing deep lamellar dissection (e.g. pneumatic, hydro-assisted, viscoelastic-assisted and/or manual dissection) which separates (i) between the posterior stroma and the pre-Descemet layer, or, alternatively, (ii) between the pre-Descemet layer and the Descemet membrane. A partial thickness trephination and anterior keratectomy is performed to remove the donor anterior corneal lamella. The deep posterior stroma (e.g. the bubble roof) is removed to create a recess, leaving the layers posterior thereto, i.e., pre-Descemet layer, the Descemet membrane, and/or the endothelium, intact. A full-thickness trephination is then performed to obtain the corneal tissue scaffold.

The obtained corneal tissue scaffold may then be decontaminated, dehydrated, sterilized, and packaged for use by surgeons and other medical professionals in appropriate surgical procedures as further detailed herein.

In some embodiments, the corneal tissue scaffold of the present system may further be pre-processed using any one or more suitable preprocessing methods and techniques, to stabilize and protect the corneal tissue scaffold during preparation, storage, and transportation. In some embodiments, preprocessing methods and techniques according to the present invention may include, but are not limited to: dehydration, lyophilization, plasticization, gelling, dehydrothermal treatment, mechanical processing, compressing, decontamination, sterilization, and the like. In some embodiments, the preprocessing prevents the corneal tissue scaffold from decomposing, putrefying, becoming infected, and/or fracturing during storage and transportation, and allows the corneal tissue scaffold to be stored at room temperature or at a temperature lower than room temperature. In some embodiments, the preprocessing also helps maintain the corneal tissue scaffold so that it has similar material properties to those of live or hydrated tissue or treated tissue. In some embodiments, the preprocessing allows for direct implantation of the corneal tissue scaffold into a recipient subject without further processing or with minimal processing after removal from the packaging.

In some embodiments of the present system, the corneal tissue scaffold and the device are pre-assembled, such that a light-projecting posterior port of the device abuts or is substantially in contact engagement with a very thin layer of corneal tissue (e.g., having a thickness of approximately 10-50µm) that is substantially transparent to light, posteriorly to the light-projecting posterior port of the device. In some embodiments, the very thin layer comprises at least the pre-Descemet layer. In some embodiments, the very thin layer comprises the pre-Descemet layer in addition to the Descemet membrane. In some embodiments, the very thin layer comprises the pre-Descemet layer, the Descemet membrane, and the endothelium.

In some embodiments, the present system, in its pre-assembled configuration comprising the device pre-mounted to a corneal tissue scaffold, is configured for surgical implantation in a recipient, in conjunction with a full-thickness transplant procedure, in which a full-thickness resection of the patient's cornea is performed, followed by placement (e.g. suturing) of the present system in place.

In some embodiments, the device and the corneal tissue scaffold form separate parts of the present system, wherein the corneal tissue scaffold is configured for surgical implantation in a recipient subject, to replace at least some of stromal layers, pre-Descemet layer, the Descemet membrane, and/or the endothelial layer in the subject, in preparation for further implantation of an intracorneal implant of the present system, as further detailed herein. In such embodiments, the pre-processed corneal tissue scaffold of the present system is configured for implantation in conjunction with a full-thickness transplant procedure, in which a full-thickness resection of the patient's cornea is performed.

In some embodiments, after implantation of the corneal tissue scaffold, the device is implanted within the recipient's cornea such that a light-projecting posterior port of the device abuts or is substantially in contact engagement with a very thin layer of corneal tissue (e.g., having a thickness of approximately 10-50µm) that is substantially transparent to light, posteriorly to the light-projecting posterior port of the device. In some embodiments, the very thin layer comprises at least the pre-Descemet layer. In some embodiments, the very thin layer comprises the pre-Descemet layer in addition to the Descemet membrane. In some embodiments, the very thin layer comprises the pre-Descemet layer, the Descemet membrane, and the endothelium.

In some embodiments, the very thin layer of corneal tissue acts as a barrier such that the intracorneal implant of the present disclosure does not extend into the anterior chamber, thereby avoiding potential complications including device luxation, hypotony, glaucoma, retroprosthetic membrane formation, or intraocular infection.

In accordance with some aspects of the present invention, a device of the present disclosure provides an intracorneal implant configured for surgical implantation within the cornea of an eye, wherein the intracorneal implant comprises an image generator mounted to the intracorneal implant, and configured and operable to receive an image data signal from an extraocular source and to generate an image projection light beam (e.g., light beam modulated with visual image information) to be projected on the retina of the eye, for generating an image thereon.

In some embodiments, the device of the present disclosure may be dimensioned and configured for surgical implantation within the eye, traversing or completely replacing any opaque tissue layers of the cornea, and wherein only substantially transparent corneal tissue is present posterior to the device.

In some embodiments, the device of the present disclosure may be dimensioned and configured for surgical implantation within the eye, such that is present posteriorly to the light-projecting posterior port of the device.

In some embodiments, the device of the present disclosure may be configured for surgical implantation within the eye, such that a light-projecting posterior port of the device abuts or is substantially in contact engagement with a very thin layer of corneal tissue (e.g., having a thickness of approximately 10-50µm) that is substantially transparent to light, located posteriorly to the light-projecting posterior port of the device. In some embodiments, the very thin layer comprises at least the pre-Descemet layer. In some embodiments, the very thin layer comprises the pre-Descemet layer in addition to the Descemet membrane. In some embodiments, the very thin layer comprises the pre- Descemet layer, the Descemet membrane, and the endothelium. In some embodiments, the very thin layer of corneal tissue acts as a barrier such that the device does not extend into the anterior chamber, thereby avoiding potential complications including device luxation, hypotony, glaucoma, retroprosthetic membrane formation, or intraocular infection.

In some embodiments, the device of the present disclosure comprises an intracorneal implant dimensioned to fit within a cornea. In some embodiments, the intracorneal implant defines a planar body having a convexity which substantially conforms to the convexity of the cornea, with opposing convex anterior face (facing the anterior surface of the eye) and concave posterior face (facing the retina of the eye).

In some embodiments, the intracorneal implant comprises an inner platform portion located about an axis of circular symmetry of the implant, and a peripheral portion, comprising one or more angularly-spaced arms that extend laterally from a peripheral edge of the inner platform. In some embodiments, the intracorneal implant further comprises a peripheral skirt portion which defines an outer circular perimeter of the intracorneal implant, wherein the one or more angularly-spaced arms connect between the inner platform and the peripheral skirt portion.

In some embodiments, an intracorneal implant of a device of the present disclosure may further comprise two or more angularly-spaced haptics that extend laterally from a peripheral edge of the implant. The haptics may function structurally to position and center the intracorneal implant at a desired location, e.g., to align with an optical axis of the eye, within the pocket formed in the cornea during surgery.

In some embodiments, one or more openings formed within the intracorneal implant of the present disclosure, e.g., located about the one or more connecting arms or the peripheral skirt, may be configured to allow adaptation of the device within the cornea, so as to obtain long-term fixation of the intracorneal implant in a desired position.

In some embodiments, the intracorneal implant of the present disclosure comprises one or more of: an image generator, an image projection controller including a hardware processor and a memory storage, an optical assembly, a wireless receiver module, and a wireless power receiving element.

In some embodiments, the device of the present disclosure comprises an image generator mounted to the intracorneal implant, and configured and operable to receive an image data signal from an extraocular source and to generate an image projection light beam (e.g., light beam modulated with visual image information) to be projected on the retina of the eye through a light-projecting posterior port, for generating an image thereon. Visual image information, as the term is used herein, can encompass any optically-communicated information including optical reproductions of an object or a scene, and/or optically- represented information, such as by text, symbols or color.

In some embodiments, the image generator is configured to receive an image data signal from an extraocular source and to generate an image projection light beam capable of traversing through any semi-opaque, and/or substantially opaque corneal tissue that is present anterior to the device, to reach the retina of the eye.

In some embodiments, the device of the present disclosure may be configured to receive an image data signal from an image data source that is external to the eye, via a non- optical data transmission modality, such as radio frequency (RF) or similar data transmission, wherein the non-optical data transmission modality is capable of traversing impaired or opaque layers of the eye (e.g., cornea, conjunctiva or other forms of tissue flaps) that are otherwise fully or partially impermeable to visible light.

Accordingly, in some embodiments, the device of the present disclosure is configured to receive a digital image data signal from an external source via a non-optical data transmission path, e.g., short-range RF transmission; to process the digital image data encoded in the image data signal; to generate a light beam modulated with the image information encoded in the digital image data; and to project the generated light beam onto the retina, via an optical transmission path which may comprise an optical assembly comprising, e.g., one or more focusing optics and/or the lens of the eye.

In some embodiments, a device of the present disclosure comprises a wireless receiver module configured to receive wirelessly an image data signal representative of image information, via a non-optical wireless transmission modality, e.g., a short-range RF communication protocol.

In some embodiments, a device of the present disclosure comprises an image projection controller which receives the input image data received by the wireless receiver module and optionally stores the image data in a memory storage module; processes the digital image data encoded in the signal, to generate and reconstruct a visual image therefrom; and operates an image generator to generate a light beam based on the image data and project the generated light beam onto the retina.

In some embodiments, a device of the present disclosure comprises an image generator for displaying and/or projecting an optical image signal representative of visual images of the type suitable for detection by a retina. In some embodiments, the image generator can incorporate any suitable image display and/or projection technology, such as liquid crystal display (LCD) element that has an illuminator element optically coupled thereto, a LED projector, a laser diode projector, a LED array, and the like. In some embodiments, the image generator comprises an optical assembly comprising any suitable focusing or other optics.

In some embodiments, the device of the present disclosure may further include a power source configured for providing electrical power to the device. In some embodiments, the power source is configured to receive power from an external power source using wireless power transmission. This is done in order to avoid using wired power connections to an external power source, or internal batteries. Wired power connections to an external power source must pass through the surface of the eye, and thus involve several practical disadvantages, as well as risks of infection, etc. Similarly, powering the device using replaceable batteries installed or mounted within the implantable device itself is impractical, as batteries are bulky, and surgical intervention will be required in order to replace them. Accordingly, in some embodiments, the present disclosure provides for transmitting power to the device into the eye inductively. Thus, for example, a primary (external) inductive coil may be placed in in the vicinity of the eye, e.g., positioned about a lens area, or encased within the rim, of a pair of glasses, and a secondary (internal) reception coil within the device may be used to receive the power transmitted by the primary coil. Accordingly, in some embodiments, the power source may be configured to be charged or to receive power via wireless power transmission, e.g., via inductive power transfer or any other suitable wireless power transmission technique.

In some embodiments, the wireless power and data transmission are carried out via transmitted light, wherein an extraocular unit is configured to transmit, via a light beam, a combined power and image data signal to the intracorneal implant. In some embodiments, the present disclosure provides for wireless and/or data transfer between an extraocular unit and the device, via transmitted light. In some embodiments, power and data transmission may be performed concurrently, such that a light-transmitted power signal may be transmitted between an extraocular unit and an intracorneal implant, wherein the power signal may encode data during at least a portion of its transmission duration. Thus, in some embodiments, a device of the present disclosure includes a wireless receiver module configured to receive power and image data signal transmitted by an extraocular unit, via a transmitted light signal. In some embodiments, the wireless receiver module may comprise, e.g., a photovoltaic cell.

In yet another aspect, a system is provided comprising an intracorneal implant configured for surgical implantation within the cornea of an eye; and an extraocular image capture unit in wireless power and data communication with the intracorneal implant, configured to transmit power and image data wirelessly to the intracorneal implant, wherein the image data represents visual images captured by the extraocular unit, and wherein the intraocular implant is configured to generate, from the received image data, an image projection light beam (e.g., light beam modulated with visual image information), and to project the generated light beam on the retina of the eye, for generating the captured visual images thereon.

In some embodiments, the extraocular unit of the present system comprises at least an imaging device for capturing visual images; an image processing module, comprising one or more hardware processors and a memory storage, for processing and storing the digital image data, to generate an image data signal for transmitting to the intracorneal implant; a wireless transmitter to transmit the image data signal to the intracorneal implant, using, e.g., any suitable short-range RF communication protocol; and a power source to power the extraocular unit and/or the intracorneal unit, e.g., using wireless power transmission.

In some embodiments, an extraocular unit of a system of the present disclosure may be formed by several components disposed on a conventional spectacle frame, or coupled to another head- worn element such as a helmet, headband, etc.

In some embodiments, the intracorneal implant of the present system comprises one or more of: an image projection controller including a hardware processor and a memory storage; an image generator; an optical assembly; a wireless receiver module; and a wireless power receiving element.

In some embodiments, the intraocular implant of the present system may be dimensioned and configured for surgical implantation within the eye, traversing or completely replacing any opaque tissue layers of the cornea, and wherein only substantially transparent corneal tissue is present posterior to the intraocular implant.

In some embodiments, the intraocular implant of the present system may be dimensioned and configured for surgical implantation within the eye, such that a layer of corneal tissue that is partially transparent, partially semi-transparent, and/or partially opaque is present posteriorly to the light-projecting posterior port of the device.

In some embodiments, the intraocular implant of the present system may be configured for surgical implantation within the eye, such that a light-projecting posterior port of the intraocular implant abuts or is substantially in contact engagement with a very thin layer of corneal tissue (e.g., having a thickness of approximately 10-50µm) that is substantially transparent to light, located posteriorly to the light-projecting posterior port of the intraocular implant. In some embodiments, the very thin layer comprises at least the pre-Descemet layer. In some embodiments, the very thin layer comprises the pre-Descemet layer in addition to the Descemet membrane. In some embodiments, the very thin layer comprises the pre-Descemet layer, the Descemet membrane, and the endothelium. In some embodiments, the very thin layer of corneal tissue acts as a barrier such that the intraocular implant does not extend into the anterior chamber, thereby avoiding potential complications including intraocular implant luxation, hypotony, glaucoma, retroprosthetic membrane formation, or intraocular infection.

In some embodiments, the intraocular implant of the present system comprises an intracorneal implant dimensioned to fit within a cornea. In some embodiments, the intracorneal implant defines a planar body having a convexity which substantially conforms to the convexity of the cornea, with opposing convex anterior face (facing the anterior surface of the eye) and concave posterior face (facing the retina of the eye). In some embodiments, the intracorneal implant is.

In some embodiments, the intracorneal implant comprises an inner platform portion located about an axis of circular symmetry of the implant, and a peripheral portion, comprising one or more angularly-spaced arms that extend laterally from a peripheral edge of the inner platform. In some embodiments, the intracorneal implant further comprises a peripheral skirt portion which defines an outer circular perimeter of the intracorneal implant, wherein the one or more angularly-spaced arms connect between the inner platform and the peripheral skirt portion.

In some embodiments, an intraocular implant of the present system may further comprise two or more angularly-spaced haptics that extend laterally from a peripheral edge of the implant. The haptics may function structurally to position and center the intracorneal implant at a desired location, e.g., to align with an optical axis of the eye, within the pocket formed in the cornea during surgery.

In some embodiments, one or more openings formed within the intracorneal implant of the present system, e.g., located about the one or more connecting arms or the peripheral skirt, may be configured to allow adaptation of the intraocular implant within the cornea, so as to obtain long-term fixation of the intracorneal implant in a desired position.

In some embodiments, the intraocular implant of the present system comprises an image generator mounted to the intracorneal implant, and configured and operable to output an image projection light beam (e.g., light beam modulated with image information) to be projected on the retina of the eye, for generating an image thereon.

In some embodiments, the image generator is configured to output an image projection light beam capable of traversing through any transparent, semi-opaque, and/or substantially opaque corneal tissue that is present posteriorly to the light-projecting posterior port of the device, to reach the retina of the eye.

In some embodiments, the intraocular implant of the present system may be configured to receive an image data signal from an extraocular unit of the present system, via a non-optical data transmission modality, such as radio frequency (RF) or similar data transmission, wherein the non-optical data transmission modality is capable of traversing impaired or opaque layers of the eye (e.g., cornea, conjunctiva or other forms of tissue flaps) that are otherwise fully or partially impermeable to visible light.

Accordingly, in some embodiments, the intraocular implant of the present system is configured to receive a digital image data signal from an extraocular unit via a non-optical data transmission path, e.g., short-range RF transmission; to process the digital image data encoded in the image data signal, to generate a light beam modulated/patterned with image information the digital image data; and to project the generated light beam onto the retina, via an optical transmission path which may comprise one or more focusing optics and/or the lens of the eye.

In some embodiments, an intraocular implant of the present system comprises a wireless receiver module in wireless communication with a wireless transmitter of the extraocular unit of the present system, and configured to receive wirelessly an image data signal representative of image information, via a non-optical wireless transmission modality, e.g., a short-range RF communication protocol.

In some embodiments, an intraocular implant of the present system comprises an image projection controller which receives the input image data received by the wireless receiver module from the extraocular unit, and optionally stored by the memory storage module; processes the digital image data encoded in the signal, to generate and reconstruct a visual image therefrom; and operates an image generator to modulate light beam based on the image data, to be projected onto the retina.

In some embodiments, an intraocular implant of the present system comprises an image generator for displaying and/or projecting an optical image signal representative of visual images of the type suitable for detection by a retina. In some embodiments, the image generator can incorporate any suitable image display and/or projection technology, such as liquid crystal display (LCD) element that has an illuminator element optically coupled thereto, a LED projector, a laser diode projector, a LED array, and the like. In some embodiments, the image generator comprises an optical assembly comprising any suitable focusing or other optics.

In some embodiments, the intraocular implant of the present system may further include a power source configured for providing electrical power to the intraocular implant. In some embodiments, the power source is configured to receive power from an external power source using wireless power transmission. This is done in order to avoid using wired power connections to an external power source, or internal batteries. Wired power connections to an external power source must pass through the surface of the eye, and thus involve several practical disadvantages, as well as risks of infection, etc. Similarly, powering the intraocular implant using replaceable batteries installed or mounted within the implantable intraocular implant itself is impractical, as batteries are bulky, and surgical intervention will be required in order to replace them. Accordingly, in some embodiments, the present system provides for transmitting power to the intraocular implant into the eye inductively. Thus, for example, a primary (external) inductive coil may be placed in in the vicinity of the eye, e.g., positioned about a lens area, or encased within the rim, of a pair of glasses, and a secondary (internal) reception coil within the intraocular implant may be used to receive the power transmitted by the primary coil. Accordingly, in some embodiments, the power source may be configured to be charged or to receive power via wireless power transmission, e.g., via inductive power transfer or any other suitable wireless power transmission technique.

In some embodiments, the wireless power and data transmission are carried out via transmitted light, wherein the extraocular unit is configured to transmit, via a light beam, a combined power and image data signal to the intracorneal implant. In some embodiments, the present system provides for wireless and/or data transfer between an extraocular unit and an intracorneal implant, via transmitted light. In some embodiments, power and data transmission may be performed concurrently, such that a light-transmitted power signal may be transmitted between an extraocular unit and an intracorneal implant, wherein the power signal may encode data during at least a portion of its transmission duration.

Thus, in some embodiments, the extraocular unit of the present system comprises a transmitter configured to transmit power and/or data signals via transmitted light. In some embodiments, the transmitter is configured to transmit one or both of power and data during any given time interval. For example, during data transmission portions, the signal may carry information that is encoded by variations in amplitude of the power signal, or by any other suitable modulation technique. Similarly, in some embodiments, an intracorneal implant of the present system of the present includes a wireless receiver module configured to receive the power and image data signal transmitted by the extraocular unit, via a transmitted light signal. In some embodiments, the wireless receiver module may comprise, e.g., a photovoltaic cell.

In yet another aspect, a system is provided comprising an intracorneal implant configured for surgical implantation within the cornea of an eye; and an extraocular image capture unit in wireless power and data communication with the intracorneal implant.

In some embodiments, the extraocular unit of the present system comprises at least an imaging device for capturing visual images; an image processing module, comprising one or more hardware processors and a memory storage, for processing and storing the digital image data, to generate an image data signal for transmitting to the intracorneal implant; a wireless transmitter to transmit the image data signal to the intracorneal implant, using, e.g., any suitable short-range RF communication protocol; a power source to power the extraocular unit and/or the intracorneal unit, e.g., using wireless power transmission.

In some embodiments, the extraocular unit of a system of the present disclosure may be formed by several components disposed on a conventional spectacle frame, or coupled to another head- worn element such as a helmet, headband, etc.

In some embodiments, the intracorneal implant of the present disclosure comprises one or more of: an image projection controller including a hardware processor and a memory storage; an image generator; an optical assembly; a wireless receiver module; and a wireless power receiving element.

In some embodiments, the intraocular implant may be dimensioned and configured for surgical implantation within the eye, traversing or completely replacing any opaque tissue layers of the cornea, and wherein only substantially transparent corneal tissue is present posterior to the intraocular implant.

In some embodiments, the intraocular implant may be dimensioned and configured for surgical implantation within the eye, such that a layer of corneal tissue that is partially transparent, partially semi-transparent, and/or partially opaque is present posteriorly to the light-projecting posterior port of the device.

In some embodiments, the intraocular implant of the present system may be configured for surgical implantation within the eye, such that a light-projecting posterior port of the intraocular implant abuts or is substantially in contact engagement with a very thin layer of corneal tissue (e.g., having a thickness of approximately 10-50µm) that is substantially transparent to light, located posteriorly to the light-projecting posterior port of the intraocular implant. In some embodiments, the very thin layer comprises at least the pre-Descemet layer. In some embodiments, the very thin layer comprises the pre-Descemet layer in addition to the Descemet membrane. In some embodiments, the very thin layer comprises the pre-Descemet layer, the Descemet membrane, and the endothelium. In some embodiments, the very thin layer of corneal tissue acts as a barrier such that the intraocular implant does not extend into the anterior chamber, thereby avoiding potential complications including intraocular implant luxation, hypotony, glaucoma, retroprosthetic membrane formation, or intraocular infection.

In some embodiments, the intraocular implant of the present system comprises an intracorneal implant dimensioned to fit within a cornea. In some embodiments, the intracorneal implant defines a planar body having a convexity which substantially conforms to the convexity of the cornea, with opposing convex anterior face (facing the anterior surface of the eye) and concave posterior face (facing the retina of the eye). In some embodiments, the intracorneal implant is.

In some embodiments, the intracorneal implant comprises an inner platform portion located about an axis of circular symmetry of the implant, and a peripheral portion, comprising one or more angularly-spaced arms that extend laterally from a peripheral edge of the inner platform. In some embodiments, the intracorneal implant further comprises a peripheral skirt portion which defines an outer circular perimeter of the intracorneal implant, wherein the one or more angularly-spaced arms connect between the inner platform and the peripheral skirt portion.

In some embodiments, an intraocular implant of the present system may further comprise two or more angularly-spaced haptics that extend laterally from a peripheral edge of the implant. The haptics may function structurally to position and center the intracorneal implant at a desired location, e.g., to align with an optical axis of the eye, within the pocket formed in the cornea during surgery.

In some embodiments, one or more openings formed within the intracorneal implant of the present system, e.g., located about the one or more connecting arms or the peripheral skirt, may be configured to allow adaptation of the intraocular implant within the cornea, so as to obtain long-term fixation of the intracorneal implant in a desired position.

In some embodiments, the intraocular implant of the present system comprises an image generator mounted to the intracorneal implant, and configured and operable to generate an image pattern on a panel, such as a liquid crystal display (LCD) panel, wherein the generated image pattern is to be projected on the retina of the eye, for generating an image thereon.

In some embodiments, an intraocular implant of the present system comprises a wireless receiver module in wireless communication with a wireless transmitter of the extraocular unit of the present system, and configured to receive wirelessly an image data signal representative of image information, via a non-optical wireless transmission modality, e.g., a short-range RF communication protocol. In some embodiments, the wireless receiver module is configured to receive an image data signal from the extraocular unit of the present system, via a non-optical data transmission modality, such as radio frequency (RF) or similar data transmission, wherein the non-optical data transmission modality is capable of traversing impaired or opaque layers of the eye (e.g., cornea, conjunctiva or other forms of tissue flaps) that are otherwise fully or partially impermeable to visible light.

Accordingly, in some embodiments, the wireless receiver module of the intraocular implant of the present system is configured to receive a digital image data signal from an extraocular unit via a non-optical data transmission path, e.g., short-range RF transmission; to process the digital image data encoded in the image data signal, to generate an image pattern or a series of image patterns on a display panel; and to project the generated image pattern onto the retina, via an optical transmission path which may comprise one or more focusing optics and/or the lens of the eye.

In some embodiments, an intraocular implant of the present system comprises an image projection controller which receives the input image data received by the wireless receiver module from the extraocular unit, and optionally stored by the memory storage module; processes the digital image data encoded in the signal, to generate and reconstruct a visual image therefrom; and operates a display panel to form an image pattern therein, to be projected onto the retina.

In some embodiments, an intraocular implant of the present system comprises an image generator for displaying an optical image representative of visual images of the type suitable for detection by a retina. In some embodiments, the image generator can incorporate any suitable image display, such as liquid crystal display (LCD) element that has an illuminator element optically coupled thereto, and the like. In some embodiments, the image generator comprises an optical assembly comprising any suitable focusing or other optics.

In some embodiments, the intraocular implant of the present system may further include a power source configured for providing electrical power to the intraocular implant. In some embodiments, the power source is configured to receive power from an external power source using wireless power transmission.

In some embodiments, the present system comprises an external illumination source, which may be included in the extraocular unit, and may be configured to illuminate the image generator of the intracorneal implant (e.g., an LCD panel), so as to project the image displayed therein onto the retina of the eye.

In some embodiments, the present system comprises an intraocular illumination source, which may be mounted or coupled to the image generator of the intracorneal extraocular unit, and may be configured to illuminate the image generator of the intracorneal implant (e.g., an LCD panel), so as to project the image displayed therein onto the retina of the eye. Thus, in some embodiments, the image generator may comprise an LCD panel configured to display a visual image thereon. In some embodiments, the LCD panel may comprise a backlight illumination source or another similar illumination source integrated within the intracorneal implant.

In some embodiments, the wireless power and data transmission are carried out via transmitted light, wherein the extraocular unit is configured to transmit, via a light beam, a combined power and image data signal to the intracorneal implant. In some embodiments, the present disclosure provides for wireless and/or data transfer between an extraocular unit and an intracorneal implant, via transmitted light. In some embodiments, power and data transmission may be performed concurrently, such that a light-transmitted power signal may be transmitted between an extraocular unit and an intracorneal implant, wherein the power signal may encode data during at least a portion of its transmission duration.

Thus, in some embodiments, the extraocular unit of the present system comprises a transmitter configured to transmit power and/or data signals via transmitted light. In some embodiments, the transmitter is configured to transmit one or both of power and data during any given time interval. For example, during data transmission portions, the signal may carry information that is encoded by variations in amplitude of the power signal, or by any other suitable modulation technique. Similarly, in some embodiments, an intracorneal implant of the present system of the present includes a wireless receiver module configured to receive the power and image data signal transmitted by the extraocular unit, via a transmitted light signal. In some embodiments, the wireless receiver module may comprise, e.g., a photovoltaic cell.

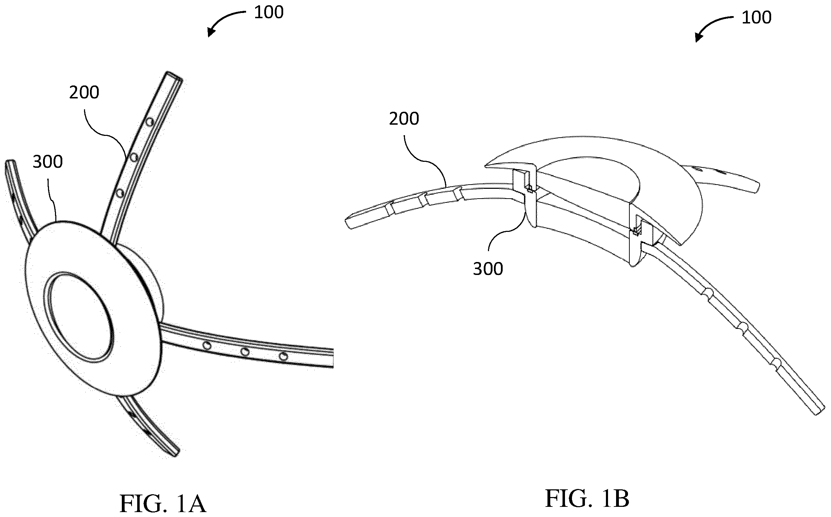

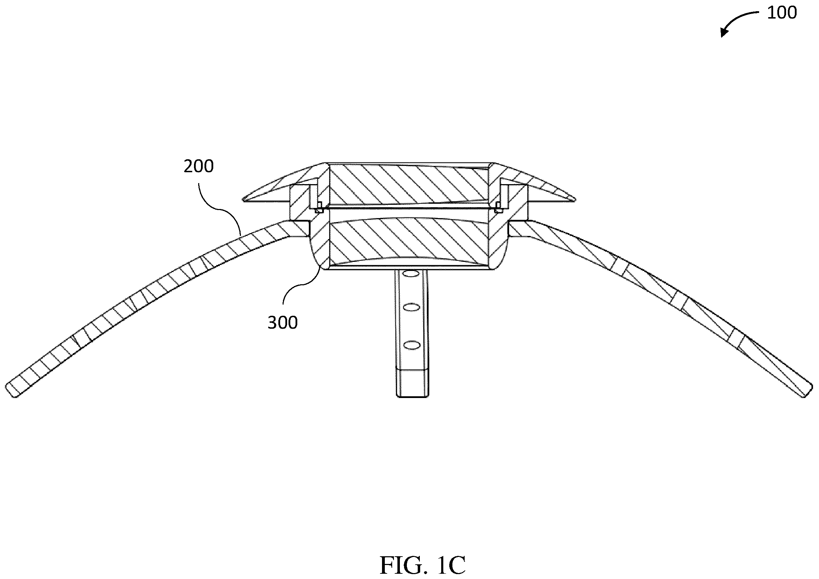

Reference is made to Figs. 1A-1C which schematically illustrate, in perspective (Fig. 1A), perspective cross-section (Fig. 1B), and side cross-section view (Fig. 1C), an exemplary device 100 of the present disclosure, comprising an intracorneal implant 200 and an optical stem 300.

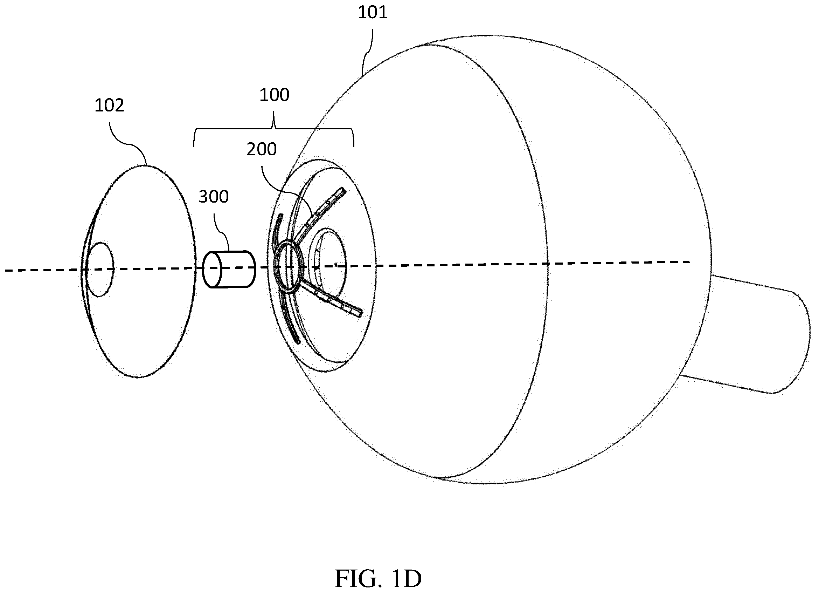

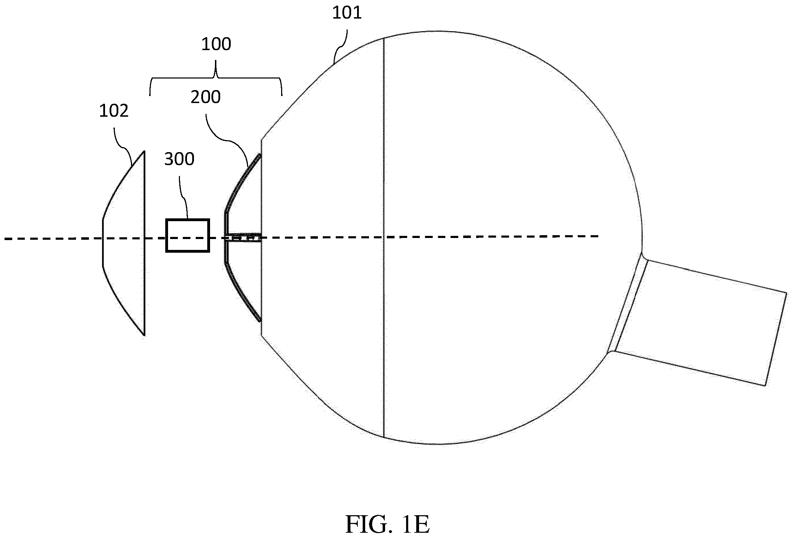

Reference is also made to Figs. 1D-1E which schematically illustrate, in exploded perspective (Fig. 1D) and side (Fig. 1E) views, device 100, comprising an intracorneal implant 200 and an optical stem 300, as implanted within eye 101.

In some embodiments, intracorneal implant 200 is configured for surgical implantation within the cornea of the eye 101. In some embodiments, intracorneal implant 200 generally comprises a central optical part, configured to receive and retain an optical stem 300 of the device 100, and a peripheral haptic part comprising two or more haptics.

In some embodiments, device 100 comprises an optical stem 300 configured to transmit light from an ambient environment and/or an external light source, through the eye 101, along an optical path and onto the retina. In some embodiments, optical stem 300 defines a light-transparent pathway, represented by a dashed line in Figs. 1D-1E, for transmitting light from the ambient environment and/or an extraocular light source through the eye.

In some embodiments, after implantation anterior corneal lamella 102, consisting of a previously-removed recipient anterior corneal lamella, donor corneal tissue, or a corneal tissue scaffold, may be sutured in place to fully or partially cover the cornea and the implanted device.

Figs. 2A-2E show an exemplary intracorneal implant 200 of device 100 (shown in Figs. 1A-1C), in top perspective (Fig. 2A), bottom perspective (Fig. 2B), side (Fig. 2C), top (Fig. 2D), and bottom (Fig. 2E) views, according to some embodiments of the present disclosure.

In some embodiments, intracorneal implant 200 is configured for surgical implantation within the cornea of the eye. In some embodiments, intracorneal implant 200 generally comprises a central optical part comprising an anchor body 202, and a peripheral haptic part comprising two or more haptics 204.

In some embodiments, intracorneal implant 200 defines a planar body having a convexity which substantially conforms to the convexity of the cornea, with opposing convex anterior face (facing the anterior surface of the eye, shown in Fig. 2A) and concave posterior face (facing the retina of the eye, shown in Fig. 2B).

In some embodiments, intracorneal implant 200 is dimensioned to fit within a cornea. For example, intracorneal implant 200 may have an overall span between opposing edges thereof of, e.g., 6-12mm (e.g., 9mm), an overall planar thickness of between 0.02-1.50mm, and a convexity having a curvature of between 32-47 diopters.

Optionally, the central optical part comprises a circumferentially-continuous annular anchor body 202 which defines a central aperture 202a configured to receive and retain optical stem 300 therein. However, in other embodiments, as shall be further detailed below, anchor body 202 may include an integrally-formed optical stem 300.

Anchor body 202 can be a ring, a rim, a cannula, a cylinder, or any other suitable three-dimensional shape which can define a circular or cylindrical aperture and/or form an optical stem (such as optical stem 300 which will be described in detail hereinbelow), and to which haptics 204 can be attached.

In some embodiments, intracorneal implant 200, including anchor body 202 and haptics 204, may be fashioned of any suitable biologically-compatible material, e.g., any metal, metal alloy (such as nitinol, stainless steel, and the like). In some embodiments, the material can be a polymer, e.g., an acrylate polymer, polycarbonate, polymethyl methacrylate (PMMA), polyvinyl chloride (PVC), polyolefin copolymers, fluoropolymers (e.g. PTFE), polyamides, polyimides, polyesters, polyurethane, copolymers thereof, silicone, a cross-linked silicone polymer, and combinations thereof.

In some embodiments, all the components of implant 200 can be made of the same material. In some embodiments, at least two of the components of implant 200 are made of different materials. One or more of the materials comprising intracorneal implant 200 can be transparent, substantially transparent, or optically transparent. One or more of the materials comprising intracorneal implant 200 can be biocompatible.

In some embodiments, intracorneal implant 200 may be fashioned integrally as a unitary intracorneal implant of a single material, or may be assembled from two or more separate components, wherein each component may be made of a different material. In some embodiments, intracorneal implant 200 or any portion thereof may be made of a hard or non-deformable material, a shape-memory material, and/or a flexible or deformable material. In some embodiments, intracorneal implant 200 or any portion thereof may have a coating of, e.g., hydroxyapatite or a similar material. For example, the anchor body 202 and haptics 204 may be made of a different material, or alternatively be made of similar materials having different mechanical properties.

In some embodiments, the peripheral haptic part of implant 200 comprises two or more angularly-spaced haptics 204 that extend laterally from anchor body 202, e.g., from an outer peripheral edge thereof. In some embodiments, haptics 204 extend outwardly from an outer peripheral edge of anchor body 202. The haptics 204 may function structurally to center and anchor an implanted intracorneal implant 200 in place. Each of haptics 204 includes a proximal end which is connected to the outer peripheral edge of anchor body 202, and a projecting segment culminating in a distal end. In some embodiments, between 2 and 6 haptics 204 may be used. In some embodiments, the haptics 204 may be divided into opposing pairs, each of which extends outwardly and away from anchor body 202 at opposite ends thereof. The haptics 204 can have any suitable shape, dimensions, and contour, as known in the art. The haptics 204 included in any given intracorneal implant 200 can be all of the same, or vary, in shape and dimensions.

During implantation of intracorneal implant 200, each of the haptics 204 may be manipulated so as to center the intracorneal implant 200 as close to the optical axis of the eye as possible, as well as to ensure that the intracorneal implant 200 has the desired placement in the rotational direction in the X-Y plane, and secure it in place inside the cornea.

In some embodiments, at least one of haptics 204 comprises one or more openings 206. In some embodiments, each of haptics 204 comprises a series of spaced-part openings 206 along at least a portion of its length. The openings 206 can be any suitable shape or dimensions, such as any regular shapes and regular polygons (e.g. circles, semi-circles, ellipses, squares, triangles, hexagons, octagons, and rectangles) and irregular shapes and polygons. The number of openings 206 on each haptic 204 can be between 0-20 or more openings. The openings 206 can be positioned anywhere on the haptics 204.

In some embodiments, openings 206 are configured to allow adaptation of the intracorneal implant 200 within the cornea through the openings 206 and fixation of the intracorneal implant 200 within the surrounding corneal tissue. In this manner, once intracorneal implant 200 has been implanted, and the eye has healed, intracorneal implant 200 becomes substantially anchored in place by the growth and remodeling of surrounding tissue.

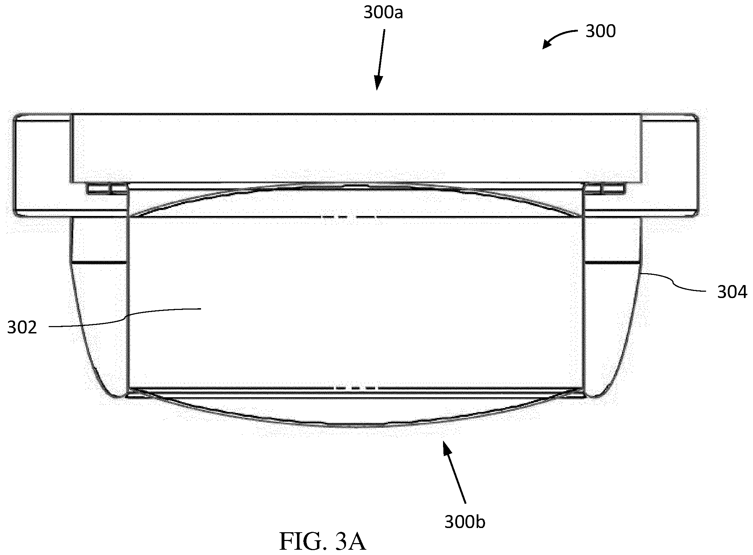

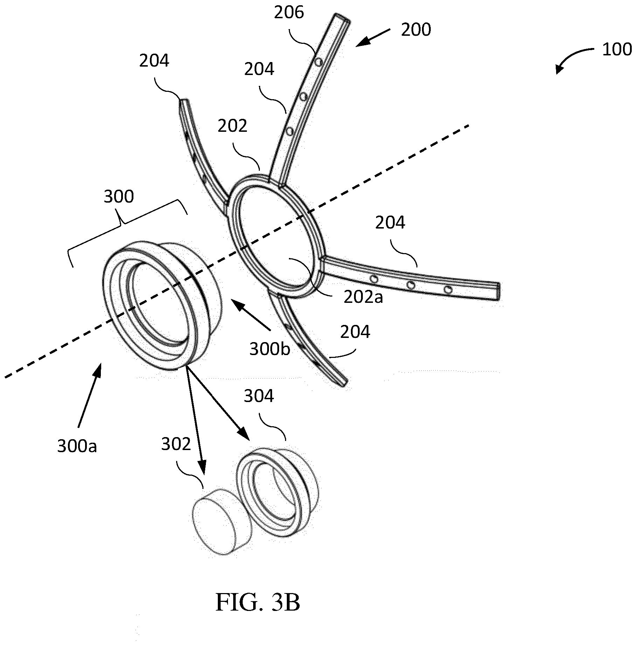

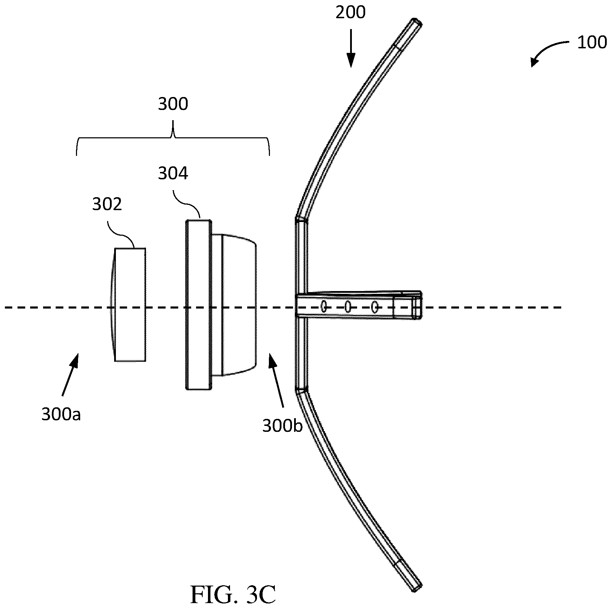

Figs. 3A-3C are overview illustrations, in cross-sectional side (Fig. 3A), exploded perspective (Fig. 3B) and exploded side (Fig. 3C) views, of an exemplary optical stem 300 of a device 100 of the present disclosure, configured to be implanted within the cornea of the eye, and to transmit light from an ambient environment and/or an extraocular light source, through the eye, along an optical path (as marked by a dashed line in Figs. 3B-3C) and onto the retina.

In some embodiments, optical stem 300 defines a light-transparent pathway for transmitting light from the ambient environment and/or an extraocular light source through the eye along an optical path, wherein the pathway defined by the optical stem may comprise at least one optical element, such as an optical lens.

In other embodiments, optical stem 300 includes an image generator incorporating any suitable image display and/or projection technology, such as liquid crystal display (LCD) element that has an illuminator element optically coupled thereto, a LED projector, a laser diode projector, a LED array, and the like. In some embodiments, the image generator is configured to receive an image data signal from an extraocular source (such as an extraocular imaging device), and to generate an optical image signal that is projected in a posterior direction through the light-projecting posterior port, onto the retina of the eye.

In some embodiments, optical stem 300 is configured to project an image beyond its light-projecting posterior port at its posterior end 300b (e.g., when device 100 is implanted in the eye, the image is projected on the retina) based on light entering a light receiving anterior port at anterior end 300a of the optical stem, which defines an anterior- most end of intracorneal implant 200. In some embodiments, the optical stem has a light receiving anterior port at anterior end 300a, closest to the eye's anterior surface. The optical stem extends axially through the cornea to a light-projecting posterior port located at posterior end 300b of the optical stem, closer to the posterior surface of the cornea. In some embodiments, the anterior end 300a of the optical stem defines an anterior-most point of the device 100, and lies just beneath, is substantially at the level of, is flush with, or is slightly proud of, the eye's anterior surface. In some embodiments, the anterior end 300a of the optical stem sits proximately to the eye's anterior surface, fully or partially underneath a thin conjunctival flap layer. In some embodiments, at least a portion of the anterior end of optical stem 300 is exposed through the eye's anterior surface and forms a means for accepting external optical elements of device 100, as shall be further described below.

In some embodiments, the anterior end 300a of the optical stem may comprise an anterior surface which acts as an optical element, e.g., a focusing lens. In some embodiments, as shall be further detailed below, anterior end 300a is configured to receive one or more external optical elements.

In some embodiments, the optical stem 300 includes one or more internal optical and/or imaging components, such as lenses, which assist in providing a focused image on the retina, wherein the optical components of optical stem 300 are optically-aligned with the light-transparent pathway defined by optical stem 300. In other embodiments, the optical stem 300 is simply a transparent path that allows external light or imaging components to pass through and project from the light-projecting posterior port and onto the retina. In other embodiments, optical stem 300 includes an image generator incorporating any suitable image display and/or projection technology, such as liquid crystal display (LCD) element that has an illuminator element optically coupled thereto, a LED projector, a laser diode projector, a LED array, and the like. In some embodiments, the image generator is configured to receive an image data signal from an extraocular source (such as an extraocular imaging device), and to generate an optical image signal that is projected in a posterior direction through the light-projecting posterior port, onto the retina of the eye.

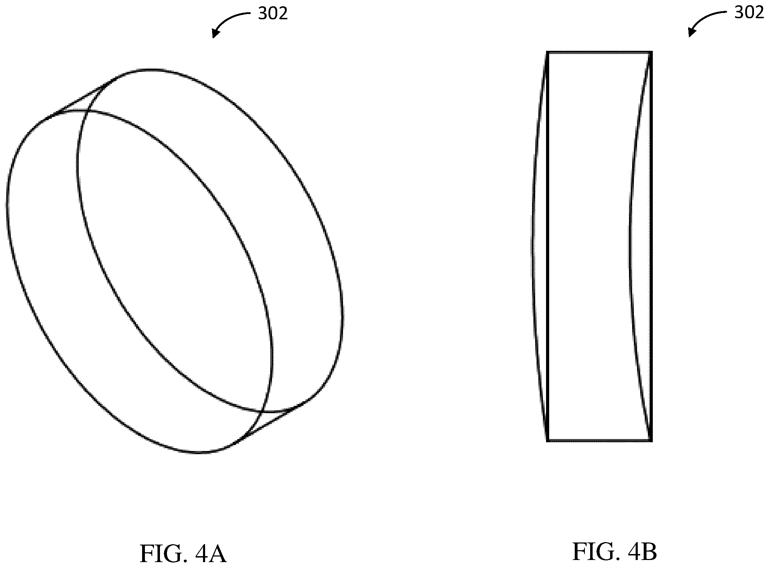

In some embodiments, optical stem 300 comprises at least an inner optical lens 302 (further detailed with reference to Figs. 4A-4B), which may be received within aperture 202a, or optionally be integrally formed with intracorneal implant 200 in lieu of aperture 202a. In some embodiments, inner optical lens 302 may be configured for mounting directly within aperture 202a of intracorneal implant 200. In some embodiments, inner optical lens 302 may be integrally formed with intracorneal implant 200, e.g., approximately centrally located with respect to anchor body 202, wherein in such case, anchor body 202 does not feature the aperture 202a.

In some embodiments, inner optical lens 302 may be configured for mounting within a separate lens housing 304, wherein lens housing 304 is configured to be received within aperture 202a of intracorneal implant 200. Lens housing 304 may comprise a cylinder or tube-like portion that houses inner optical lens 302 through which light passes in order to project images to the retina. The lens housing 304 used herein may be made from any appropriate and biologically compatible material, such as polymethylmethacrylate (PMMA), polysulfone, trimethyl terminated polydimethylsiloxane (PDMS), polysterene-polyisobutylene-polysterene (SIBS) etc. The lens housing 304 may be any size sufficient to accommodate vision, such as to permit an appropriate amount of light to pass through.

In some embodiments, optical lens 302 may be integrally formed with the lens housing 304. In some embodiments, lens housing 304 may be integrally formed with intracorneal implant 200, e.g., approximately centrally located with respect to anchor body 202, wherein in such case, anchor body 202 does not feature the aperture 202a. In some embodiments, optical lens 302 and lens housing 304 may both be integrally formed with intracorneal implant 200, e.g., such that lens housing 304 and optical lens 302 are approximately centrally located with respect to anchor body 202, wherein in such case, anchor body 202 does not feature the aperture 202a.

Reference is now made to Figs. 4A-4B which show, in perspective (Fig. 4A) and side (Fig. 4B) views, an exemplary inner optical lens 302 of the present disclosure, such as may be used in conjunction with device 100.

In some embodiments, inner optical lens 302 may be any convex, concave, monofocal, multifocal, Fresnel, diffractive, prismatic, focusing, or other type of lens that can be used to treat refractive error (such as myopia, hyperopia, or astigmatism). In some embodiments, inner optical lens 302 may be configured, alone or in combination with one or more additional inner and or external lenses, to focus an image onto the retina.

In some embodiments, inner optical lens 302 may be integrally-formed with the central optical part of intracorneal implant 200, e.g., integrally-formed with anchor body 202, such that inner optical lens 302 forms an optical lens within aperture 202a. In other embodiments, inner optical lens 302 is formed separately of intracorneal implant 200, and may be configured to be received and retained within aperture 202a, e.g., by mounting inner optical lens 302 within aperture 202a using a precision fit, a press fit, a click-fit connector, a threaded coupling, an annular groove-and-channel engagement, by fusing, by bonding between an inner peripheral edge of anchor body 202 which defines aperture 202a and a peripheral edge of inner optical lens 302, and/or any other suitable mounting method or technique. In some embodiments, inner optical lens 302 may be configured to be mounted within aperture 202a in a sealed coupling or engagement configured to prevent passage or migration of biological agents or other contaminants through the coupling of inner optical lens 302 and aperture 202a.

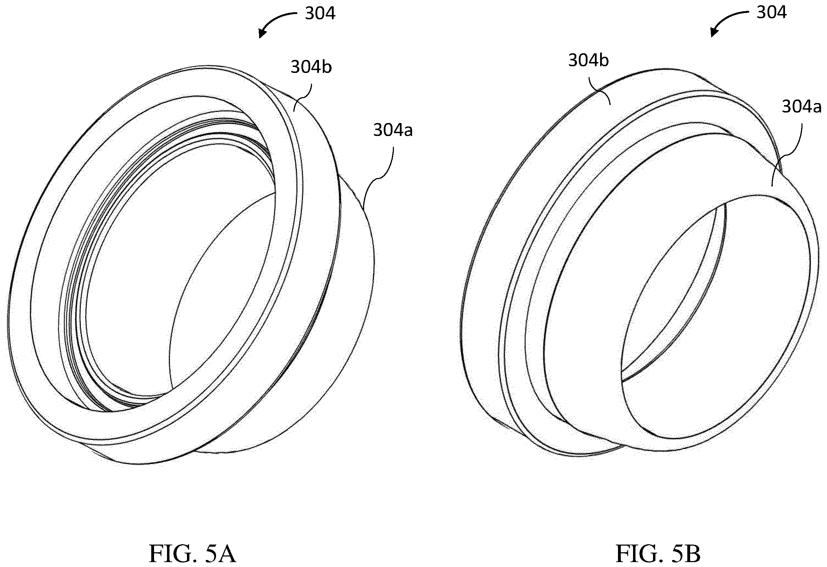

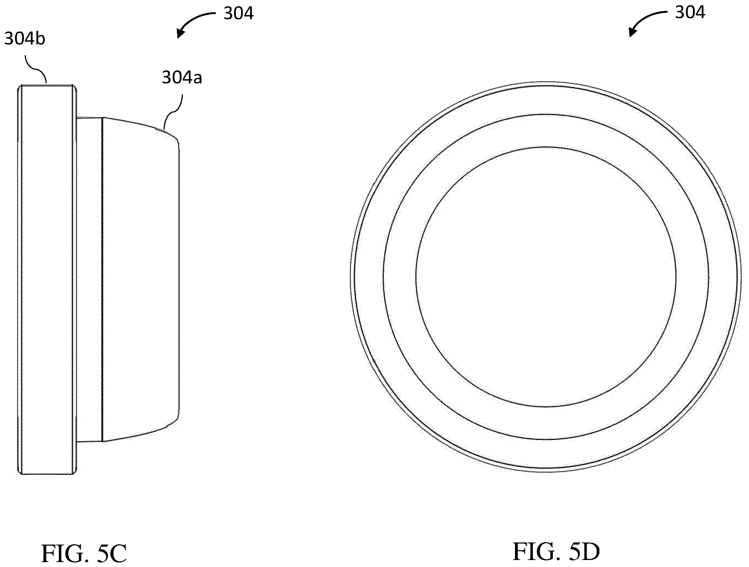

Reference is now made to Figs. 5A-5D which show, in front perspective (Fig. 5A), back perspective (Fig. 5B), side (Fig. 5C), and top (Fig. 5D) views, an exemplary intracorneal lens housing 304 of the present disclosure, such as may be used in conjunction with device 100 and inner optical lens 302.

In some embodiments, lens housing 304 defines any substantially cylindrical or tube-like body configured to be attached or mounted within anchor body 202, e.g., within aperture 202a, and oriented in relation to the eye such that lens housing 304 defines a light-transparent pathway for transmitting light from the ambient environment and/or an extraocular light source through the eye along an optical path. In some embodiments, lens housing 304 may comprise a base portion 304a configured to be received and mounted within aperture 202a of anchor body 202, e.g., using a precision fit, a press fit, a click-fit, an annular groove-and-channel engagement, a threaded engagement, by fusing, by bonding between an inner peripheral edge of anchor body 202 which defines aperture 202a and a peripheral edge of base portion 304a, and/or any other suitable mounting method or technique. In some embodiments, base portion 304a may be configured to be mounted within aperture 202a in a sealed coupling or engagement configured to prevent passage or migration of biological agents or other contaminants through the coupling of base portion 304a and aperture 202a.

In some embodiments, lens housing 304 comprises a larger-diameter lens holding portion 304b configured to receive and retain inner optical lens 302 therein. In some embodiments, lens housing 304 may be configured to be mounted within aperture 202a in a sealed coupling or engagement configured to prevent passage or migration of biological agents or other contaminants through the coupling of lens housing 304 and aperture 202a.

In some embodiments, lens housing 304 may be fashioned of any suitable biologically-compatible material, e.g., any metal, metal alloy (such as nitinol, stainless steel, and the like). In some embodiments, the material can be a polymer, e.g., an acrylate polymer, polymethyl methacrylate (PMMA), polyvinyl chloride (PVC), polyolefin copolymers, fluoropolymers (e.g. PTFE), polyamides, polyimides, polyesters, polyurethane, copolymers thereof, silicone, a cross-linked silicone polymer, and combinations thereof.

It should be noted that any two or more of the components of intracorneal implant 200, including anchor body 202 and haptics 204, and/or the components of optical stem 300, including optical lens 302 and lens housing 304, may be constructed as a single unit or piece, from a single material, using any suitable manufacturing process such as, but not limited to, molding, additive printing, and the like.

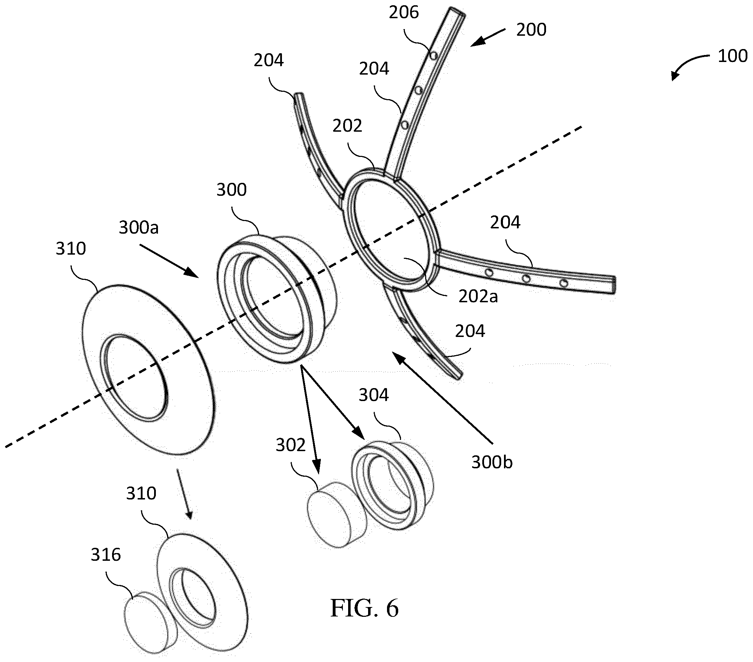

Fig. 6 is an exploded perspective view of an exemplary device 100 of the present disclosure, including intracorneal implant 200 and optical stem 300, configured to transmit light from an ambient environment and/or an extraocular light source, through the eye, along an optical path (as marked by a dashed line) and onto the retina. In some embodiments, device 100 further comprises optional external optical elements which are extra-ocular, including, e.g., end plate 310 and/or external optical lens 316, which may be mounted to anterior end 300a of optical stem 300, as shall be further described below. In some embodiments, external optical lens 316 is integrally-formed with end plate 310.

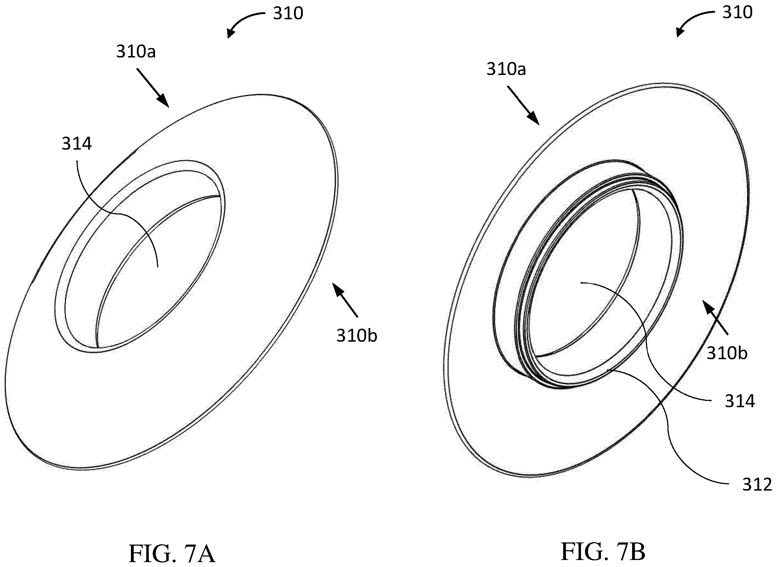

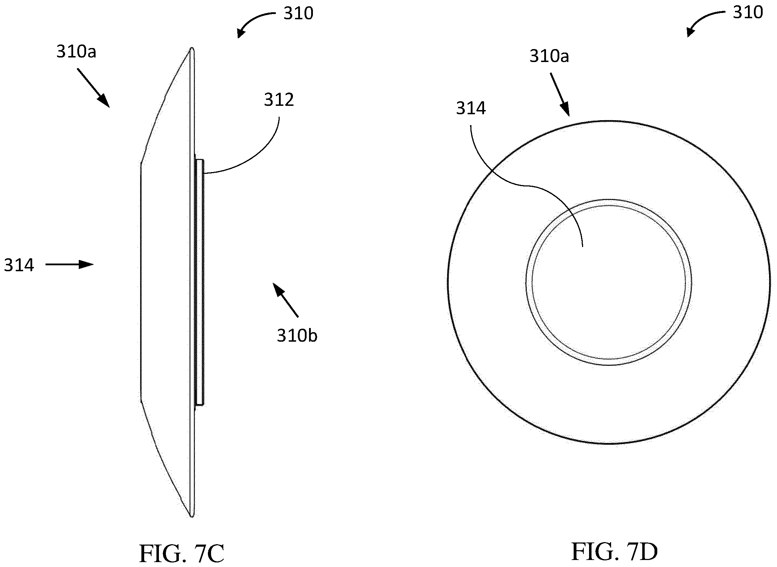

Reference is made to Figs. 7A-7D which show in front perspective (Fig. 7A), back perspective (Fig. 7B), side (Fig. 7C), and top (Fig. 7D) views, an exemplary external anterior end plate 310 which may be further comprised in device 100.

In some embodiments, the anterior end 300a of optical stem 300 may be configured for mounting one or more optional external optical elements of device 100, which may provide device 100 with an external optical lens 316 that may be easily accessible post-surgically for optical adjusting and/or upgrading purposes, e.g., for adjusting a refractive or focusing power of device 100.

As noted above and as shall be further explained below, in some embodiments, upon implantation of device 100, the anterior end 300a of optical stem 300 may configured to be positioned substantially at, is flush with, or slightly proud of, the eye's anterior surface. In some embodiments, the anterior end 300a of the optical stem 300 sits proximately to the eye's surface, fully or partially underneath a thin conjunctival flap layer. In some embodiments, at least a portion of the anterior end 300a of optical stem 300 is exposed to the ambient environment through the eye's anterior surface.

In all such embodiments, the present disclosure provides for an end plate 310 which may be removably and/or adjustably attachable to anterior end 300a, e.g., magnetically removably attachable to anterior end 300a. In some embodiments, end plate 310 may be removably attachable to anterior end 300a, e.g., magnetically removably attachable to anterior end 300a. In some embodiments, end plate 310 may be adjustably attachable to anterior end 300a, for example, by rotating end plate 310 about a central axis thereof to adjust optical properties of an external lens received therein. In some embodiments, end plate 310 may be configured for attaching to anterior end 300a in a manner which ensures a specific desired angular placement of end plate 310 relative to anterior end 300a.

In some embodiments, end plate 310 may define a generally circular curved-surface body having a convexity which substantially conforms to the convexity of the anterior surface of the cornea, with opposing convex anterior face 310a and concave posterior face 310b. In some embodiments, end plate 310 has a centrally-located raised annular flange 312 on its posterior face 310b, which annular flange 312 defines a through opening 314. In some embodiments, end plate 310 may be attachable to anterior end 300a and oriented in relation to the eye, such that end plate 310, through its opening 314, defines a light-transparent pathway for transmitting light from the ambient environment and/or an extraocular light source through the eye along an optical path.

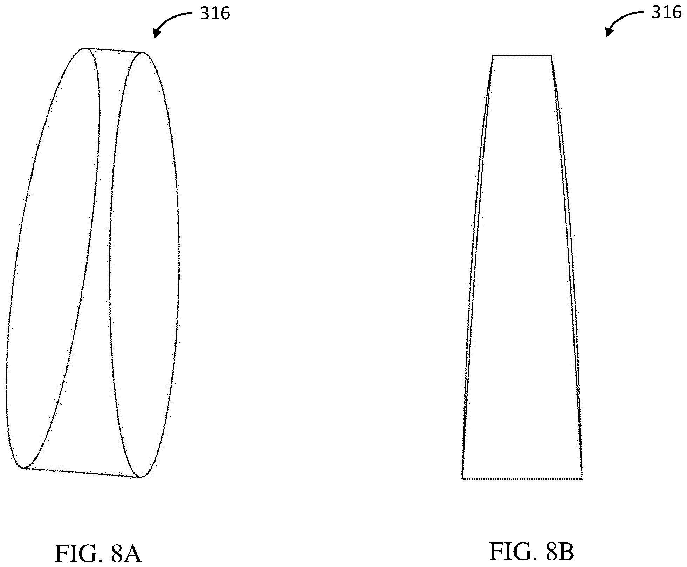

With continued reference to Figs. 7A-7D and reference to Figs. 8A-8B, in some embodiments, anterior end plate 310 may be configured for receiving one or more optical elements therein, such as an external optical lens 316. In some embodiments, external optical lens 316 may be a rotationally asymmetrical lens about its optical axis, configured to adjust the optical properties of lens 316 in relation to an optical axis defined by the optical pathway shown by a dashed line in Fig. 6. External optical lens 316 may be removably received and adjusted within opening 314 of end plate 310 (e.g., by rotating it about its central axis). Alternatively, external optical lens 316 may be fixedly mounted within opening 314 of end plate 310, wherein end plate 310 may be adjusted about its central axis when attached to anterior end 300a of optical stem 300. In some embodiments, external optical lens 316 can be oriented within anterior end plate 310, to adjust and fine-tune the optical properties of lens 316 in relation to an optical axis defined by the optical pathway shown by a dashed line in Fig. 6. In some embodiments, end plate 310 can be adjusted relative to anterior end 300a of optical stem 300 with an appropriate tool, thereby causing lens 316 to change or adjust and fine-tune the optical properties of lens 316 in relation to an optical axis defined by the optical pathway shown by a dashed line in Fig. 6.

In some embodiments, one or more of end plate 310 and external optical lens 316 may be custom-made for a particular patient, to adjust and correct any deficiencies resulting from the optical specification of the implanted device. These parts may then be mounted post-implantation and adjusted in-situ, to modify or fine-tune the refractive and focusing powers of external optical lens 316 according to the need of the particular patient.

In some embodiments, one or more of end plate 310 and external lens 316 may be colored or feature a design configured for cosmesis and/or to impart a natural appearance to the eye (e.g. the anterior end of end plate 310 may be colored to resemble/mimic the natural color of the iris).

It should be noted that end plate 310 and external optical lens 316 may be constructed as a single unit or piece, from a single material, using any suitable manufacturing process such as, but not limited to, molding, additive printing, and the like.

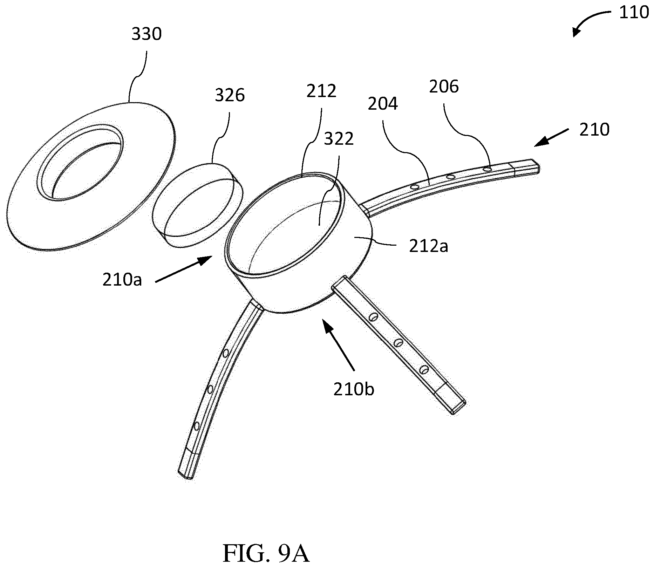

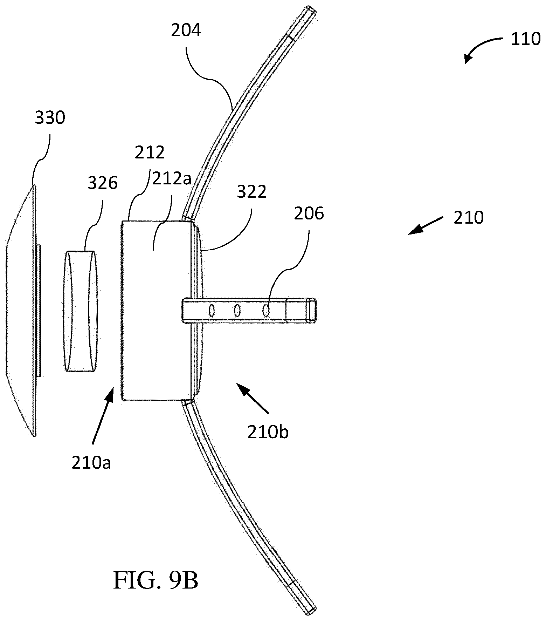

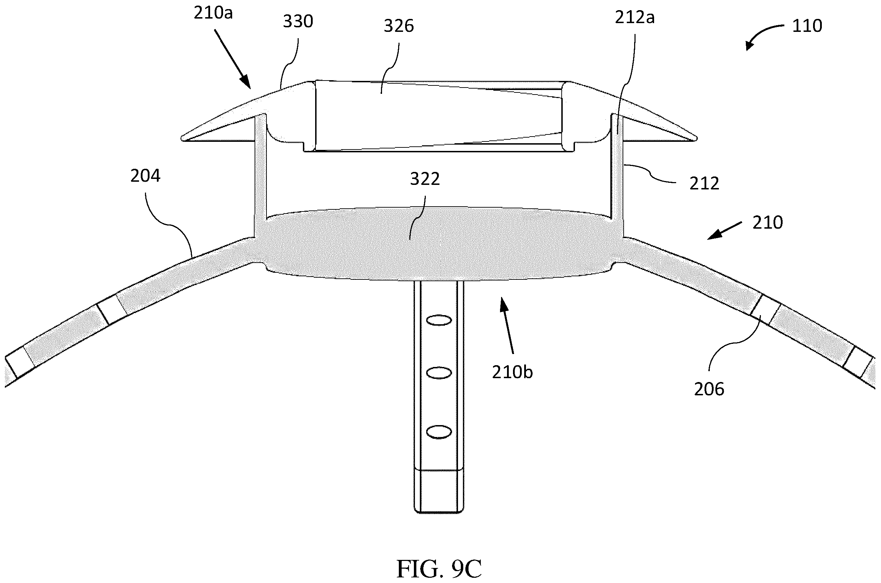

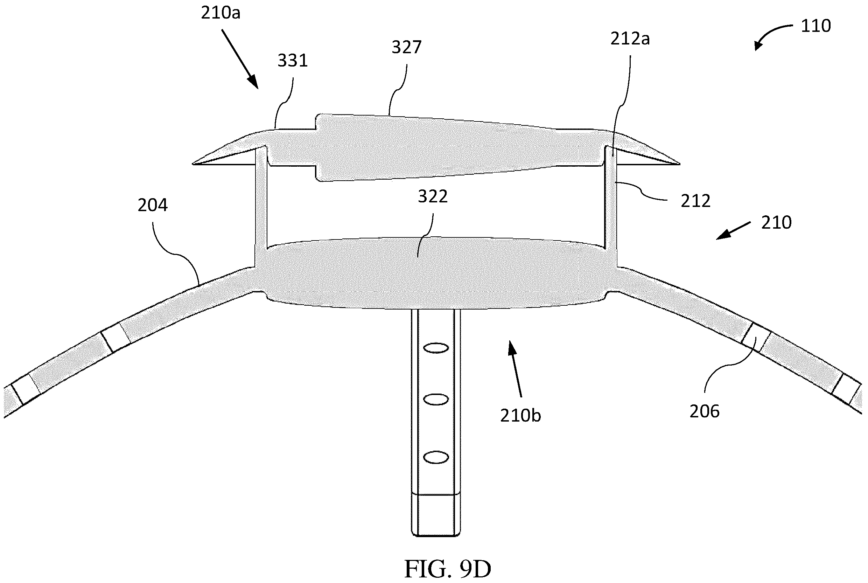

Reference is made to Figs. 9A-9D, which show, in exploded perspective (Fig. 9A), exploded side (Fig. 9B) and cross-sectional (Figs. 9C and 9D) views, an exemplary embodiment of a device 110 of the present disclosure, comprising an intracorneal implant 210, an inner optical lens 322, an end plate 330 and an external optical lens 326. In some embodiments, intracorneal implant 210 is configured for surgical implantation within the cornea of the eye. In some embodiments, intracorneal implant 210 generally comprises a central optical part comprising a lens housing body 212, and a peripheral haptic part comprising two or more haptics 204 similar to those described with reference to Figs. 2A-2E. In some embodiments, lens housing body 212 defines a cylinder or tube-like body that houses lens 322 oriented in vision-facilitating relation to the eye through which light passes in order to project an image to the retina, and comprising an annular flange 212a raised in the anterior direction. In some embodiments, an anterior rim of flange 212a defines an anterior-most end 210a of intracorneal implant 210.

In some embodiments, intracorneal implant 210, including lens housing body 212 and haptics 204, may be fashioned of any suitable biologically-compatible material, e.g., any metal, metal alloy (such as nitinol, stainless steel, and the like). In some embodiments, the material can be a polymer, e.g., an acrylate polymer, polymethyl methacrylate (PMMA), polyvinyl chloride (PVC), polyolefin copolymers, fluoropolymers (e.g. PTFE), polyamides, polyimides, polyesters, polyurethane, copolymers thereof, silicone, a cross-linked silicone polymer, and combinations thereof.

In some embodiments, inner optical lens 322 may be integrally formed with the central optical part of intracorneal implant 210, e.g., integrally-formed with lens housing body 212. In other embodiments, optical lens 322 is formed separately of intracorneal implant 210, and may be configured to be received and retained within lens housing body 212, e.g., by mounting optical lens 322 within lens housing body 212 using a precision fit, a press fit, a click-fit connector, an annular groove-and-channel engagement, a threaded coupling, by fusing, by bonding between an inner peripheral edge of lens housing body 212 and a peripheral edge of optical lens 322, and/or any other suitable mounting method or technique. In some embodiments, optical lens 322 may be configured to be mounted within lens housing body 212 in a sealed coupling or engagement configured to prevent passage or migration of biological agents or other contaminants through the coupling of optical lens 322 and lens housing body 212.

In some embodiments, upon implantation, the anterior end 210a of the lens housing body 212, which defines an anterior-most point of the device 110, lies just beneath, is substantially at the level of, is flush with, or is slightly proud of, the eye's anterior surface. In some embodiments, the anterior end 210a of the lens housing body 212 sits proximately to the eye's anterior surface, fully or partially underneath a thin conjunctival flap layer. In some embodiments, at least a portion of the anterior end 210a of lens housing body 212 is exposed through the eye's anterior surface and forms a means for accepting external optical elements of device 110, such as end plate 330 and/or one or more external lenses, which may thus be easily accessible post-surgically for adjusting and/or upgrading purposes, e.g., for adjusting a refractive or focusing power of device 110.

Fig. 9D shows a cross-sectional view of an exemplary variation of device 110, wherein an external lens 327 is integrally-formed with an end plate 331.

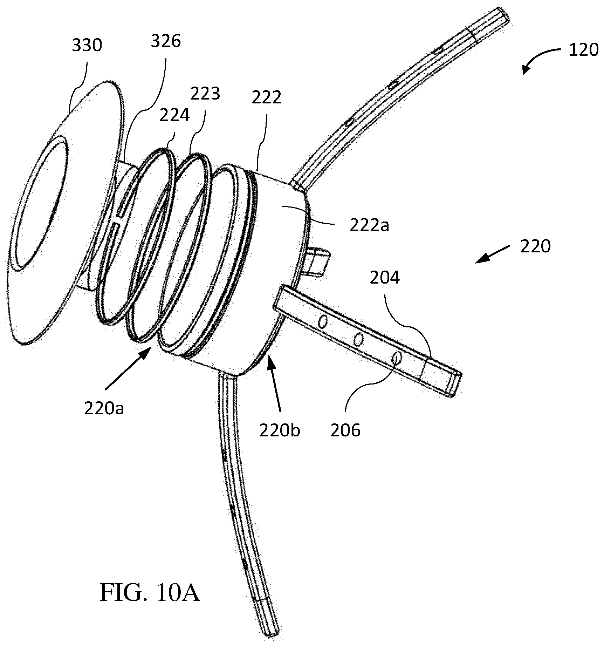

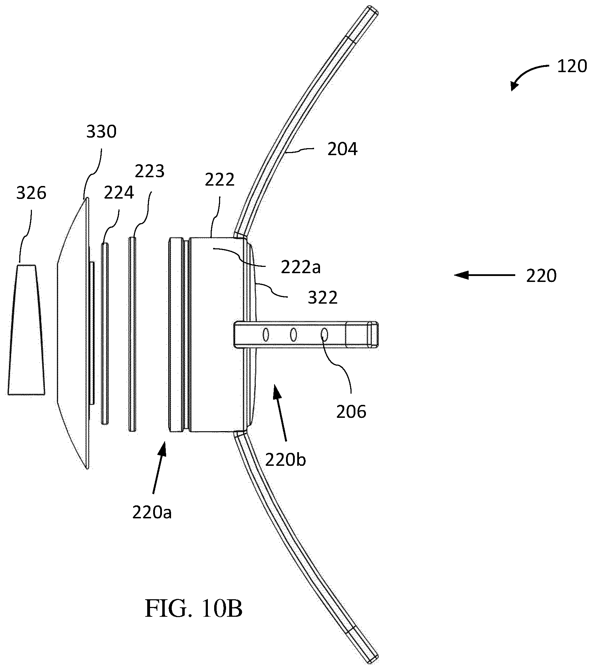

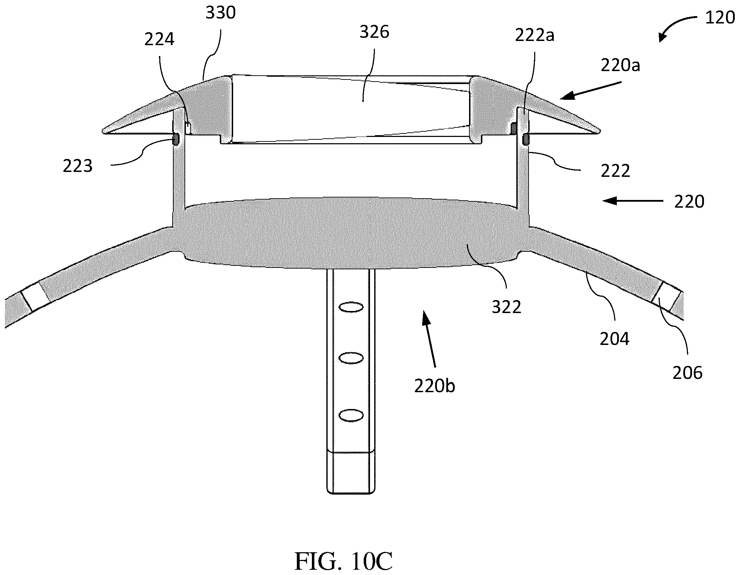

Reference is made to Figs. 10A-10C, which show, in exploded perspective (Fig. 10A), exploded side (Fig. 10B) and cross-sectional (Fig. 10C) views, an exemplary embodiment of device 120 of the present disclosure, comprising an intracorneal implant 220, an inner optical lens 322, an end plate 330 and an external optical lens 326. In some embodiments, intracorneal implant 220 is configured for surgical implantation within the cornea of the eye. In some embodiments, intracorneal implant 220 generally comprises a central optical part comprising a lens housing body 222, and a peripheral haptic part comprising two or more haptics 204 similar to those described with reference to Figs. 2A-2E. In some embodiments, lens housing body 222 defines a cylinder or tube-like body that houses lens 322 oriented in relation to the eye through which light passes in order to project an image to the retina, and comprising an annular flange 222a raised in the anterior direction. In some embodiments, an anterior edge or rim of flange 222a defines an anterior-most end 220a of intracorneal implant 220.

In some embodiments, intracorneal implant 220, including lens housing body 222 and haptics 204, may be fashioned of any suitable biologically-compatible material, e.g., any metal, metal alloy (such as nitinol, stainless steel, and the like). In some embodiments, the material can be a polymer, e.g., an acrylate polymer, polymethyl methacrylate (PMMA), polyvinyl chloride (PVC), polyolefin copolymers, fluoropolymers (e.g. PTFE), polyamides, polyimides, polyesters, polyurethane, copolymers thereof, silicone, a cross-linked silicone polymer, and combinations thereof.

In some embodiments, inner optical lens 322 may be integrally formed with the central optical part of intracorneal implant 220, e.g., integrally-formed with lens housing body 222. In other embodiments, optical lens 322 is formed separately of intracorneal implant 220, and may be configured to be received and retained within lens housing body 222, e.g., by mounting optical lens 322 within lens housing body 222 using a precision fit, a press fit, a click-fit connector, an annular groove-and-channel engagement, a threaded coupling, by fusing, by bonding between an inner peripheral edge of lens housing body 222 and a peripheral edge of optical lens 322, and/or any other suitable mounting method or technique. In some embodiments, optical lens 322 may be configured to be mounted within lens housing body 222 in a sealed coupling or engagement configured to prevent passage or migration of biological agents or other contaminants through the coupling of optical lens 322 and lens housing body 222.

In some embodiments, upon implantation, the anterior end 220a of the lens housing body 222, which defines an anterior-most point of the device 120, lies just beneath, is substantially at the level of, is flush with, or is slightly proud of, the eye's anterior surface. In some embodiments, the anterior end 220a of the lens housing body 222 sits proximately to the eye's anterior surface, fully or partially underneath a thin conjunctival flap layer. In some embodiments, at least a portion of the anterior end of lens housing body 222 is exposed through the eye's anterior surface and forms a means for accepting external optical elements of device 120, such as end plate 330 and/or one or more external optical lenses 326, that may thus be easily accessible post-surgically for adjusting and/or upgrading purposes, e.g., for adjusting a refractive or focusing power of device 120.

In some embodiments, device 120 further comprises a magnetic coupling system configured for magnetically removably attaching end plate 330 to anterior end 220a. In some embodiments, end plate 330 may be removably and rotatably attachable to anterior end 220a, e.g., magnetically removably and rotatably attachable to anterior end 220a. In some embodiments, flange 222a may comprise an annular magnetic ring 223 located proximately to an anterior end of flange 222a. Similarly, end plate 330 may comprise an annular magnetic ring 224 located proximately to a light-projecting posterior port of end plate 330. Thus, when end plate 330 is positioned about anterior end 220a of device 120, magnetic rings 223 and 224 attract each other and act to secure end plate 330 to the anterior end 220a of device 120.

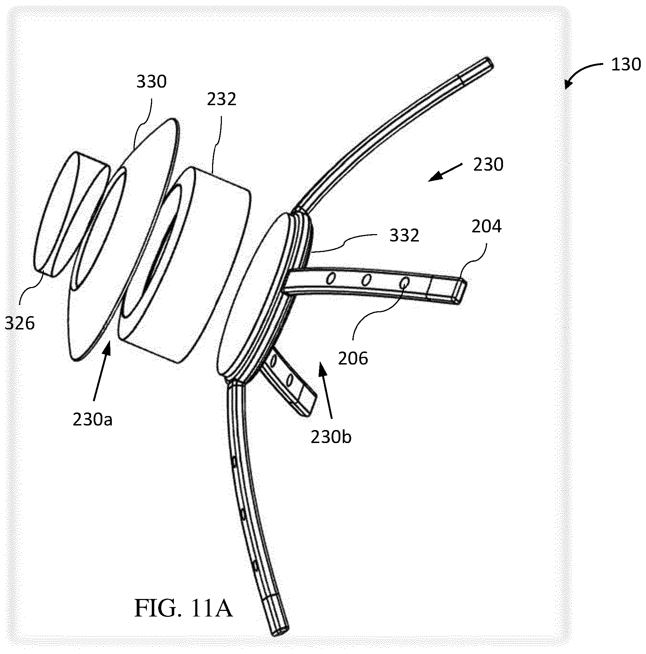

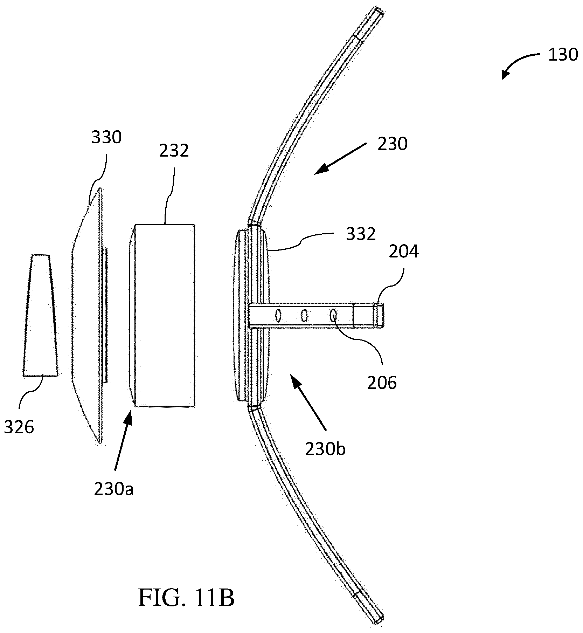

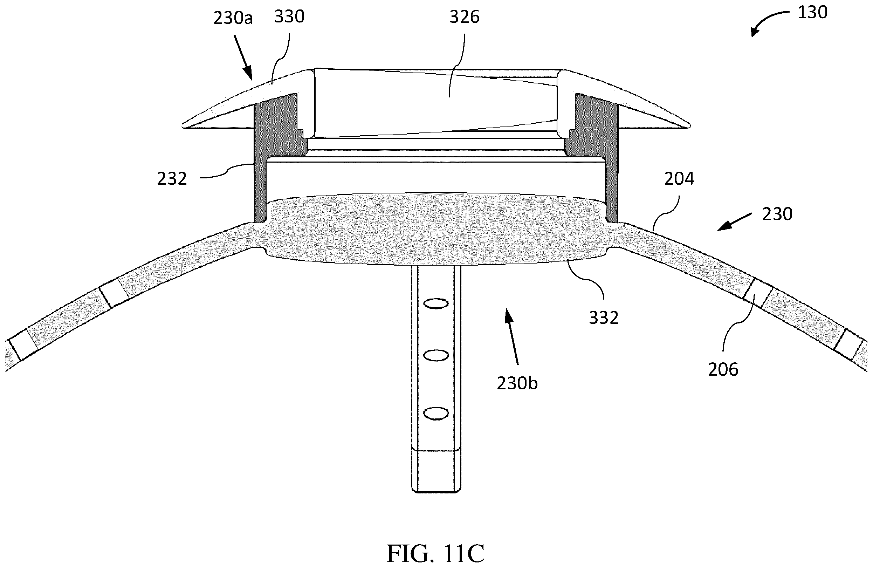

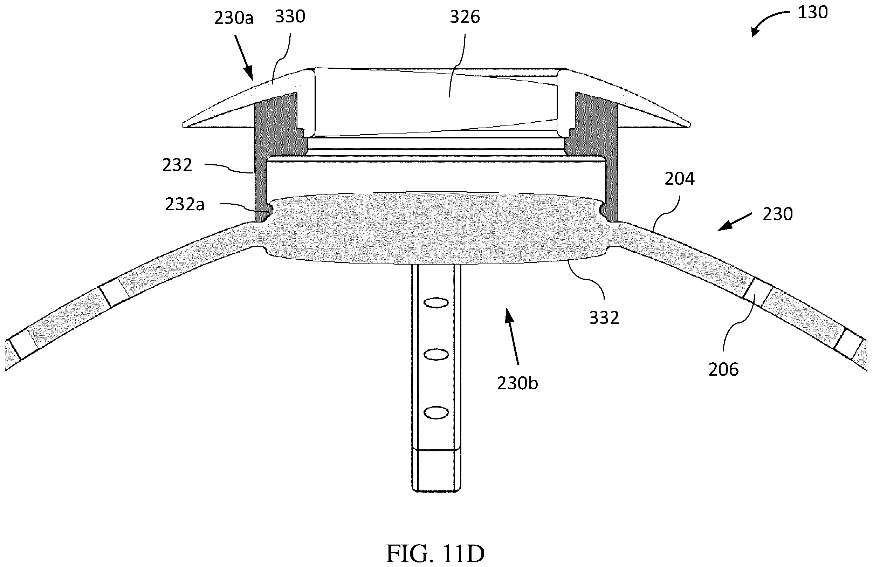

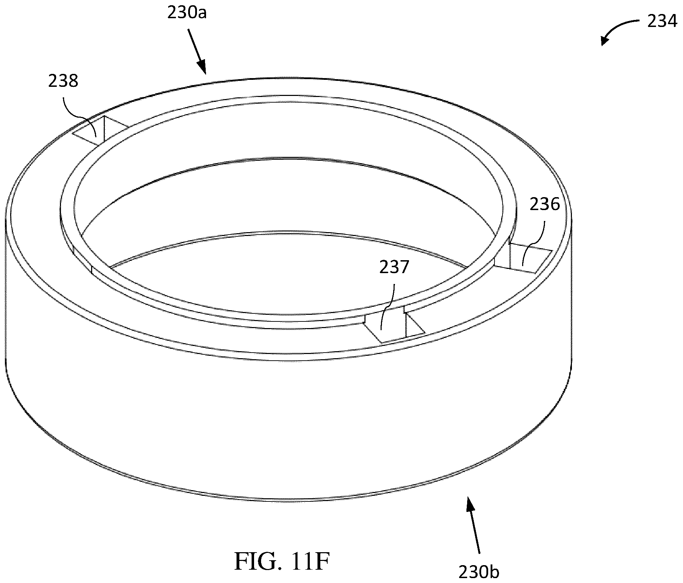

Reference is made to Figs. 11A-11D, which show, in exploded perspective (Fig. 11A), exploded side (Fig. 1 IB) and cross-sectional (Figs. 11C and 1 ID) views, an exemplary embodiment of device 130 of the present disclosure, comprising an intracorneal implant 230, an inner optical lens 332, an end plate 330, and an external optical lens 326. In some embodiments, intracorneal implant 230 is configured for surgical implantation within the cornea of the eye. In some embodiments, intracorneal implant 230 generally comprises a central optical part comprising an inner optical lens 332 and a peripheral haptic part comprising two or more haptics 204 similar to those described with reference to Figs. 2A-2E. In some embodiments, inner optical lens 332 may be integrally formed with the central part of intracorneal implant 230. In other embodiments, optical lens 332 is formed separately of intracorneal implant 230, and may be configured to be received and retained within the central optical part of intracorneal implant 230, e.g., by mounting optical lens 332 using a precision fit, a press fit, an annular groove-and-channel engagement, a click-fit connector, a threaded coupling, by fusing, by bonding between an inner peripheral edge of the central optical part of intracorneal implant 230 and a peripheral edge of optical lens 332, and/or any other suitable mounting method or technique. In some embodiments, optical lens 332 may be configured to be mounted within the central part of intracorneal implant 230 in a sealed coupling or engagement configured to prevent passage or migration of biological agents or other contaminants through the coupling of optical lens 332 and the central optical part of intracorneal implant 230.

In some embodiments, device 130 further comprises a separate tubular extension 232 which defines a cylinder or tube-like body that extends in the anterior direction from the central optical part of intracorneal implant 230, oriented in relation to the eye through which light passes in order to project images to the retina. In some embodiments, an anterior edge or rim of tubular extension 232 defines an anterior-most end 230a of intracorneal implant 230.

In some embodiments, intracorneal implant 230, including tubular extension 232 and haptics 204, may be fashioned of any suitable biologically-compatible material, e.g., any metal, metal alloy (such as nitinol, stainless steel, and the like). In some embodiments, the material can be a polymer, e.g., an acrylate polymer, polymethyl methacrylate (PMMA), polyvinyl chloride (PVC), polyolefin copolymers, fluoropolymers (e.g. PTFE), polyamides, polyimides, polyesters, polyurethane, copolymers thereof, silicone, a cross-linked silicone polymer, and combinations thereof.

In some embodiments, upon implantation, the anterior end 230a of the tubular extension 232, which defines an anterior-most point of the intracorneal implant 230, lies just beneath, is substantially at the level of, is flush with, or is slightly proud of, the eye's anterior surface. In some embodiments, the anterior end 230a of the tubular extension 232 sits proximately to the eye's anterior surface, fully or partially underneath a thin conjunctival flap layer. In some embodiments, at least a portion of the anterior end of tubular extension 232 is exposed through the eye's anterior surface and forms a means for accepting external optical elements of device 130, such as end plate 330 and/or one or more external optical lenses 326, that may thus be easily accessible post-surgically for adjusting and/or upgrading purposes, e.g., for adjusting a refractive or focusing power of device 130.

In some embodiments, tubular extension 232 may be coupled to the central optical part of intracorneal implant 230 such that a through inner bore defined by tubular extension 232 is oriented in a relation to the eye to create a light-transparent pathway for transmitting light therethrough. In some embodiments, tubular extension 232 may be coupled to the central optical part of intracorneal implant 230 using, e.g., a precision fit, a press fit, a click-fit, an annular groove- and-channel engagement, a threaded coupling, by fusing, by bonding between an inner peripheral edge of tubular extension 232 and a peripheral edge of the central optical part of intracorneal implant 230, and/or any other suitable mounting method or technique. In some embodiments, as can be seen in the variations illustrated in cross-sectional view in Fig. 1 ID, tubular extension 232 may be coupled to the central optical part of intracorneal implant 230 by a click system comprising, e.g., an annular protrusion 232a on an inner peripheral edge of tubular extension 232, configured to engage a corresponding annular groove on a peripheral edge of the central optical part of intracorneal implant 230 (or vice-versa). In some embodiments, tubular extension 232 may be coupled to the central optical part of intracorneal implant 230 in a sealed coupling or engagement configured to prevent passage or migration of biological agents or other contaminants through the coupling of tubular extension 232 and the central optical part of intracorneal implant 230.

In some embodiments, device 130 further comprises a magnetic coupling system configured for magnetically removably attaching end plate 330 to anterior end 230a. In some embodiments, end plate 330 may be removably and rotatably attachable to anterior end 230a, e.g., magnetically removably and rotatably attachable to anterior end 230a. In some embodiments, tubular extension 232 may comprise an annular magnetic ring located proximately to an anterior end of tubular extension 232. Similarly, end plate 330 may comprise an annular magnetic ring located proximately to a light-projecting posterior port of end plate 330. Thus, when end plate 330 is positioned about anterior end 230a of device 130, the respective magnetic rings attract each other and act to secure end plate 330 to the anterior end 230a of device 130.

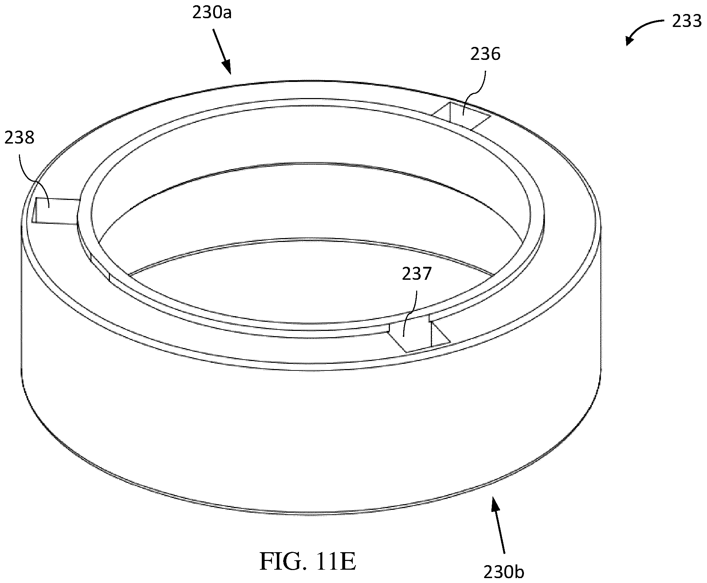

Figs. 11E-11G show exemplary tubular extensions 233, 234, 235 comprising a magnetic coupling system configured for magnetically removably attaching end plate 330 to anterior end 230a.

In some embodiments, exemplary tubular extension 233 (Fig. 1 IE) may comprise one or more, e.g., 1, 2, 3, 4, 5, 6 or more, e.g., magnets 236, 237, 238 substantially equally annularly-spaced an anterior end of tubular extension 233. Similarly, end plate 330 may comprise a corresponding set of annularly-spaced magnets located proximately to a light projecting posterior port of end plate 330. Thus, when end plate 330 is positioned about anterior end 230a of device 130, the respective magnets attract each other and act to secure end plate 330 to the anterior end 230a of device 130.

In some embodiments, exemplary tubular extension 234 (Fig. 1 IF) may comprise one or more, e.g., 1, 2, 3, 4, 5, 6 or more, e.g., magnets 236, 237, 238 that are annularly-spaced an anterior end of tubular extension 233 in a non-symmetrical manner. Similarly, end plate 330 may comprise a corresponding set of magnets located proximately to a light projecting posterior port of end plate 330. The non-symmetrical arrangement is configured to ensure a specific desired angular placement of end plate 330 relative to tubular extension 234. Thus, when end plate 330 is positioned about anterior end 230a of device 130, the respective magnets attract each other and act to secure end plate 330 to the anterior end 230a of device 130 in the specific desired angular placement.

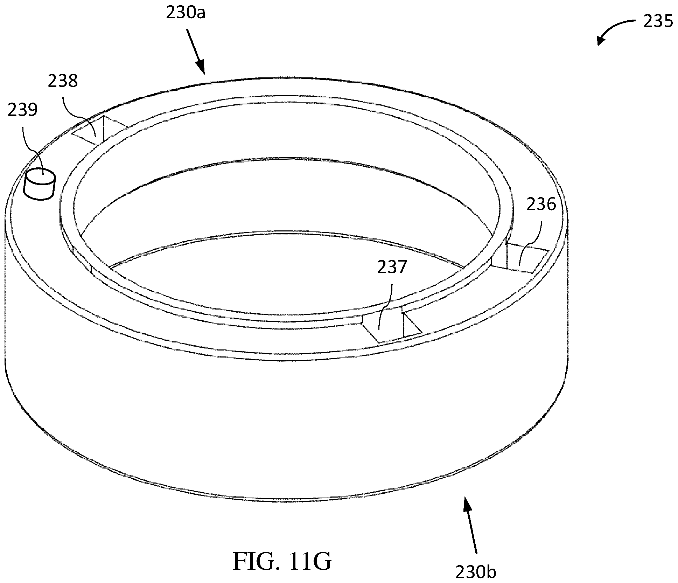

In some embodiments, exemplary tubular extension 235 (Fig. 11G) may comprise one or more, e.g., 1, 2, 3, 4, 5, 6 or more, e.g., magnets 236, 237, 238 annularly-spaced an anterior end of tubular extension 235. Similarly, end plate 330 may comprise a corresponding set of annularly-spaced magnets located proximately to a light-projecting posterior port of end plate 330. In addition, tubular extension 235 may comprise an indexing pin 239 projecting from an anterior end of tubular extension 235, configured to engage a corresponding opening is located proximately to a light-projecting posterior port of end plate 330. The engagement of indexing pin 239 and the corresponding opening is configured to lock a specific desired angular placement of end plate 330 relative to tubular extension 235. Thus, when end plate 330 is positioned about anterior end 230a of device 130, the respective magnets attract each other and act to secure end plate 330 to the anterior end 230a of device 130, while indexing pin 239 ensures the specific desired angular placement.

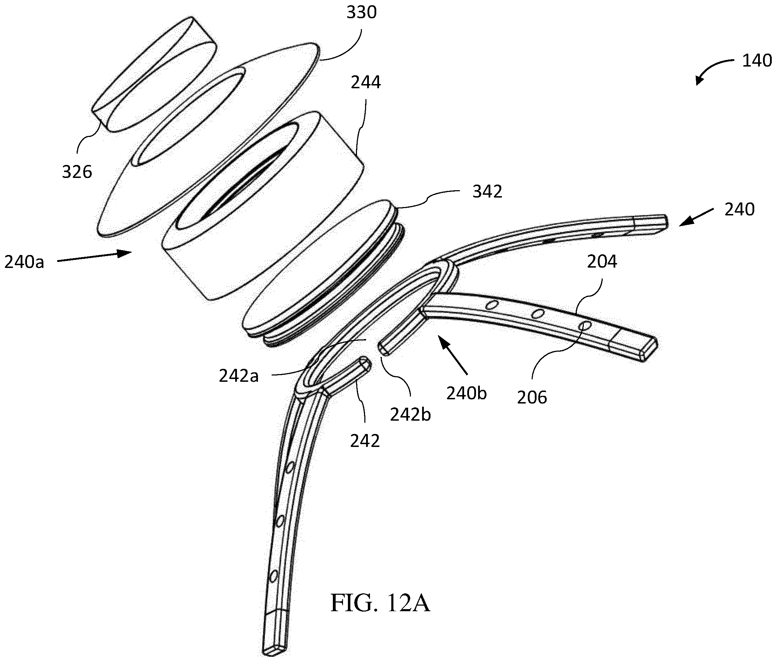

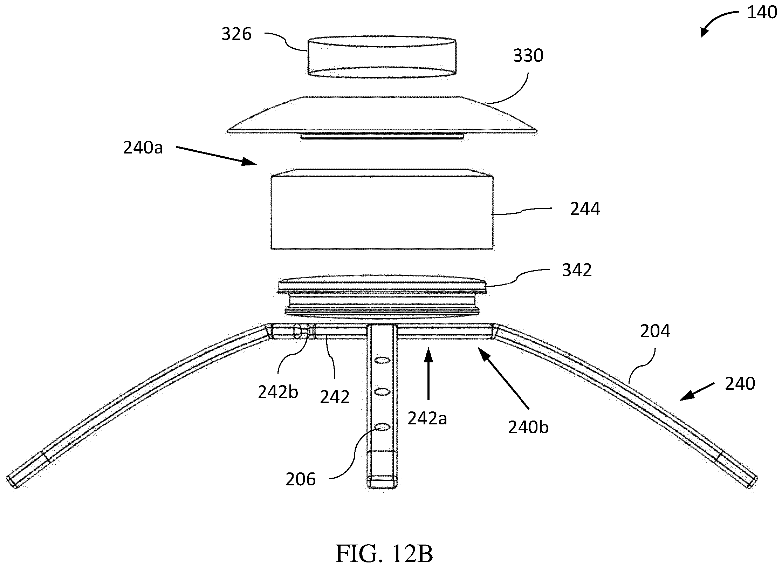

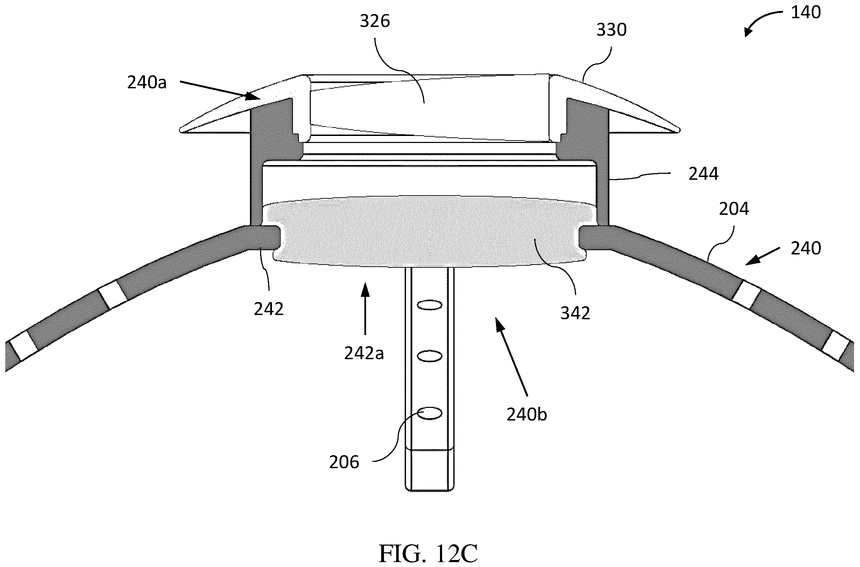

Reference is made to Figs. 12A-12C, which show, in exploded perspective (Fig. 12A), exploded side (Fig. 12B) and cross-sectional (Fig. 12C) views, an exemplary embodiment of device 140 of the present disclosure, comprising an intracorneal implant 240, an inner optical lens 342, an end plate 330 and/or an external optical lens 326. In some embodiments, intracorneal implant 240 is configured for surgical implantation within the cornea of the eye. In some embodiments, intracorneal implant 240 generally comprises a central optical part comprising an annular anchor body 242 which defines a central aperture 242a configured to receive and retain an inner optical lens 342 therein. In some embodiments, anchor body 242 may define a circumferentially-continuous annular ring or may have an opening portion 242b at a side thereof. In some embodiments, the peripheral haptic part comprises two or more haptics 204 similar to those described with reference to Figs. 2A-2E.

Anchor body 242 can be a ring, a rim, a cannula, a cylinder, or any other suitable three-dimensional shape which can define a circular or cylindrical aperture and to which haptics 204 can be attached.

In some embodiments, inner optical lens 342 may be integrally-formed with the central optical part of intracorneal implant 240, e.g., integrally-formed with anchor body 242, such that optical lens 342 forms an optical lens within aperture 242a. In other embodiments, optical lens 342 is formed separately of intracorneal implant 240, and may be configured to be received and retained within aperture 242a, e.g., by mounting optical lens 342 within aperture 242a using a precision fit, a press fit, a click-fit, a threaded coupling, an annular groove-and-channel engagement, by fusing, by bonding between an inner peripheral edge of anchor body 242 which defines aperture 242a and a peripheral edge of optical lens 342, and/or any other suitable mounting method or technique. In some embodiments, optical lens 342 may be configured to be mounted within aperture 242a in a sealed coupling or engagement configured to prevent passage or migration of biological agents or other contaminants through the coupling of optical lens 342 and aperture 242a.

In some embodiments, inner optical lens 342 may be integrally formed with the central optical part of intracorneal implant 240. In other embodiments, optical lens 342 is formed separately of intracorneal implant 240, and may be configured to be received and retained within the central optical part of intracorneal implant 240, e.g., by mounting optical lens 342 using a precision fit, a press fit, a click fit, a threaded coupling, by fusing, by bonding between an inner peripheral edge of the central optical part of intracorneal implant 240 and a peripheral edge of optical lens 342, and/or any other suitable mounting method or technique. In some embodiments, optical lens 342 may be configured to be mounted within the central optical part of intracorneal implant 240 in a sealed coupling or engagement configured to prevent passage or migration of biological agents or other contaminants through the coupling of optical lens 342 and the central optical part of intracorneal implant 240.

In some embodiments, device 140 further comprises a separate tubular extension 244 which defines a cylinder or tube-like body that extends in the anterior direction from the central optical part of intracorneal implant 240, oriented in relation to the eye through which light passes in order to project images to the retina. In some embodiments, an anterior edge or rim of tubular extension 244 defines an anterior-most end 240a of intracorneal implant 240.

In some embodiments, intracorneal implant 240, including anchor body 242, and haptics 204, may be fashioned of any suitable biologically-compatible material, e.g., any metal, metal alloy (such as nitinol, stainless steel, and the like). In some embodiments, the material can be a polymer, e.g., an acrylate polymer, polymethyl methacrylate (PMMA), polyvinyl chloride (PVC), polyolefin copolymers, fluoropolymers (e.g. PTFE), polyamides, polyimides, polyesters, polyurethane, copolymers thereof, silicone, a cross-linked silicone polymer, and combinations thereof.

In some embodiments, upon implantation, the anterior end 240a of the tubular extension 244, which defines an anterior-most point of the intracorneal implant 240, lies just beneath, is substantially at the level of, is flush with, or is slightly proud of, the eye's anterior surface. In some embodiments, the anterior end 240a of the tubular extension 244 sits proximately to the eye's anterior surface, fully or partially underneath a thin conjunctival flap layer. In some embodiments, at least a portion of the anterior end of tubular extension 244 is exposed through the eye's anterior surface and forms a means for accepting external optical elements of device 140, such as end plate 330 and/or one or more external optical lenses 326, that may thus be easily accessible post-surgically for adjusting and/or upgrading purposes, e.g., for adjusting a refractive or focusing power of device 140.

In some embodiments, tubular extension 244 may be coupled to the central optical part of intracorneal implant 240 such that a through inner bore defined by tubular extension 244 is oriented in relation to the eye to create a light-transparent pathway for transmitting light therethrough. In some embodiments, tubular extension 244 may be coupled to the central optical part of intracorneal implant 240 using, e.g., a precision fit, a press fit, a threaded coupling, a click-fit connector, by fusing, by bonding between an inner peripheral edge of tubular extension 244 and a peripheral edge of the central optical part of intracorneal implant 240, and/or any other suitable mounting method or technique. In some embodiments, tubular extension 244 may be coupled to the central optical part of intracorneal implant 240 by circumferentially-engaging an anterior portion of optical lens 342 using, e.g., a precision fit, a press fit, a threaded coupling, by an annular channel-and-groove engagement, by a click system, by fusing, by bonding between an inner peripheral edge of tubular extension 244 and a peripheral edge of the central optical part of intracorneal implant 240, and/or any other suitable mounting method or technique. In some embodiments, tubular extension 244 may be coupled to the central optical part of intracorneal implant 240 in a sealed coupling or engagement configured to prevent passage or migration of biological agents or other contaminants through the coupling of tubular extension 244 and the central optical part of intracorneal implant 240.

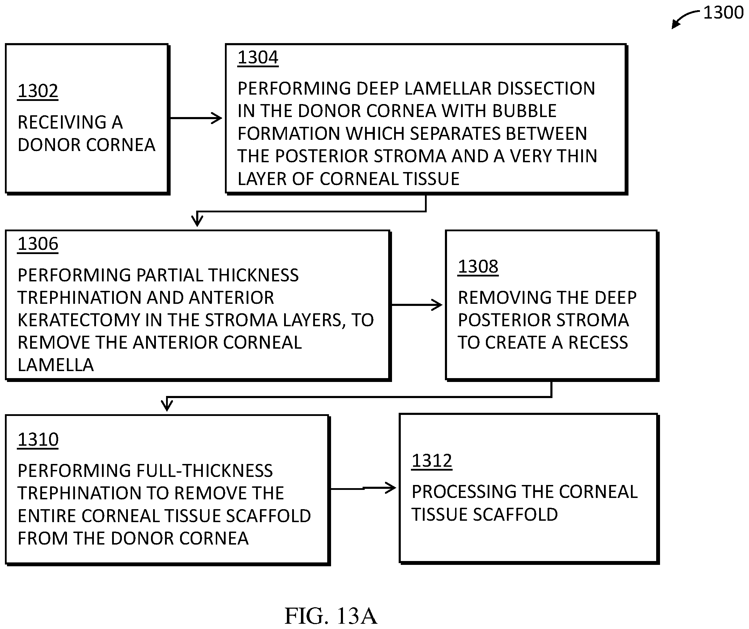

Reference is made to Figs. 13A-13F, which illustrate the functional steps in a process 1300 for the preparation of an exemplary pre-processed corneal tissue scaffold derived from a donor cornea.

The particular series of steps depicted in conjunction with process 1300 is not intended to be limiting. Other sequences of steps may also be performed according to alternative embodiments without departing from the techniques of this disclosure. For example, alternative embodiments of the present invention may perform the steps outlined above in a different order or in parallel. Moreover, the individual steps may include multiple sub-steps that may be performed in various sequences as appropriate. Furthermore, additional steps may be added or removed depending on the particular applications.

In some embodiments, the corneal tissue scaffold may be configured for use in conjunction with a device of the present disclosure, such as any one or more of devices 100 (described with reference to Figs. 1A-1C, 3D-3E, and 6), device 110 (described with reference to Figs. 9A-9D), 120 (described with reference to Figs. 10A-10C), 130 (described with reference to Figs. 11A-11D), and/or 140 (described with reference to Figs. 12A-12C).

In some embodiments, a corneal tissue scaffold of the present disclosure may comprise various compositions of corneal layers at different regions thereof. For example, the corneal tissue scaffold of the present disclosure may comprise, at a central substantially transparent region thereof, a very thin layer of corneal tissue (e.g., having a thickness of approximately 10-50µm), that is substantially transparent to light. In some embodiments, the very thin layer comprises at least the pre-Descemet layer. In some embodiments, the very thin layer comprises the pre-Descemet layer in addition to the Descemet membrane. In some embodiments, the very thin layer comprises the pre-Descemet layer, the Descemet membrane, and the endothelium. Accordingly, a generally circular central region or portion of the corneal tissue scaffold may comprise (i) only the pre-Descemet layer, or (ii) the pre-Descemet layer in combination with the Descemet membrane, or (iii) the pre-Descemet layer in combination with the Descemet membrane and the endothelium. In some embodiments, the central region of the corneal tissue scaffold is thus configured to be substantially transparent to light, and to be located so as to fall along an optical path of a device of the present disclosure configured to transmit light from an ambient environment and/or an external light source, through the eye, along an optical path and onto the retina. In some embodiments, regions of the corneal tissue scaffold surrounding the central substantially transparent region, may have a different composition of corneal layers, comprising at least some of the stromal layer, pre-Descemet layer, the Descemet membrane, and/or the endothelium.

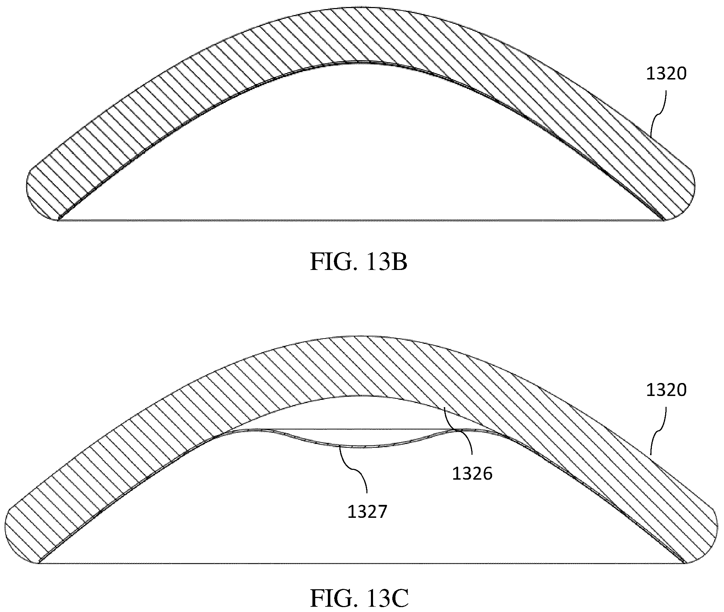

With continued reference to Fig. 13 A, in step 1302, and as shown in Fig. 13B, process 1300 may begin with in step 1302, by receiving a donor cornea 1320.

In step 1304, as shown in Fig. 13C, a deep lamellar dissection (e.g. pneumatic, hydro-assisted, viscoelastic-assisted and/or manual dissection) is performed in the donor cornea 1320 with a bubble 1326 formation which separates between the posterior stroma and a very thin layer of corneal tissue 1327 having a thickness of approximately 10-50µm, and substantially transparent to light). In some embodiments, thin layer 1327 comprises at least the pre-Descemet layer. In some embodiments, thin layer 1327 comprises the pre-Descemet layer in addition to the Descemet membrane. In some embodiments, thin layer 1327 comprises the pre-Descemet layer, the Descemet membrane, and the endothelium.

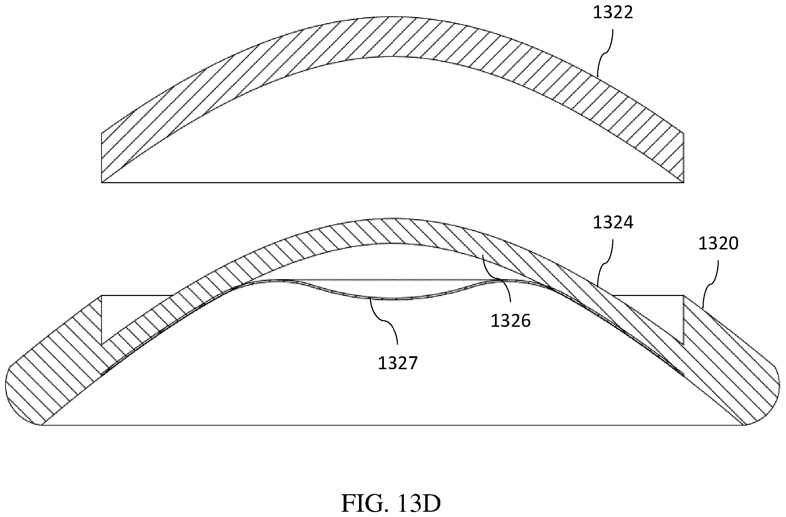

In Step 1306, as shown in Fig. 13D, a partial thickness trephination and anterior keratectomy is performed in the stromal layers of donor cornea 1320, to remove the anterior corneal lamella 1322 leaving the posterior corneal layers 1324.

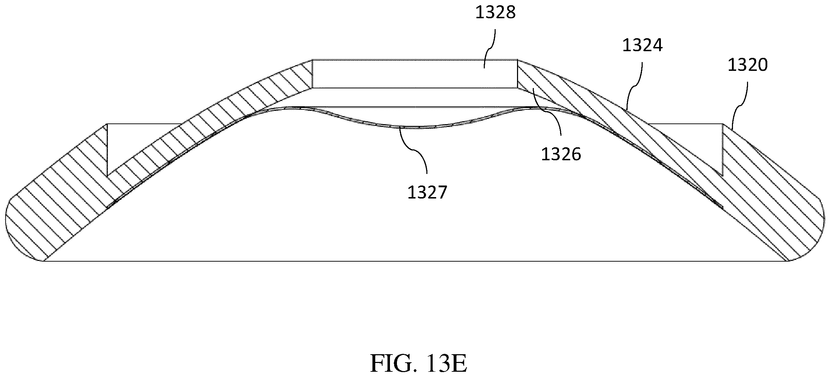

In step 1308, as shown in Fig. 13E, the deep posterior stroma (e.g., within or around the region above the bubble 1326) is removed to create a recess 1328, while leaving thin posterior layer 1327 intact.

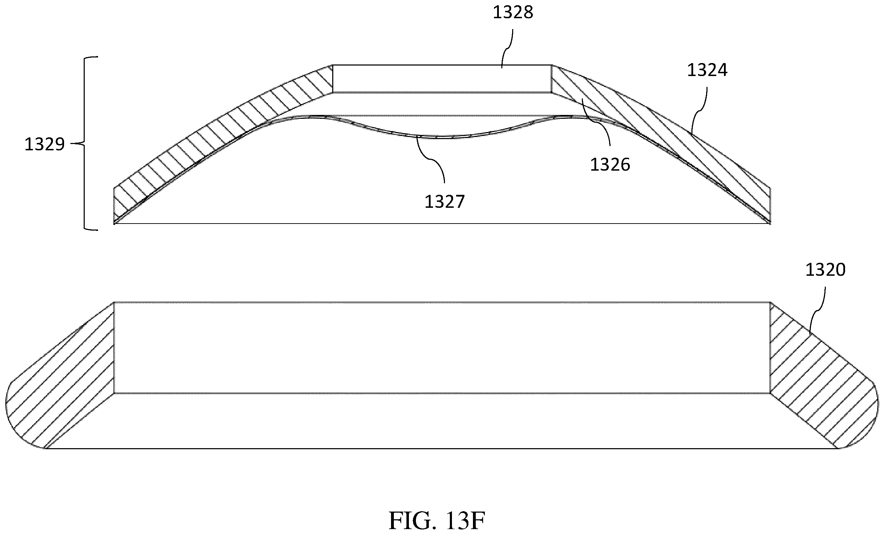

In step 1310, as shown in Fig. 13F, a full-thickness trephination is performed to remove the entire corneal tissue scaffold 1329, including posterior stroma 1324 and separated thin layer 1327, from the donor cornea 1320.

In some embodiments, optionally, the anterior corneal lamella 1322 removed as shown in Fig. 13D, is used for constructing a preassembled system of the present disclosure.

In some embodiments, the corneal tissue scaffold 1329 is then pre-processed using any one or more suitable preprocessing method and techniques, to stabilize and protect the corneal tissue scaffold 1329 during preparation, storage, and transportation.

In step 1312, the corneal tissue scaffold obtained in step 1310 may then be decontaminated, dehydrated, sterilized, and packaged for use by surgeons and other medical professionals in appropriate surgical procedures as further detailed herein.

In some embodiments, further processing methods and techniques according to the present invention may include, but are not limited to: dehydration, lyophilization, plasticization, gelling, dehydrothermal treatment, mechanical processing, compressing, sterilization, and the like.

In some embodiments, the preprocessing prevents the corneal tissue scaffold 1329 from decomposing, putrefying, becoming infected, and/or fracturing during storage and transportation, and allows the corneal tissue scaffold 1329 to be stored at room temperature or at a temperature lower than room temperature. In some embodiments, the preprocessing also helps maintain the corneal tissue scaffold 1329 so that it has similar material properties to those of live or hydrated tissue or treated tissue. In some embodiments, the preprocessing allows for direct implantation of the corneal tissue scaffold 1329 into a recipient subject without further processing or with minimal processing after removal from the packaging.

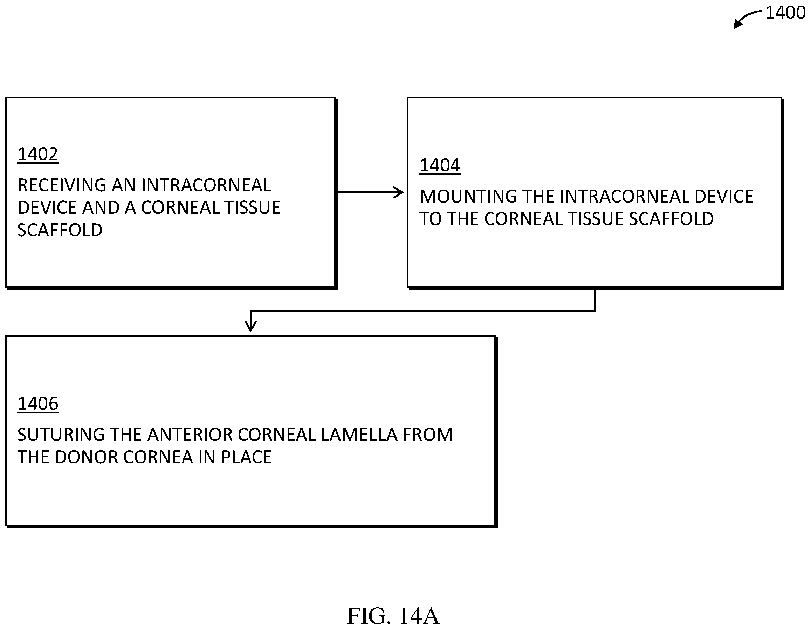

Figs. 14A-14D illustrate an exemplary process 1400 for constructing a preassembled system 1411 of the present disclosure, comprising the corneal tissue scaffold with the pre-mounted device of the present disclosure. The corneal tissue scaffold may be the exemplary corneal tissue scaffold 1329 described with reference to Figs. 13A-13F; the device may be any one of exemplary devices 100 (described with reference to Figs. 1A-1C, 3D-3E, and 6), device 110 (described with reference to Figs. 9A-9D), 120 (described with reference to Figs. 10A-10C), 130 (described with reference to Figs. 11A-1 ID), and/or 140 (described with reference to Figs. 12A-12C).

The particular series of steps depicted in conjunction with process 1400 is not intended to be limiting. Other sequences of steps may also be performed according to alternative embodiments without departing from the techniques of this disclosure. For example, alternative embodiments of the present invention may perform the steps outlined above in a different order or in parallel. Moreover, the individual steps may include multiple sub-steps that may be performed in various sequences as appropriate. Furthermore, additional steps may be added or removed depending on the particular applications.

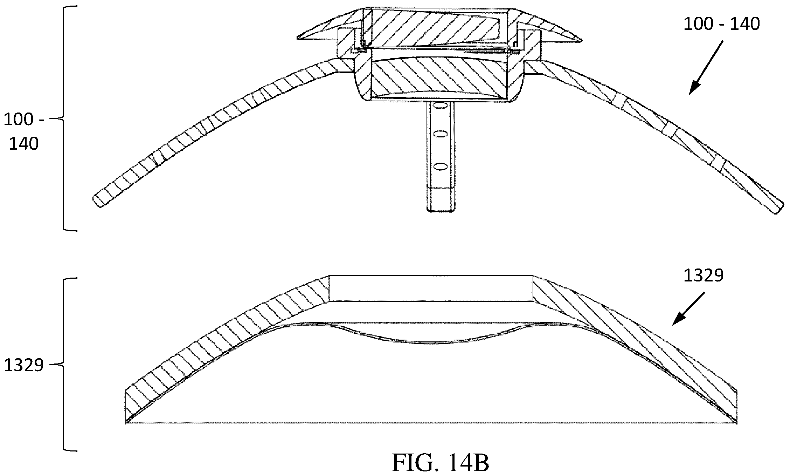

With continued reference to Fig. 14A and as shown in Fig. 14B, process 1400 begins in step 1402 by receiving a device 100-140 and a corneal tissue scaffold 1329.

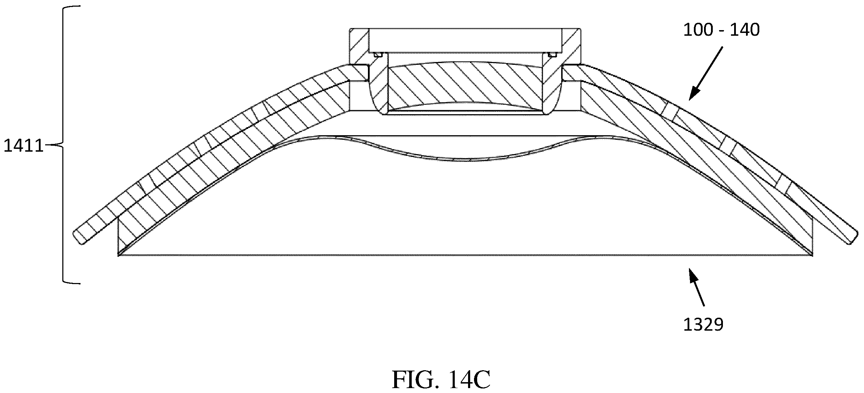

In step 1404, as shown in Fig. 14C, device 100-140 is mounted to a corneal tissue scaffold 1329.

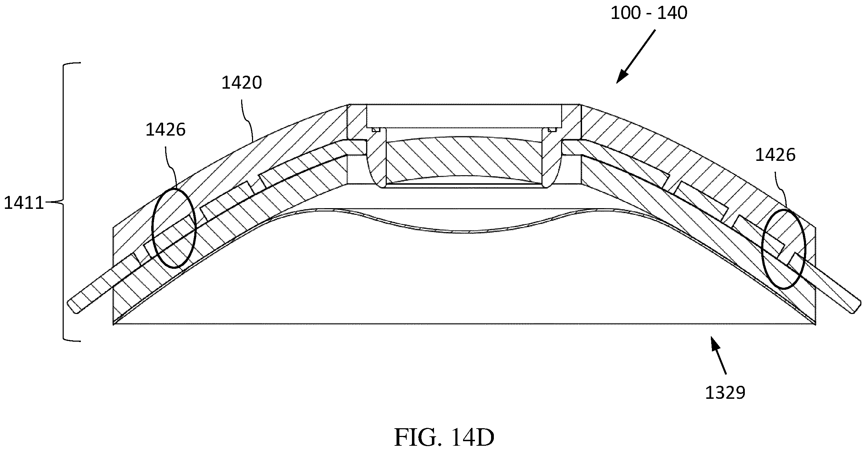

In step 1406, as shown in Fig. 14D, the previously-removed anterior corneal lamella 1420 from the donor cornea (see, for example, the description with reference to Fig. 13D) is sutured 1426 in place.

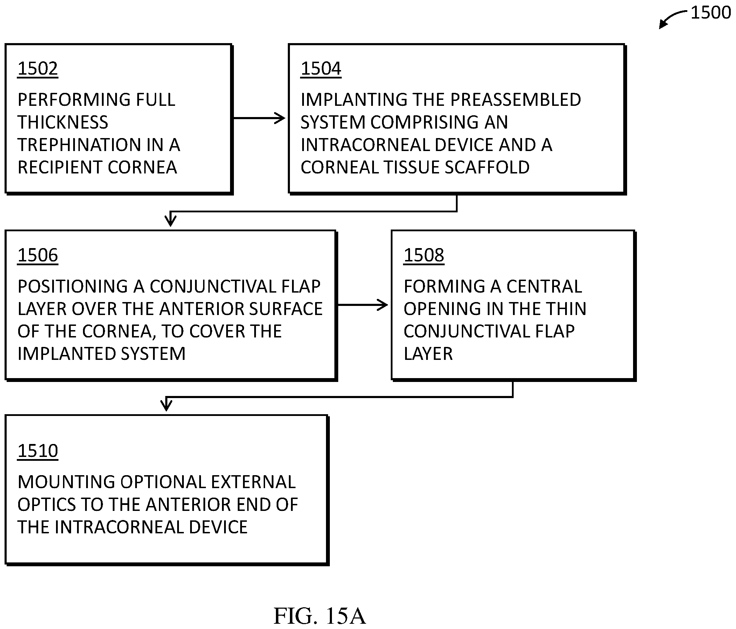

Figs. 15A-15F show the functional steps in an exemplary process 1500 for surgical implantation of a preassembled system of the present disclosure, such as exemplary preassembled system 1411 described with reference to Figs. 14A-14D, in the cornea of a recipient.

The particular series of steps depicted in conjunction with process 1500 is not intended to be limiting. Other sequences of steps may also be performed according to alternative embodiments without departing from the techniques of this disclosure. For example, alternative embodiments of the present invention may perform the steps outlined above in a different order or in parallel. Moreover, the individual steps may include multiple sub-steps that may be performed in various sequences as appropriate. Furthermore, additional steps may be added or removed depending on the particular applications.

In addition, the various healing and/or tissue regeneration stages, which may be necessary or advisable in practice between some of the various steps of this exemplary process, have been omitted in the interest of conciseness.

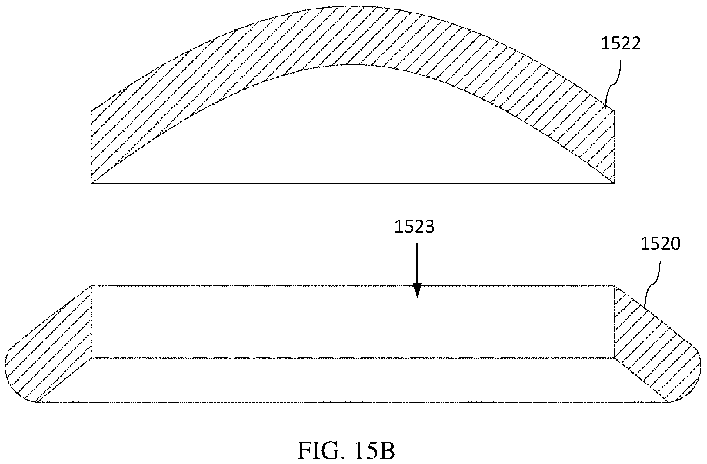

With continued reference to Fig. 15 A, as shown in Fig. 15B, process 1500 begins in step 1502, by performing full thickness trephination in a recipient cornea 1520, to remove the anterior corneal lamella 1522, and create a hollow aperture 1523.

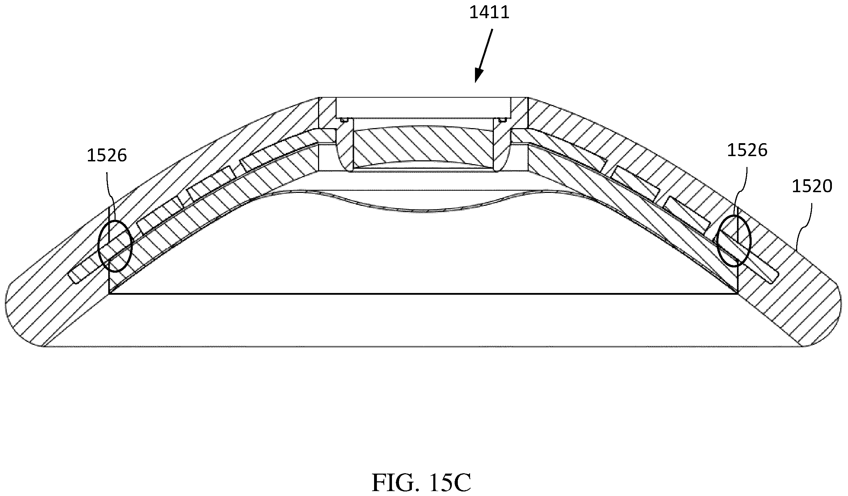

In step 1504, as shown in Fig. 15C, preassembled system 1411 may be implanted in cornea 1520, within the aperture 1523, and sutured 1526.

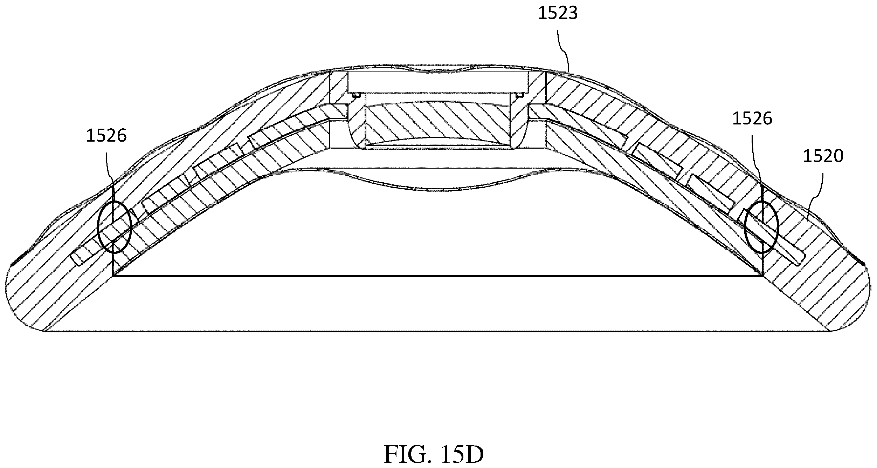

In step 1506, as shown in Fig. 15D, a thin conjunctival flap layer 1523 may be positioned over an anterior surface of cornea 1520, to cover implanted preassembled system 1411.

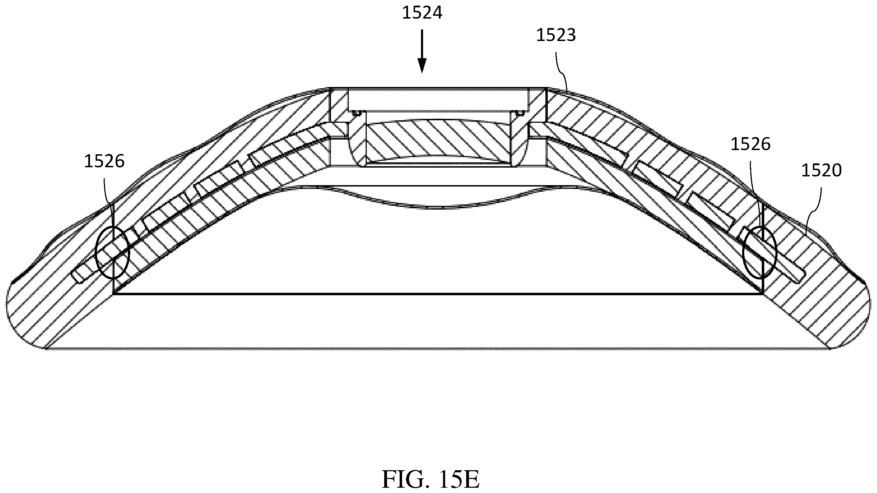

In step 1508, as shown in Fig. 15E, a central opening 1524 may be formed in the thin conjunctival flap layer 1523, correspondingly to an anterior end of the optical stem of the implanted device, such that the anterior end of the optical stem lies just beneath, is substantially at the level of, is flush with, or is slightly proud of, the eye's anterior surface.

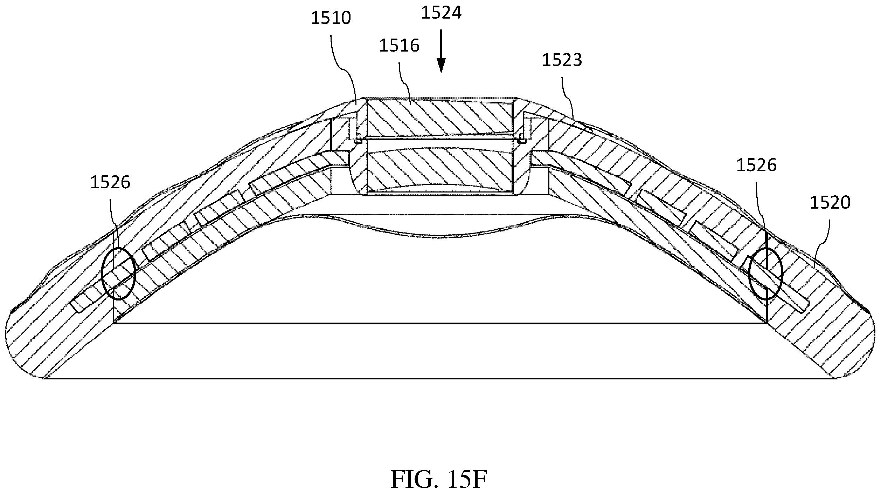

In step 1510, as shown in Fig. 15F, optional external optical elements, including, e.g., end plate 1510 and external optical lens 1516 may be mounted to the anterior end of the device 100-140, which is part of preassembled system 1411.

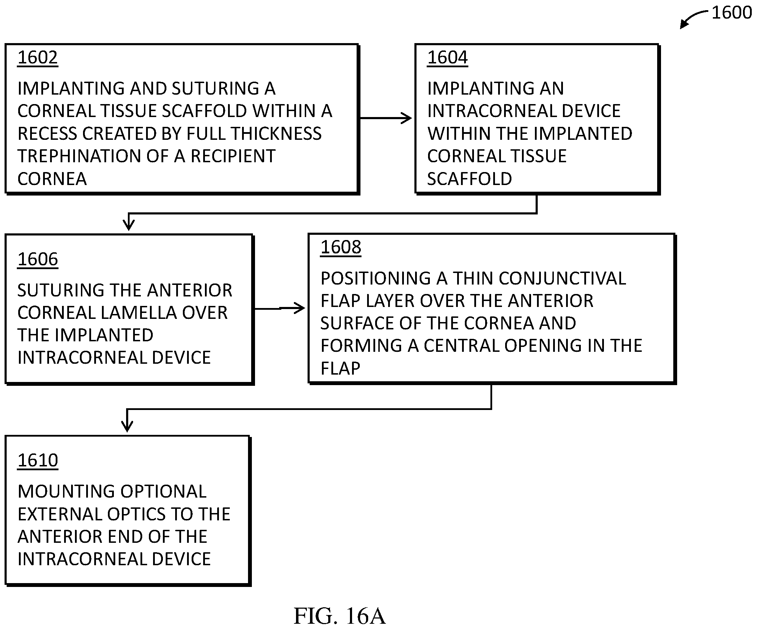

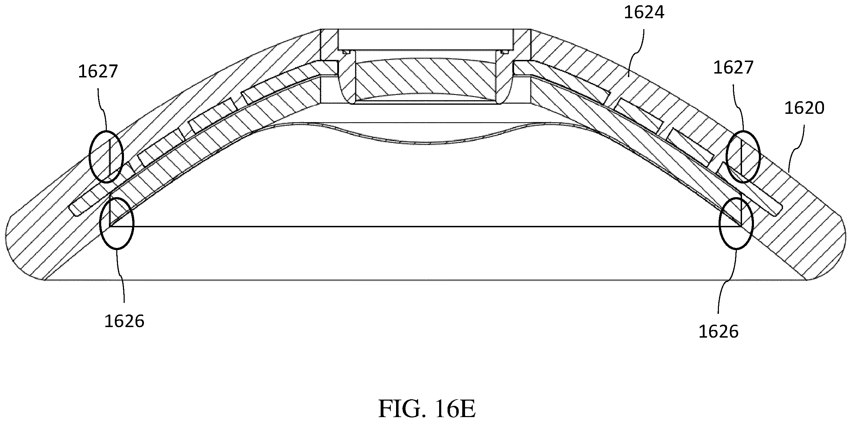

Figs. 16A-16G show the functional steps in an exemplary process 1600 for surgical implantation of a pre-processed corneal tissue scaffold, such as exemplary corneal tissue scaffold 1329 described with reference to Figs. 13A-13F, followed by an implantation of a device of the present technique therewithin.

The particular series of steps depicted in conjunction with process 1600 is not intended to be limiting. Other sequences of steps may also be performed according to alternative embodiments without departing from the techniques of this disclosure. For example, alternative embodiments of the present invention may perform the steps outlined above in a different order or in parallel. Moreover, the individual steps may include multiple sub-steps that may be performed in various sequences as appropriate. Furthermore, additional steps may be added or removed depending on the particular applications.

In addition, the various healing and/or tissue regeneration stages, which may be necessary or advisable in practice between some of the various steps of this exemplary process, have been omitted in the interest of conciseness.

After full thickness trephination of the recipient cornea, implantation of exemplary corneal tissue scaffold 1329 is performed and then followed by an implantation of a device 100-140 of the present disclosure, which may be any one of exemplary devices 100 (described with reference to Figs. 1A-1C, 3D-3E, and 6), device 110 (described with reference to Figs. 9A-9D), 120 (described with reference to Figs. 10A-10C), 130 (described with reference to Figs. 11A-11D), and/or 140 (described with reference to Figs. 12A-12C).

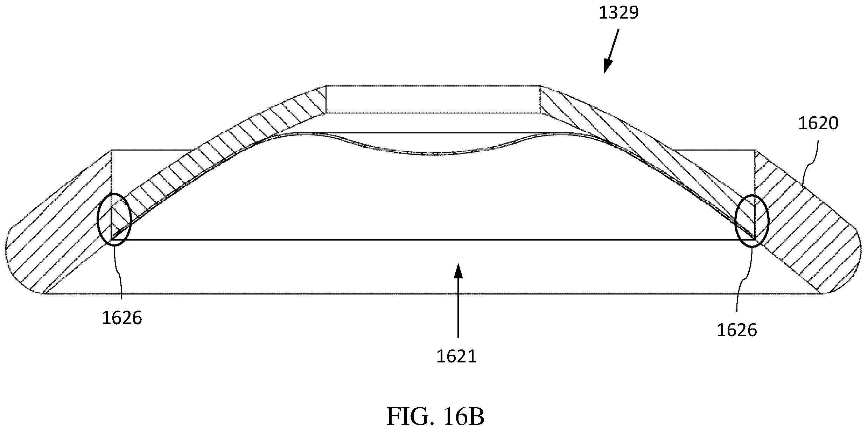

With continued reference to Fig. 16A, and as shown in Fig. 16B, process 1600 begins in step 1602, where corneal tissue scaffold 1329 is implanted and sutured 1626 within a recess 1621 created by full thickness trephination of the recipient cornea 1620.

Recipient cornea 1620 can be prepared by first performing a partial trephination and anterior keratectomy in the stromal layers, to remove the recipient anterior corneal lamella of the recipient cornea, and then a full-thickness trephination to create recess 1621.

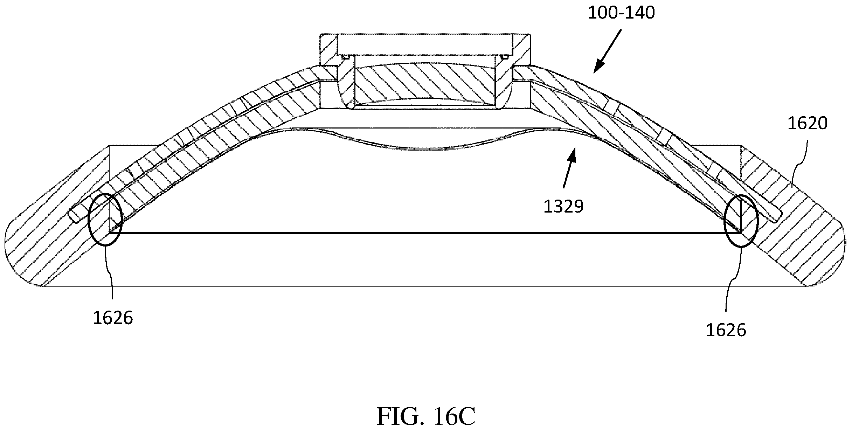

In step 1604, as shown in Fig. 16C, device 100-140 is implanted and fixated in place.

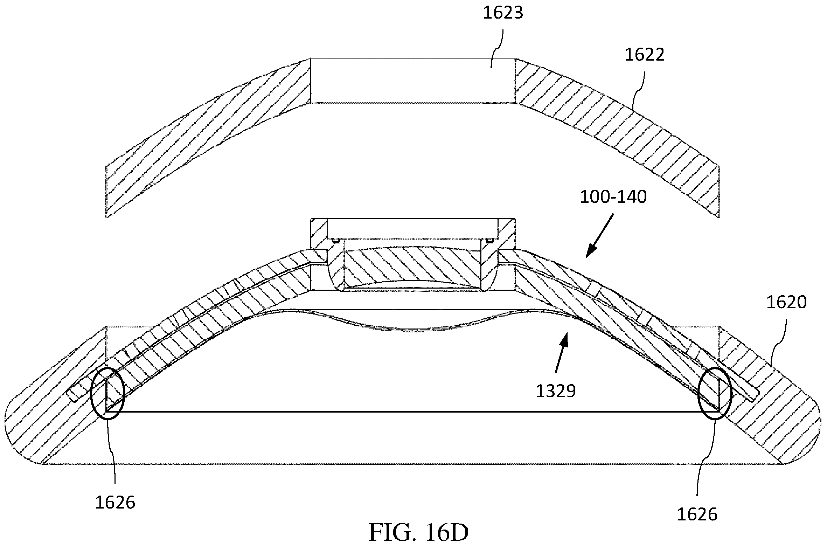

In step 1606, as shown in Figs. 16D-16E, a central aperture 1623 is made within the anterior corneal lamella 1622 (which may be the previously-removed recipient anterior corneal lamella, or a donor anterior corneal lamella). Anterior corneal lamella 1622 is then sutured 1627 to cover the implanted device 100-140, such that an anterior end of device 100-140 extends through aperture 1623.

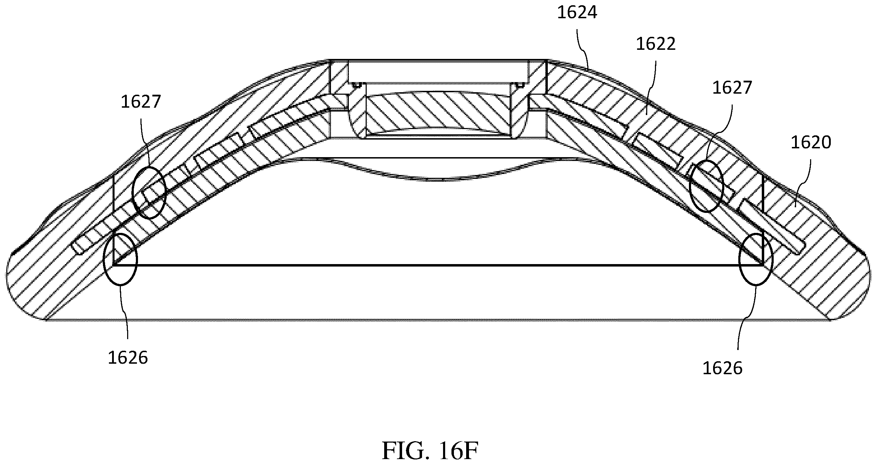

In step 1608, as shown in Fig. 16F, a thin conjunctival flap layer 1624 may be positioned over the anterior surface of cornea 1622. A central opening 1628 is then formed in thin conjunctival flap layer 1622, correspondingly to an anterior end of the optical stem of device 100-140, such that the anterior end of the optical stem lies just beneath, is substantially at the level of, is flush with, or is slightly proud of, the eye's anterior surface.

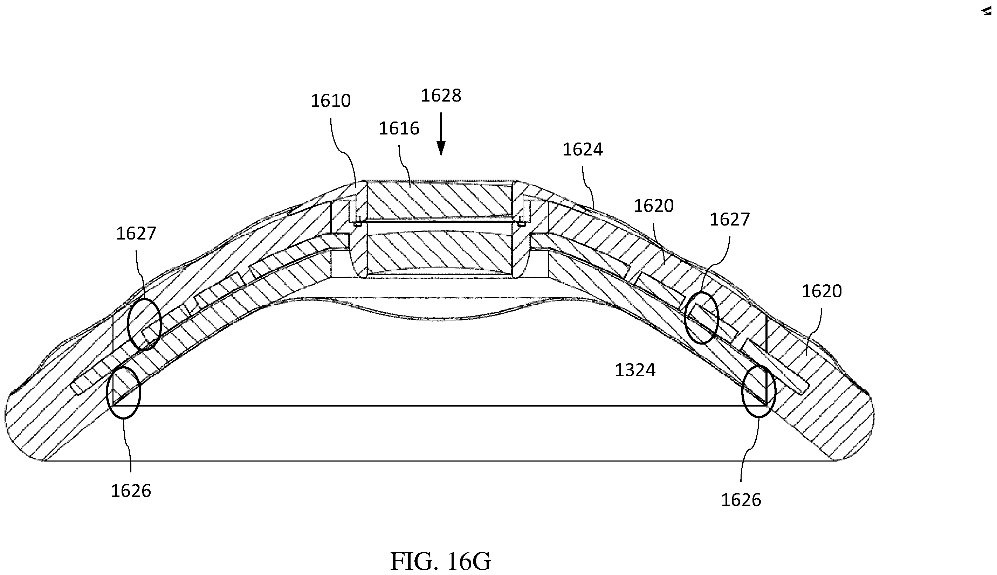

In step 1610, as shown in Fig. 16G, optional external elements, including, e.g., end plate 1610 and external optical lens 1616 may be mounted to the anterior end of the device 100-140.

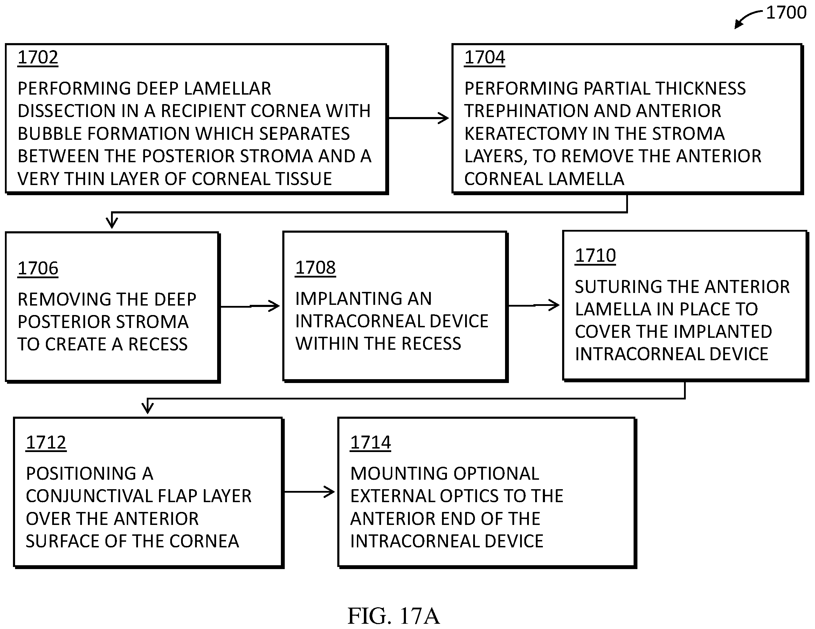

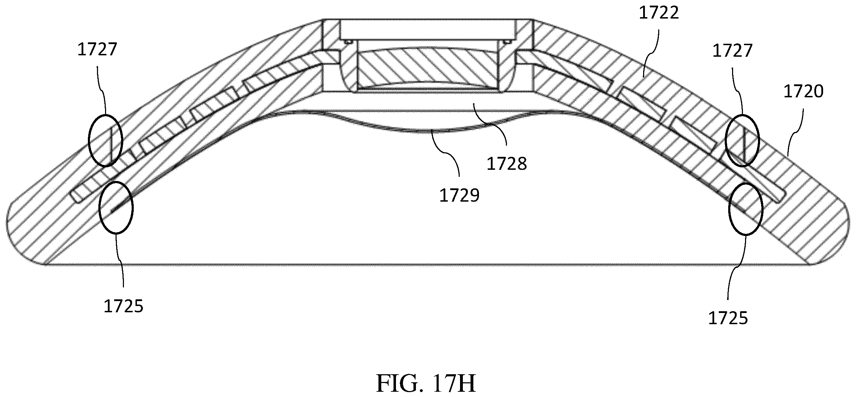

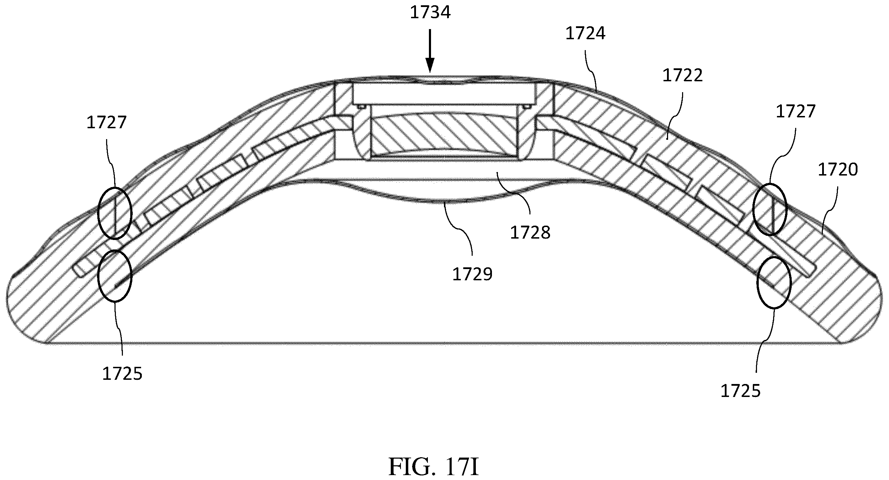

Figs. 17A-17J show the functional steps in an exemplary process 1700 for surgical implantation of a device 100-140 of the present disclosure, which may be any one of exemplary devices 100 (described with reference to Figs. 1A-1C, 3D-3E, and 6), device 110 (described with reference to Figs. 9A-9D), 120 (described with reference to Figs. 10A- 10C), 130 (described with reference to Figs. 11A-11D), and/or 140 (described with reference to Figs. 12A-12C).

The particular series of steps depicted in conjunction with process 1700 is not intended to be limiting. Other sequences of steps may also be performed according to alternative embodiments without departing from the techniques of this disclosure. For example, alternative embodiments of the present invention may perform the steps outlined above in a different order or in parallel. Moreover, the individual steps may include multiple sub-steps that may be performed in various sequences as appropriate. Furthermore, additional steps may be added or removed depending on the particular applications.

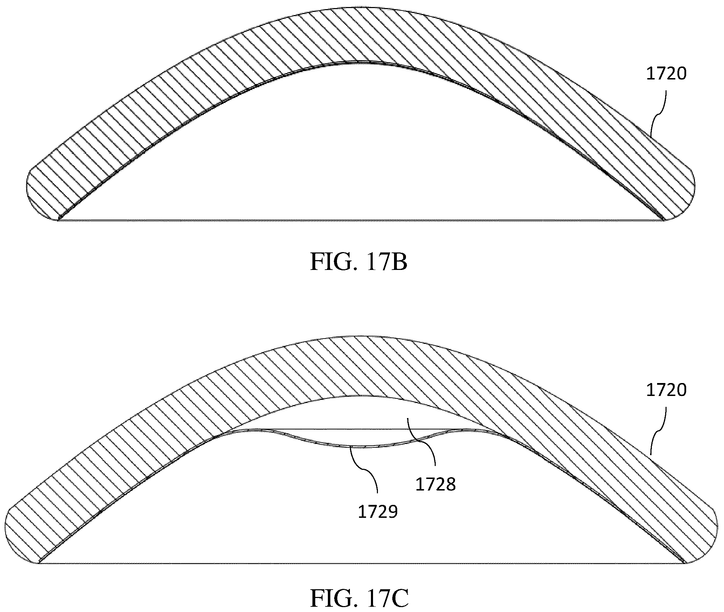

With continued reference to Fig. 17A, and as shown in Figs. 17B-17C, process 1700 begins in step 1702, with preparation of recipient cornea 1720.

As shown in Fig. 17C, a deep lamellar dissection (e.g. pneumatic, hydro-assisted, viscoelastic-assisted and/or manual dissection) is performed in the donor cornea 1720 with a bubble 1728 formation which separates between the posterior stroma and a very thin layer of corneal tissue 1729 having a thickness of approximately 10-50µm, and substantially transparent to light. In some embodiments, thin layer 1729 comprises at least the pre-Descemet layer. In some embodiments, thin layer 1729 comprises the pre-Descemet layer in addition to the Descemet membrane. In some embodiments, thin layer 1729 comprises the pre-Descemet layer, the Descemet membrane, and the endothelium.

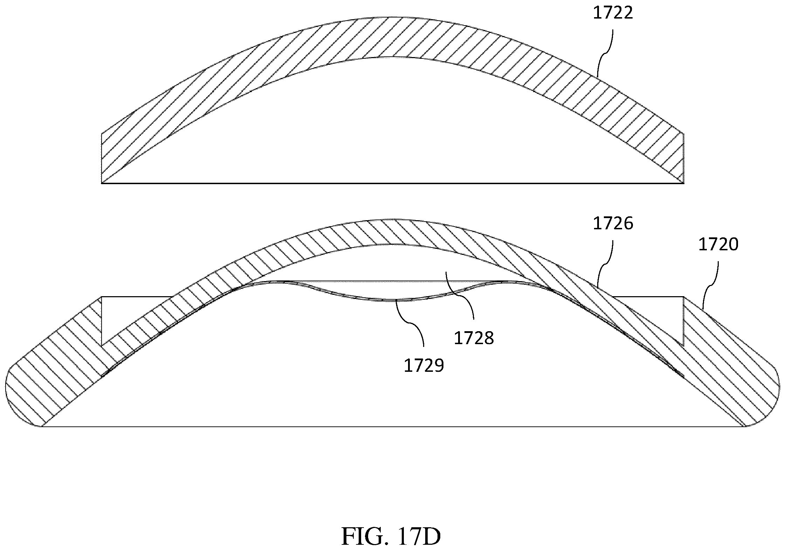

In step 1704, as shown in Fig. 17D, a partial thickness trephination and anterior keratectomy is then performed in the stromal layers of donor cornea 1720, to remove the anterior corneal lamella 1722, leaving the posterior stromal layers 1726.

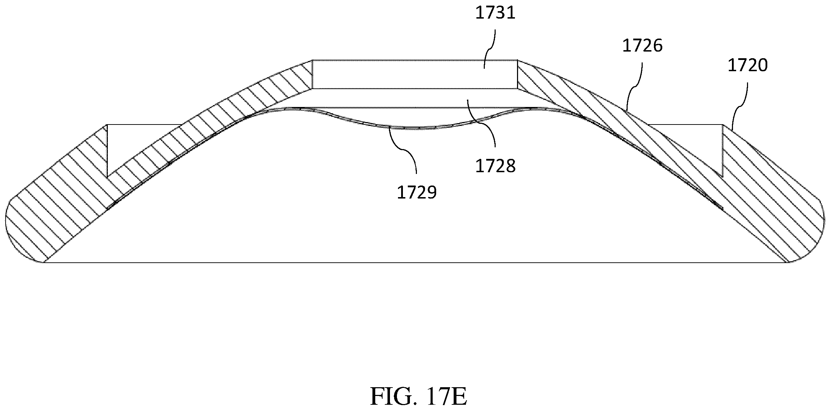

In step 1706, as shown in Fig. 17E, a removal of deep posterior stroma within or around the region above the bubble 1728 is performed in the posterior stroma 1726, to create a recess 1731, while leaving thin posterior layer 1729 having a thickness of approximately 10-50µm, and substantially transparent to light). In some embodiments, the very thin layer comprises at least the pre-Descemet layer. In some embodiments, the very thin layer comprises the pre-Descemet layer in addition to the Descemet membrane. In some embodiments, the very thin layer comprises the pre-Descemet layer, the Descemet membrane, and the endothelium.

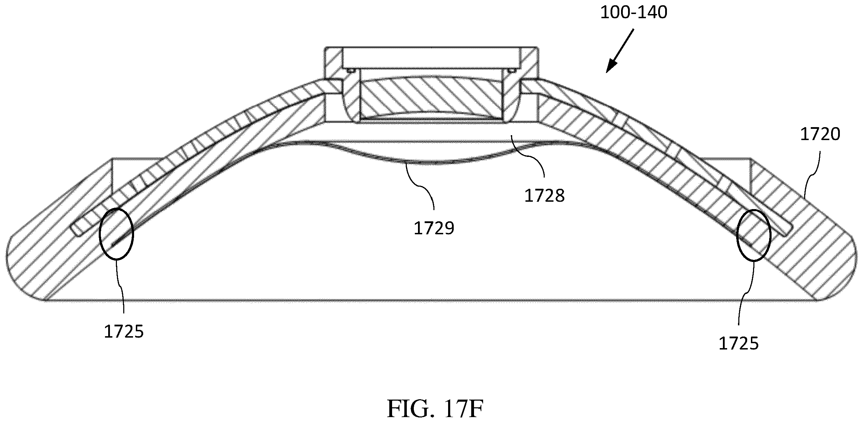

In step 1708, as shown in Fig. 17F, device 100-140 is implanted in place and fixated 1725, such that a light-projecting posterior port of the optical stem of device 100- 140 is positioned through recess 1731.

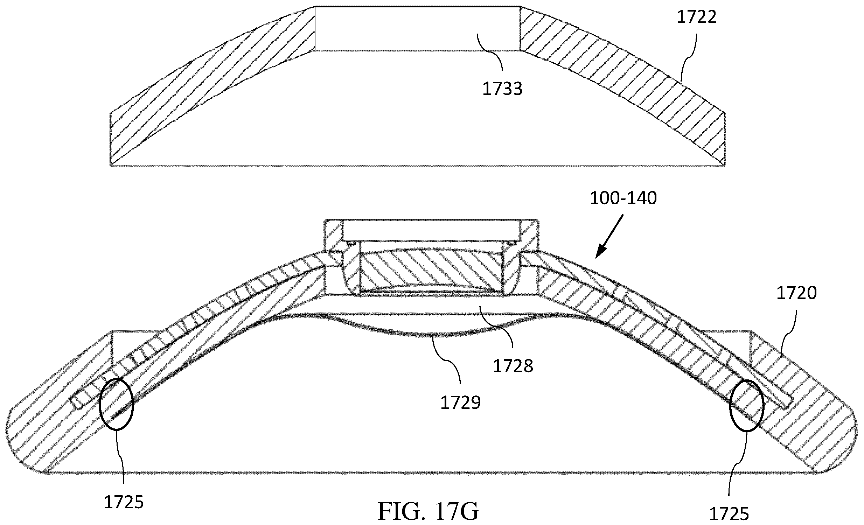

In step 1710, as shown in Figs. 17G-17H, a central aperture 1733 is made within the anterior corneal lamella 1722 (e.g. the previously-removed recipient anterior corneal lamella or donor anterior corneal lamella). Anterior corneal lamella 1722 is then sutured 1727 to cover the implanted device 100-140, such that an anterior end of device 100-140 extends through aperture 1733.

In step 1712, as shown in Fig. 171, a thin conjunctival flap layer 1724 may be positioned over the anterior surface of cornea 1720. A central opening 1734 is then formed in thin conjunctival flap layer 1724, correspondingly to an anterior end of the optical stem of device 100-140, such that the anterior end of the optical stem lies just beneath, is substantially at the level of, is flush with, or is slightly proud of, the eye's anterior surface.

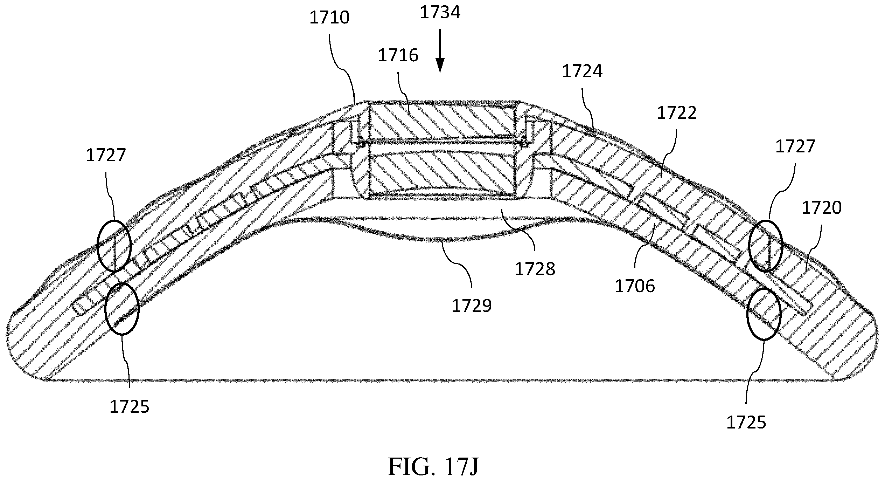

In step 1714, as shown in Fig. 17J, optional external elements, including, e.g., end plate 1710 and external optical lens 1716 may be mounted to the anterior end of the device 100-140.

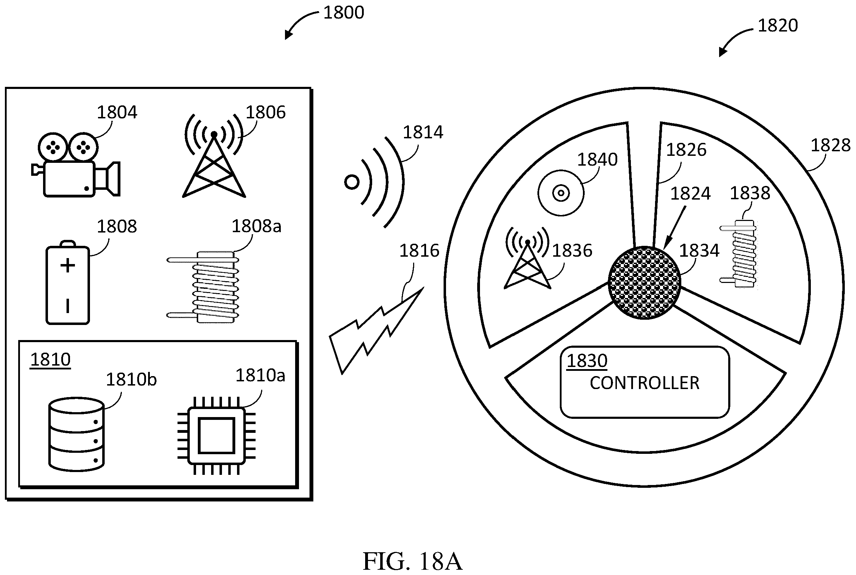

Fig. 18A is a schematic illustration of an exemplary system for intracorneal projecting of images onto a retina, according to some embodiments of the present disclosure.

In some embodiments, a system of the present disclosure comprises an extraocular image capture unit 1800 configured to capture one or more still visual images or moving visual images (video).

Extraocular image capture unit 1800 as described herein is only an exemplary embodiment of the present invention, and in practice may be implemented in any combination of both hardware and software components. Extraocular unit 1800 may have more or fewer components and modules than shown, may combine two or more of the components, or may have a different configuration or arrangement of the components. Extraocular unit 1800 may include any additional component enabling it to function as an operable computer system, such as a motherboard, data busses, power supply, a network interface card, a display, an input device (e.g., keyboard, pointing device, touch-sensitive display), etc. (not shown).

In some embodiments, extraocular unit 1800 may comprise an image processing module 1810 comprising one or more hardware processor(s) 1810a, and a memory storage device 1810b, comprising, e.g., a random-access memory (RAM) and one or more nontransitory computer-readable storage device(s).

Storage device 1810b may have stored thereon program instructions and/or components configured to operate hardware processor 1810a of image processing module 1810. The program instructions may include one or more software modules, such as one or more image processing algorithms. The software components may include an operating system having various software components and/or drivers for controlling and managing general system tasks (e.g., memory management, storage device control, power management, etc.), and facilitating communication between various hardware and software components.

In some embodiments, extraocular unit 1800 comprises an imaging device 1804, which may be any suitable digital imaging device configured for capturing still or video images of scenes, objects, and/or any other optically-represented information, such as by text, symbols or color. In some embodiments, imaging processing module 1810 is configured to implement any suitable one or more image processing algorithms, and has an image signal generator for generating digital data signals that can represent a visual images captured by imaging device 1804. In some embodiments, storage device 1810b may include an electrical circuit data memory of the type that is suitable for storing electrical digital image data signals.

In some embodiments, extraocular unit 1800 may comprise a wireless transmitter or a transceiver 1806 configured to transmit an image data signal using non-optical data transmission modalities, e.g., any suitable short-range RF transmission protocol, digital or analog, such as Bluetooth, ZigBee, and the like.

In some embodiments, extraocular unit 1800 may comprise a power source 1808 which may include, e.g., a rechargeable battery pack comprising one or more battery cells of the type commonly used for providing a portable battery supply, and a facility for short-range wireless power transmission. In some embodiments, the battery pack may provide power to a primary inductive coil 1808a. The primary inductive coil 1808a may send power wirelessly 1816, e.g., through skin, bone, eye tissue, etc., to a secondary reception inductive coil within an intracorneal implant, as shall be further described below.

In some embodiments, the functions of wireless data transmission and wireless power transmission may both be conducted by the primary inductive coil 1808a, wherein the primary inductive coil 1808a may be configured for joint transmission of power and data.

In some embodiments, extraocular unit 1800 may be in wireless data 1814 and wireless power 1816 communication with an exemplary intracorneal implant 1820, configured for surgical implantation within the cornea of an eye.

In some embodiments, intraocular implant 1820 may be dimensioned and configured for surgical implantation within the eye, traversing or completely replacing any opaque tissue layers of the cornea, and wherein only substantially transparent corneal tissue is present posterior to the device.

In some embodiments, exemplary intracorneal implant 1820 may be dimensioned and configured for surgical implantation within the cornea of an eye, such that a layer of corneal tissue that is partially transparent, partially semi-transparent, and/or partially opaque is present posteriorly to the light-projecting posterior port of the device.

As shall be further detailed below, intracorneal implant 1820 is dimensioned to fit within a cornea. In some embodiments, intracorneal implant 1820 defines a planar body having a convexity which substantially conforms to the convexity of the cornea, with opposing convex anterior face (facing the anterior surface of the eye) and concave posterior face (facing the retina of the eye).

In some embodiments, intracorneal implant 1820 comprises an inner platform portion 1824 located about an axis of circular symmetry of the implant, and a peripheral portion, comprising one or more angularly-spaced arms 1826 that extend laterally from a peripheral edge of inner platform 1824. In some embodiments, intracorneal implant 1820 further comprises a peripheral skirt portion 1828 which defines an outer circular perimeter of intracorneal implant 1820, wherein the one or more arms 1826 connect between inner platform 1824 and peripheral skirt portion 1828.

In some embodiments, exemplary intracorneal implant 1820 is dimensioned to fit within a cornea, posteriorly to any opaque corneal tissue layers, and to generate images that are detectable by a healthy retina or a healthy portion of a retina of the eye. For example, intracorneal implant 1820 may have an overall span between opposing edges thereof of, e.g., 6- 12mm (e.g., 9mm), an overall planar thickness of between 0.02- 1.50mm, and a convexity having a curvature of between 32-47 diopters.

In some embodiments, exemplary intracorneal implant 1820 comprises one or more components mounted to intracorneal implant 1820, including, e.g., an image projection controller 1830, comprising, e.g., a hardware processor and memory storage device, an image generator 1834 mounted to inner platform portion 1824, a wireless receiver or transceiver 1836, a wireless power receiving element 1838, and optical assembly 1840.

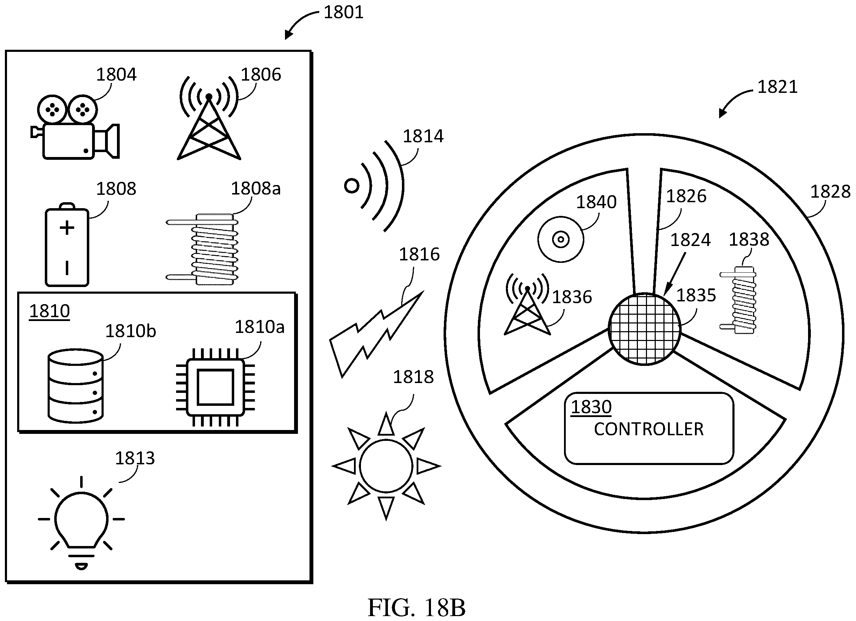

Fig. 18B is a schematic illustration of a second exemplary system for intracorneal projecting of images onto a retina, according to some embodiments of the present disclosure.

In some embodiments, a second exemplary system of the present disclosure comprises an extraocular image capture unit 1801 configured to capture one or more still visual images, or moving visual images (video).

Extraocular image capture unit 1801 as described herein is only an exemplary embodiment of the present invention, and in practice may be implemented in any combination of both hardware and software components. Extraocular unit 1801 may have more or fewer components and modules than shown, may combine two or more of the components, or may have a different configuration or arrangement of the components. Extraocular unit 1801 may include any additional component enabling it to function as an operable computer system, such as a motherboard, data busses, power supply, a network interface card, a display, an input device (e.g., keyboard, pointing device, touch-sensitive display), etc. (not shown).

In some embodiments, extraocular unit 1801 may comprise an image processing module 1810 comprising one or more hardware processor(s) 1810a, and a memory storage device 1810b, comprising, e.g., a random-access memory (RAM) and one or more nontransitory computer-readable storage device(s).

Storage device 1810b may have stored thereon program instructions and/or components configured to operate hardware processor 1810a of image processing module 1810. The program instructions may include one or more software modules, such as one or more image processing algorithms. The software components may include an operating system having various software components and/or drivers for controlling and managing general system tasks (e.g., memory management, storage device control, power management, etc.), and facilitating communication between various hardware and software components.

In some embodiments, extraocular unit 1801 comprises an imaging device 1804, which may be any suitable digital imaging device configured for capturing still or video images of scenes, objects, and/or any other optically-represented information, such as by text, symbols or color. In some embodiments, imaging processing module 1810 is configured to implement any suitable one or more image processing algorithms, and has an image signal generator for generating digital data signals that can represent a visual images captured by imaging device 1804. In some embodiments, storage device 1810b may include an electrical circuit data memory of the type that is suitable for storing electrical digital image data signals.

In some embodiments, extraocular unit 1801 may comprise a wireless transmitter or a transceiver 1806 configured to transmit an image data signal using non-optical data transmission modalities, e.g., any suitable short-range RF transmission protocol, digital or analog, such as Bluetooth, ZigBee, and the like.

In some embodiments, extraocular unit 1801 may comprise a power source 1808 which may include, e.g., a rechargeable battery pack comprising batteries of the type commonly used for providing a portable battery supply, and a facility for short-range wireless power transmission. In some embodiments, the battery pack may provide power to a primary inductive coil 1808a. The primary inductive coil 1808a may send power wirelessly 1816, e.g., through skin, bone, eye tissue, etc., to a secondary reception inductive coil within an intracorneal implant, as shall be further described below.

In some embodiments, the functions of wireless data transmission and wireless power transmission may both be carried out by the primary inductive coil 1808a, wherein the primary inductive coil 1808a may be configured for joint transmission of power and data.

In some embodiments, extraocular unit 1801 may comprise an illumination source 1813 for illuminating a liquid crystal display (LCD) mounted to the intracorneal implant. In some embodiments, illumination source 1813 may comprise any suitable illumination source, e.g., one or more light emitting diodes (LEDs), such as white light-emitting LEDs, color LEDs, or high intensity power LEDs. In some embodiments, illumination source 1813 may be configured to emit sufficient illumination so as to traverse impaired or opaque corneal layers that are at least partially impermeable to visible light, and to reach the intracorneal implant 1821 implanted within the cornea of the eye.

Extraocular unit 1801 may be in wireless data 1814 and wireless power 1816 communication, as well as in illumination communication 1818, with an intracorneal implant 1821 configured for surgical implantation within the cornea of an eye.

In some embodiments, exemplary intracorneal implant 1821 may be dimensioned and configured for surgical implantation within the cornea of an eye, at a position that is posterior to any opaque tissue layers of the cornea, and where transparent corneal tissue or no corneal tissue is present posterior to the image generator of the device.

As shall be further detailed below, intracorneal implant 1821 is dimensioned to fit within a cornea. In some embodiments, intracorneal implant 1821 defines a planar body having a convexity which substantially conforms to the convexity of the cornea, with opposing convex anterior face (facing the anterior surface of the eye) and concave posterior face (facing the retina of the eye).

In some embodiments, intracorneal implant 1821 comprises an inner platform portion 1824 located about an axis of circular symmetry of the implant, and a peripheral portion, comprising one or more angularly-spaced arms 1826 that extend laterally from a peripheral edge of inner platform 1824. In some embodiments, intracorneal implant 1821 further comprises a peripheral skirt portion 1828 which defines an outer circular perimeter of intracorneal implant 1821, wherein the one or more arms 1826 connect between inner platform 1824 and peripheral skirt portion 1828.

In some embodiments, exemplary intracorneal implant 1821 is dimensioned to fit within a cornea, traversing or completely replacing any opaque tissue layers of the cornea, and wherein only substantially transparent corneal tissue is present posterior to the device.

In some embodiments, exemplary intracorneal implant 1821 is dimensioned to fit within a cornea, such that a layer of corneal tissue that is partially transparent, partially semi-transparent, and/or partially opaque is present posteriorly to the light-projecting posterior port of the device.

For example, intracorneal implant 1821 may have an overall span between opposing edges thereof of, e.g., 6-12mm (e.g., 9mm), an overall planar thickness of between 0.020- 1.5mm, and a convexity having a curvature of between 32-47 diopters.

In some embodiments, intracorneal implant 1821 comprises one or more components mounted to intracorneal implant 1821, including, e.g., an image projection controller 1830, a memory storage device 1832, an LCD panel 1835 mounted to inner platform portion 1824, a wireless receiver or transceiver 1836, a wireless power receiving element 1838, and an optical assembly 1840.

In some embodiments, LCD panel 1835 does not include a backlight or any similar illumination source integrated within intracorneal implant 1821. By way of explanation, an LCD panel display comprises a matrix of pixels which may be controllably darkened or made transparent, to represent any pattern or image. LCD panels typically do not emit light directly, but rather rely on using an integrated backlight to produce images. The power requirements of the LCD panel itself are minimal, while those of the backlight are significantly higher. Accordingly, in the context of an LCD panel mounted to a device implanted within a cornea, it would be advantageous to eliminate the need for an integrated backlight within the implanted device, and instead rely on an external illumination source. This may lead to a significant reduction in power consumption, physical footprint, and complexity of the implanted device. Accordingly, in some embodiments, LCD panel 1835 is configured to be illuminated by external illumination source 1813 of extraocular unit 1800.

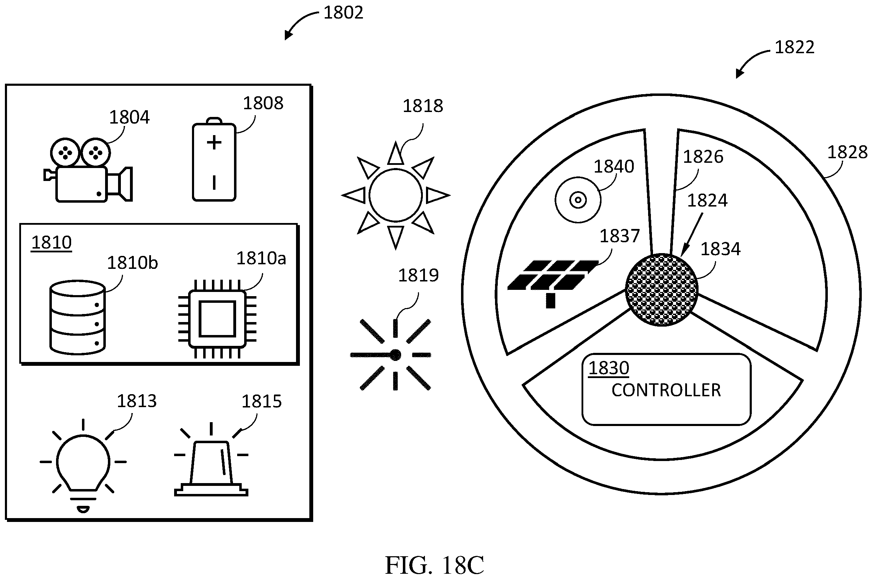

Fig. 18C is a schematic illustration of a third exemplary system for intracorneal projecting of images onto a retina, according to some embodiments of the present disclosure.

Extraocular image capture unit 1802 as described herein is only an exemplary embodiment of the present invention, and in practice may be implemented in any combination of both hardware and software components. Extraocular unit 1802 may have more or fewer components and modules than shown, may combine two or more of the components, or may have a different configuration or arrangement of the components. Extraocular unit 1802 may include any additional component enabling it to function as an operable computer system, such as a motherboard, data busses, power supply, a network interface card, a display, an input device (e.g., keyboard, pointing device, touch-sensitive display), etc. (not shown).

In some embodiments, extraocular unit 1802 may comprise an image processing module 1810 comprising one or more hardware processor(s) 1810a, and a memory storage device 1810b, comprising, e.g., a random-access memory (RAM) and one or more nontransitory computer-readable storage device(s).

Storage device 1810b may have stored thereon program instructions and/or components configured to operate hardware processor 1810a of image processing module 1810. The program instructions may include one or more software modules, such as one or more image processing algorithms. The software components may include an operating system having various software components and/or drivers for controlling and managing general system tasks (e.g., memory management, storage device control, power management, etc.), and facilitating communication between various hardware and software components.

In some embodiments, extraocular unit 1802 comprises an imaging device 1804, which may be any suitable digital imaging device configured for capturing still or video images of scenes, objects, and/or any other optically-represented information, such as by text, symbols or color. In some embodiments, imaging processing module 1810 is configured to implement any suitable one or more image processing algorithms, and has an image signal generator for generating digital data signals that can represent a visual images captured by imaging device 1804. In some embodiments, storage device 1810b may include an electrical circuit data memory of the type that is suitable for storing electrical digital image data signals.

In some embodiments, extraocular unit 1802 may comprise a power source 1808 which may include, e.g., a rechargeable battery pack comprising batteries of the type commonly used for providing a portable battery supply, and a facility for short-range wireless power transmission.

In some embodiments, extraocular unit 1802 may comprise an illumination source 1813 for illuminating a liquid crystal display (LCD) mounted to the intracorneal implant. In some embodiments, illumination source 1813 may comprise any suitable illumination source, e.g., one or more light emitting diodes (LEDs), such as white light-emitting LEDs, color LEDs, or high intensity power LEDs. In some embodiments, illumination source 1813 may be configured to emit sufficient illumination so as to traverse impaired or opaque corneal layers that are at least partially impermeable to visible light, and to reach the intracorneal implant 1822 implanted within the cornea of the eye.

In some embodiments, extraocular unit 1802 may comprise a light beam source 1815 configured to transmit a light beam comprising a power signal and/or a data signal encoding information. In some embodiments, light beam source 1815 may comprise a visible light source or an invisible light source (e.g., an infrared light source). In some embodiments, light beam source 1815 may be selected from the group consisting of: LED, laser diode, and the like. In some embodiments, light beam source 1815 may be configured to perform the functions of wireless data transmission and wireless power transmission.

Accordingly, extraocular unit 1802 may be in illumination communication 1818, as well as in wireless data and wireless power communication 1819, with an intracorneal implant 1822 configured for surgical implantation within the cornea of an eye.

In some embodiments, exemplary intracorneal implant 1822 may be dimensioned and configured for surgical implantation within the cornea of an eye, traversing or completely replacing any opaque tissue layers of the cornea, and wherein only substantially transparent corneal tissue is present posterior to the device.

In some embodiments, exemplary intracorneal implant 1822 may be dimensioned and configured for surgical implantation within the cornea of an eye, such that a layer of corneal tissue that is partially transparent, partially semi-transparent, and/or partially opaque is present posteriorly to the light-projecting posterior port of the device.

In some embodiments, intracorneal implant 1822 defines a planar body having a convexity which substantially conforms to the convexity of the cornea, with opposing convex anterior face (facing the anterior surface of the eye) and concave posterior face (facing the retina of the eye).

In some embodiments, intracorneal implant 1822 comprises an inner platform portion 1824 located about an axis of circular symmetry of the implant, and a peripheral portion, comprising one or more angularly-spaced arms 1826 that extend laterally from a peripheral edge of inner platform 1824. In some embodiments, intracorneal implant 1822 further comprises a peripheral skirt portion 1828 which defines an outer circular perimeter of intracorneal implant 1822, wherein the one or more arms 1826 connect between inner platform 1824 and peripheral skirt portion 1828.

In some embodiments, exemplary intracorneal implant 1822 is dimensioned to fit within a cornea, posteriorly to any opaque corneal tissue layers, and to generate images that are detectable by a healthy retina or a healthy portion of a retina of the eye. For example, intracorneal implant 1822 may have an overall span between opposing edges thereof of, e.g., 6- 12mm (e.g., 9mm), an overall planar thickness of between 0.020- 1.5mm, and a convexity having a curvature of between 32-47 diopters.

In some embodiments, receiver 1837 comprises, e.g., a photovoltaic cell for receiving power and a photodiode for receiving data transmitted by light beam source 1815 over wireless data and wireless power communication 1819.

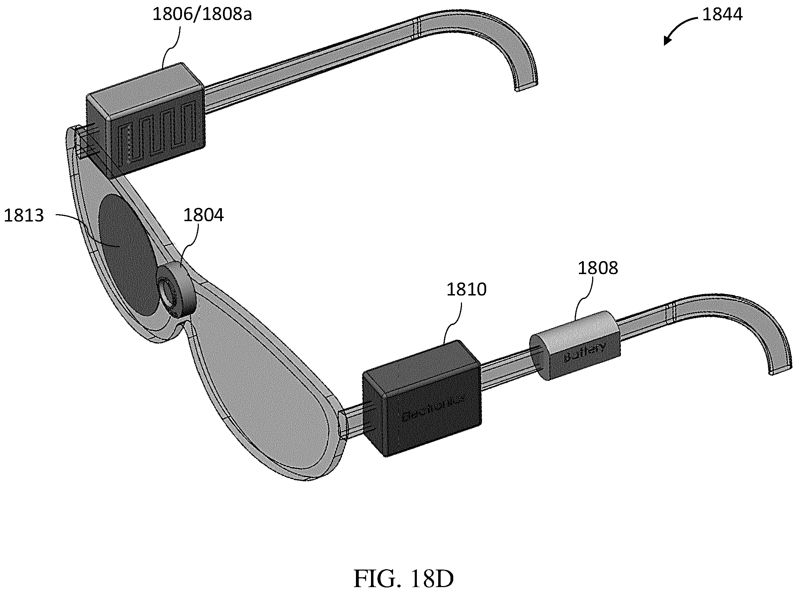

Fig. 18D illustrates an exemplary extraocular unit 1844 of the present disclosure, wherein the various components of the extraocular unit are mounted to a pair of spectacles to be worn by a user, including, but not limited to, imaging device 1804, transmitter/transceiver 1806, primary inductive coil 1808a, power source 1808, image processing module 1810, and/or illumination source 1813. However, in other exemplary embodiments, the various components of extraocular unit 1800 may be mounted to any one or more head-worn elements, such as a helmet, headband, headgear, etc. In some embodiments, imaging device 1804 may be advantageously located to approximate a point-of-view of either or both eyes of the wearer.

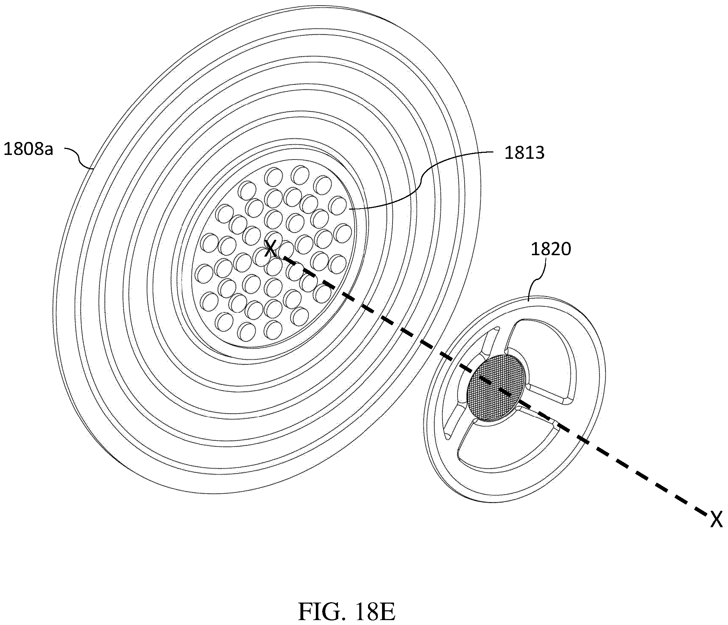

Fig. 18E shows an exemplary illumination source 1813 of the present disclosure, described with reference to Figs. 18B-18C, which may be mounted within the lens area of the spectacles (as shown in Fig. 18D), such that the illumination source 1813 is configured to illuminate the intracorneal implant 1820 implanted within the cornea of the eye. Thus, illumination source 1813 may be mounted to the lens area of a pair of spectacles to be worn by a user, such that illumination source 1813 aligns with an optical axis X-X of the eye and can illuminate an LCD panel mounted to the intracorneal implant 1820, so as to project an image displayed by the LCD panel onto the retina of the eye (e.g., via an optical transmission path which may comprise the lens of the eye and/or one or more focusing optic of the present disclosure). In some embodiments, primary inductive coil 1808a may be mounted, e.g., in the surrounding area of illumination source 1813.

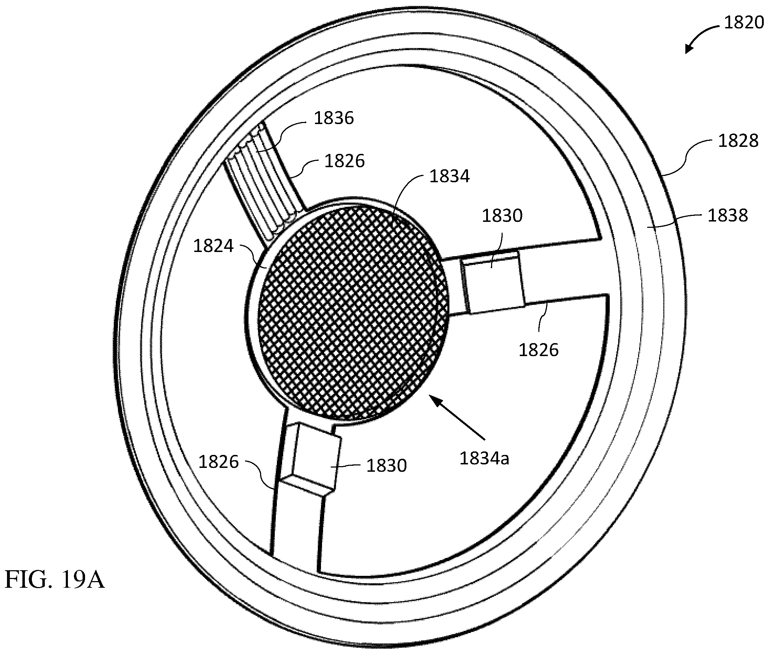

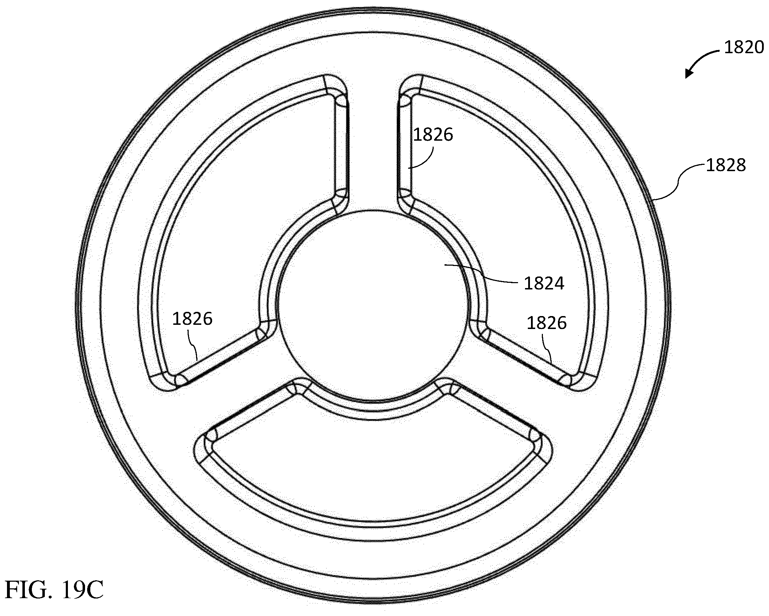

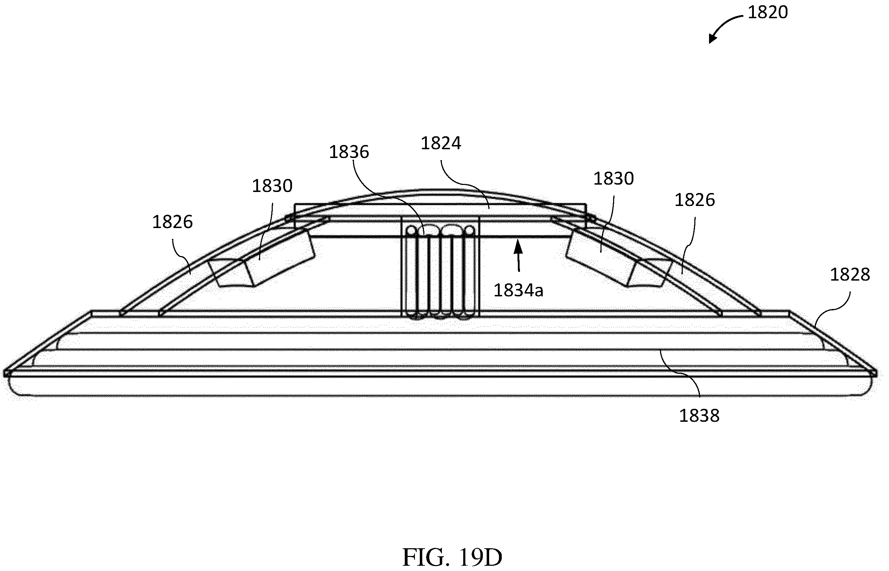

Figs. 19A-19D show front perspective (19A), posterior (19B), anterior (19C), and side (19D) views, respectively, of an exemplary intracorneal implant 1820, according to some embodiments of the present disclosure.

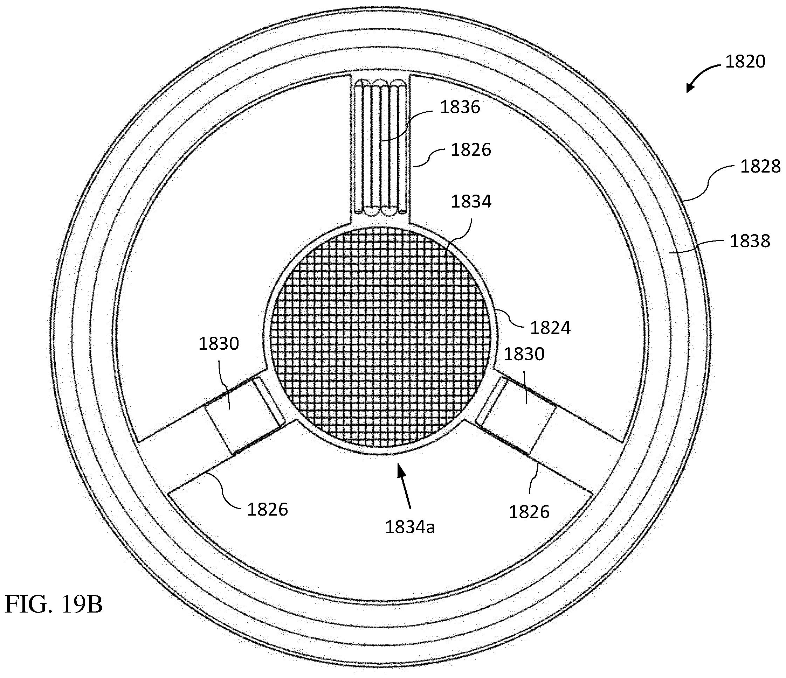

In some embodiments, intracorneal implant 1820 defines a planar body having a convexity which substantially conforms to the convexity of the cornea, with opposing concave posterior face (facing the retina of the eye, as shown in Fig. 19A) and convex anterior face (facing the anterior surface of the eye, as shown in Fig. 19B).

In some embodiments, intracorneal implant 1820 comprises an inner platform portion 1824 located about an axis of circular symmetry of the implant, and a peripheral portion, comprising one or more angularly-spaced arms 1826 that extend laterally from a peripheral edge of inner platform 1824. In some embodiments, the inner platform portion 1824 comprises at least 1, 2, 3, 4, 5, or more arms 1826.

In some embodiments, intracorneal implant 1820 further comprises a peripheral skirt portion 1828 which defines an outer circular perimeter of intracorneal implant 1820, wherein the one or more arms 1826 connect between inner platform 1824 and peripheral skirt portion 1828.

In some embodiments, intracorneal implant 1820 may be dimensioned and configured for surgical implantation within the cornea of an eye, traversing or completely replacing any opaque tissue layers of the cornea, and wherein only substantially transparent corneal tissue is present posterior to the device.

In some embodiments, intracorneal implant 1820 is dimensioned and configured for surgical implantation within the cornea of an eye, such that a layer of corneal tissue that is partially transparent, partially semi-transparent, and/or partially opaque is present posteriorly to the light-projecting posterior port of the device.

In some embodiments, exemplary intracorneal implant 1820 is dimensioned to fit within a cornea, posteriorly to any opaque corneal tissue layers, and to generate images that are detectable by a healthy retina or a healthy portion of a retina of the eye. For example, intracorneal implant 1820 may have an overall span between opposing edges thereof of, e.g., 6- 12mm (e.g., 9mm), an overall planar thickness of between 0.02- 1.50mm, and a convexity having a curvature of between 32-47 diopters.

In some embodiments, intracorneal implant 1820 may be fashioned of any suitable biologically-compatible material, e.g., any metal, metal alloy (such as nitinol, stainless steel, and the like), and/or polymer. In some embodiments, intracorneal implant 1820 may be fashioned integrally as a unitary intracorneal implant of a single material, or may be assembled from two or more separate components, wherein each component may be made of a different material. In some embodiments, intracorneal implant 1820 or any portion thereof may be made of a hard or non-deformable material, a shape-memory material, and/or a flexible or deformable material. In some embodiments, intracorneal implant 1820 or any portion thereof may have a coating of, e.g., an epoxy material, and the like.

In some embodiments, intracorneal implant 1820 is shaped to define one or more openings, e.g., as defined by the spaces between peripheral skirt portion 1828, inner platform portion 1824, and the one or more arms 1826. In some embodiments, the one or more openings are configured to allow adaptation of the intracorneal implant 1820 within the cornea through the openings and fixation of the intracorneal implant 1820 within the surrounding corneal tissue. In this manner, once intracorneal implant 1820 has been implanted, and the eye has healed, intracorneal implant 1820 becomes substantially anchored in place by the growth and remodeling of surrounding tissue. In some embodiments, one or more openings define a total opening surface area that is between, e.g., 30-50% of the total intracorneal implant 1820 surface area.

In some embodiments, image generator 1834 may be dimensioned and configured to be coupled to inner platform portion 1824, so as to display and/or project through a lightprojecting posterior port 1834a an image in a posterior direction in relation to a surface of the eye, towards and onto the retina of the eye (e.g., via an optical transmission path which may comprise the lens of the eye and/or one or more focusing optic of the present disclosure).

In some embodiments, image projection controller 1830 receives an input image data received by wireless receiver module 1836 and optionally stores the image data in a memory storage module. Image projection controller 1830 processes the digital image data encoded in the signal, to generate and reconstruct a visual image therefrom, and operates image generator 1834 to generate a light beam based on the image data, and to project the generated light beam onto the retina. In some embodiments, image projection controller 1830 may be implemented as one or more circuitry modules coupled to any part of intracorneal implant 1820, for example, to one or more posterior surfaces of arms 1826, as shown in Figs. 19A and 19B.

In some embodiments, image generator 1834 is configured and operable to receive an image data signal from an extraocular source and to generate an image projection light beam (e.g., light beam modulated with visual image information) to be projected on the retina of the eye through light-projecting posterior port 1834a, for generating an image thereon. In some embodiments, image generator 1834 is configured to output an image projection light beam capable of traversing through any transparent, semi-opaque, and/or substantially opaque corneal tissue that is present posteriorly to the light-projecting posterior port of the device, to reach the retina of the eye.

In some embodiments, image generator 1834 includes a display element connected to image projection controller 1830. Image generator 1834 can generate and project an image projection light beam onto the retina of the eye. In some embodiments, image generator 1834 may comprise, e.g., a color or monochrome liquid crystal display (LCD), and a backlight source.

In other embodiments, image generator 1834 comprises a micro-optical array mounted on a flexible printed circuit board ("PCB") substrate to support the electronic and optical components. The micro-display may comprise miniaturized pixels each with a size within a range, e.g., from 2 microns to 100 microns. The micro-display can be coupled to and supported by inner platform portion 1824 of intracorneal implant 1820. In some embodiments, the micro-display may be monochromatic or polychromatic. In some embodiments, the micro-display forms an array of pixels, characterized by a pixel size and a pixel pitch. As described herein, pixel sizes may range from 2 microns to 100 microns, and the pixel pitch may range from 10 microns to 1.0 mm, for example.

In some embodiments, image generator 1834 may be configured to receive illumination from an extraocular unit of the present disclosure, e.g., exemplary extraocular unit 1801 in Fig. 18B and 1802 in Fig 18C. In such embodiments, the extraocular unit may be configured to provide the illumination source for an LCD panel of image generator 1834.

Optical assembly 1840 may be mounted posteriorly to image generator 1834 and optically coupled therewith, to focus or apply any other suitable or desirable optical manipulation to the image projection light beam generate and projected by image generator 1834. Optical assembly 1840 may include, e.g., an adjustable imaging lens, which may act like the crystalline lens of a normal healthy eye, which focuses the images transmitted through the cornea onto the retina at the back of the eye.

In some embodiments, wireless receiver or transceiver 1836 may be implemented as electric circuitry coupled to any part of intracorneal implant 1820, for example, to one or more posterior surfaces of arms 1826, as shown in Figs. 19A and 19B. Wireless receiver or transceiver 1836 may be configured to be in wireless data communication with wireless transmitter or transceiver 1806 of extraocular unit 1800, to receive an image data signal transmitted by transmitter or transceiver 1806 using non-optical data transmission modalities, e.g., any suitable short-range RF transmission protocol, such as Bluetooth, ZigBee, and the like.

In some embodiments, a power receiving element 1838 may be implemented as a secondary reception inductive coil, mounted onto intracorneal implant 1820, e.g., disposed about a posterior surface of peripheral skirt portion 1828, and/or at any other suitable location. In some embodiments, power receiving element 1838 comprises an inductive coil made from wound wire. Alternatively, the secondary reception inductive coil may be made from a thin film polymer sandwich with wire traces deposited between layers of thin film polymer.

In some embodiments, the functions of wireless data reception and wireless power reception may both be carried out by the wireless power receiving element 1838, wherein the secondary reception inductive coil 1838 may be configured for joint reception of power and data.

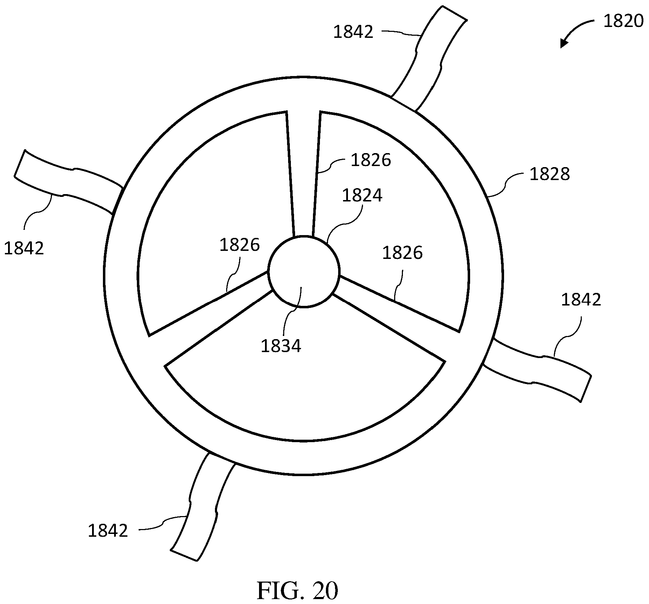

Fig. 20 is a schematic illustration of an exemplary intracorneal implant 1820 comprising a plurality of haptics 1842. In some embodiments, an intracorneal implant 1820 of the present disclosure may further comprise one or more haptics 1842 that extend laterally from a peripheral edge of the implant. The haptics may function structurally to center the intracorneal implant within the pocket formed in the cornea during surgery.

The haptics 1842 extend outward from peripheral skirt portion 1828 of intracorneal implant 1820, and are spaced apart from each other. Each of haptics 1842 includes a proximal end which is connected to the outer peripheral edge of peripheral skirt portion 1828, and a projecting segment culminating in a distal end.

In some embodiments, between 2 and 6 haptics 1842 may be used. In some embodiments, the haptics 1842 may be divided into opposing pairs, each of which extends outwardly and away from peripheral skirt portion 1828 at opposite ends thereof.

During implantation of intracorneal implant 1820, each of the haptics 1842 may be manipulated so as to center the intracorneal implant 1820 as close to the optical axis of the eye as possible, as well as to ensure that the intracorneal implant 1820 has the desired placement in the rotational direction in the X-Y plane, and secure it in place inside the cornea.

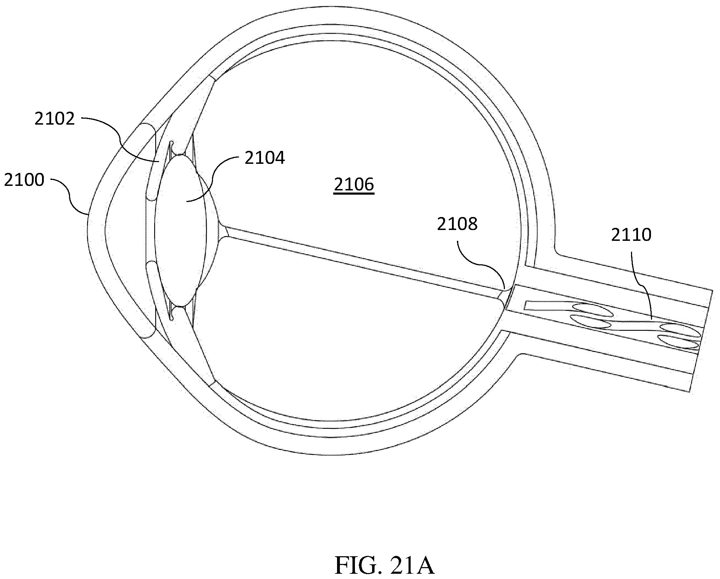

Figs. 21A-21D illustrate steps in the process for surgical implantation of an intracorneal implant 1820 within the cornea 2100.

In some embodiments, an intracorneal implant of the present disclosure may be configured for surgical implantation within the cornea using any suitable surgical method, e.g., by:
- Creating a partial anterior corneal stromal flap or lamella, performing deep lamellar dissection to create a recess, and positioning the implant in a recess beneath the flap or lamella.
- Creating a partial anterior corneal stroma flap or lamella, forming a hollow pocket within the cornea, positioning the implant within the pocket and securing the implant with the corneal flap or lamella.
- Creating a partial anterior corneal stroma flap or lamella, forming a hollow pocket within the cornea, positioning an implant which is pre-assembled preoperatively or assembled intraoperatively within a corneal tissue scaffold; and securing the implant with the corneal flap or lamella. The corneal tissue scaffold may be the exemplary corneal tissue scaffold 1329 described with reference to Figs. 13A-13F.
- Conjunctival flap surgery or mucous membrane grafting to permanently cover the cornea may also be performed concurrently or sequentially after surgical implantation.

Fig. 21A illustrates the human eye. Shown are the cornea 2100, iris 2102, lens 2104, vitreous region 2106, retina 2108, and optical nerve 2110.

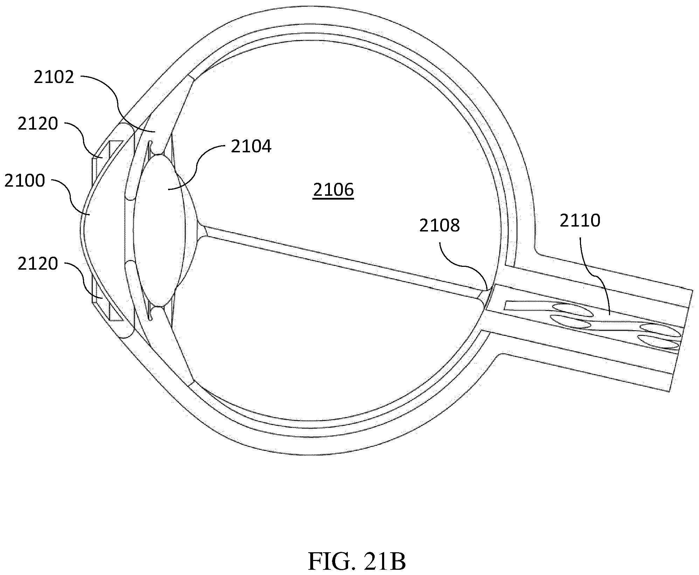

Fig. 21B shows a pocket 2120 formed in the cornea 2100, configured to receive an intracorneal implant 1820 of the present disclosure. In some embodiments, pocket 2120 may be formed using any suitable surgical method, e.g., by creating a partial anterior corneal stromal flap or lamella, performing deep lamellar dissection to create pocket 2120, and positioning intracorneal implant 1820 in pocket 2120 beneath the flap or lamella. In other embodiments, pocket 2120 may be formed by creating a partial anterior corneal stroma flap or lamella, forming a hollow pocket within the cornea, positioning intracorneal implant 1820 within the pocket, and securing intracorneal implant 1820 with the corneal flap or lamella.

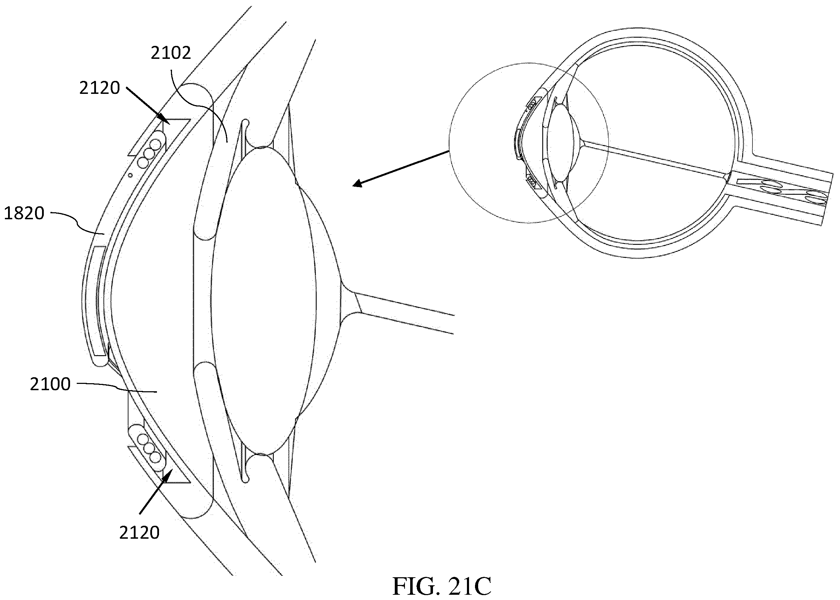

Fig. 21C shows intracorneal implant 1820 implanted within a pocket 2120 within cornea 2100.

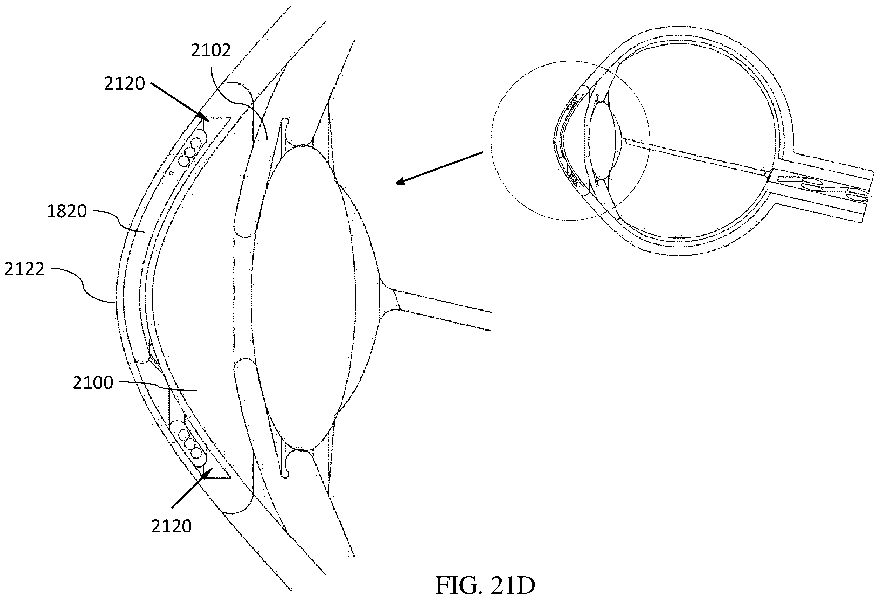

Fig. 21D shows a corneal lamella 2122 placed and sutured over the pocket 2120, to complete the implantation process. In some embodiments, conjunctival flap surgery or mucous membrane grafting to permanently cover the cornea may also be performed concurrently or sequentially after surgical implantation of intracorneal implant 1820 within pocket 2120.

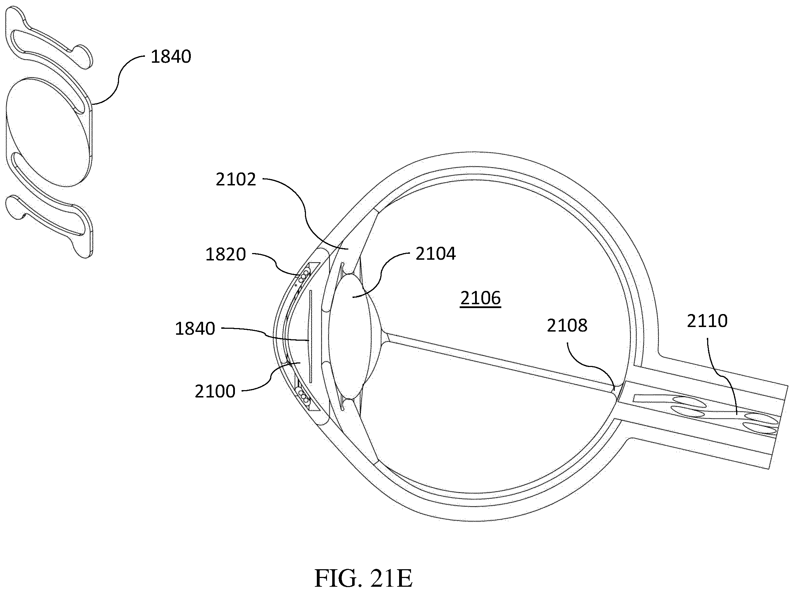

Fig. 21E shows optional optical assembly 1840 in detail and as implanted within the eye. Optical assembly 1840 may be mounted posteriorly of intracorneal implant 1820, and optically coupled therewith, to focus the projected image onto the retina. Optical assembly 1840 may comprise any suitable one or more optical lenses or other optical elements. In some embodiments, optical assembly 1840 may be configured to focus the projected optical image onto the retina, e.g., in conjunction with, or in the absence of, the natural lens of the eye 2104. In some embodiments, optical assembly 1840 may include, e.g., an adjustable imaging lens, which may act like the crystalline lens of a normal healthy eye, which focuses the images transmitted through the cornea onto the retina at the back of the eye.

Fig. 22 shows a cross-sectional view of an eye having an intracorneal implant 1820 implanted within cornea 2100. As can be seen, image generator 1834 is configured to project images onto retina 2108 (e.g., via an optical transmission path which may comprise the lens of the eye and/or one or more optical assembly of the present disclosure).

The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

In the description and claims, each of the terms "substantially," "essentially," and forms thereof, when describing a numerical value, means up to a 20% deviation (namely, ±20%) from that value. Similarly, when such a term describes a numerical range, it means up to a 20% broader range - 10% over that explicit range and 10% below it).

In the description, any given numerical range should be considered to have specifically disclosed all the possible subranges as well as individual numerical values within that range, such that each such subrange and individual numerical value constitutes an embodiment of the invention. This applies regardless of the breadth of the range. For example, description of a range of integers from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1 , 4, and 6. Similarly, description of a range of fractions, for example from 0.6 to 1.1 , should be considered to have specifically disclosed subranges such as from 0.6 to 0.9, from 0.7 to 1.1, from 0.9 to 1, from 0.8 to 0.9, from 0.6 to 1.1, from 1 to 1.1 etc., as well as individual numbers within that range, for example 0.7, 1, and 1.1.

The descriptions of the various embodiments of the present invention have been presented for purposes of illustration, but are not intended to be exhaustive or limited to the explicit descriptions. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

In the description and claims of the application, each of the words "comprise," "include," and "have," as well as forms thereof, are not necessarily limited to members in a list with which the words may be associated.

Where there are inconsistencies between the description and any document incorporated by reference or otherwise relied upon, it is intended that the present description controls.

## Claims

1. A device comprising: an intracorneal implant dimensioned and adapted for implantation within the cornea of an eye, said intracorneal implant defining a planar body having a convexity which substantially conforms to the convexity of the cornea, said intracorneal implant comprising:
i. a central optical part comprising an optical stem having an anterior end and extending axially through the cornea to a posterior end, and
ii. a peripheral haptic part comprising two or more angularly-spaced haptics that extend laterally from said central optical part, wherein, when said intracorneal implant is implanted within said cornea, said anterior end is located substantially at the anterior surface of said eye, and said posterior end abuts or is substantially in contact engagement with a thin layer of corneal tissue located posteriorly to said posterior end.

2. The device of claim 1, wherein said optical stem is configured to project an image in a posterior direction, through said thin layer of corneal tissue, and onto the retina of said eye.

3. The device of claim 2, wherein said anterior end is a light-receiving end, and wherein said optical stem defines a light-transparent pathway oriented for transmitting light received at said anterior end from an extraocular light source through said optical stem and through said thin layer of corneal tissue, onto the retina of said eye.

4. The device of claim 2, wherein said optical stem includes an image generator configured to receive an image data signal from an extraocular source, and to project an image in a posterior direction, through said thin layer of corneal tissue, onto the retina of said eye.

5. The device of any one of claims 1-4, wherein said optical stem comprises at least one optical lens.

6. The device of any one of claim 1-5, wherein said thin layer of corneal tissue is substantially transparent.

7. The device of any one of claim 1-6, wherein said thin layer of corneal tissue has a thickness of approximately 10-50µm.

8. The device of any one of claim 1-7, wherein said thin layer of corneal tissue comprises, in at least a central portion thereof, the pre-Descemet layer and the Descemet membrane of said cornea.

9. The device of any one of claim 1-7, wherein said thin layer of corneal tissue further comprises, in at least said central portion thereof, the Descemet membrane of said cornea.

10. The device of any one of claim 8 or 9, wherein said thin layer of corneal tissue further comprises, in at least said central portion thereof, the endothelial layer of said cornea.

11. The device of any one of claim 1-10, wherein said thin layer of corneal tissue is part of a corneal tissue scaffold configured for surgical implantation in said eye within a through- recess formed by a full-thickness corneal trephination in said eye.

12. The device of claim 11, wherein said thin layer of corneal tissue forms a central, substantially transparent, region of said corneal tissue scaffold.

13. The device of any one of claims 11-12, wherein said corneal tissue scaffold is preprocessed using at least one of the following techniques: dehydration, lyophilization, plasticization, gelling, dehydrothermal treatment, mechanical processing, compressing, and sterilization.

14. The device of any one of claims 1-13, wherein each of said haptics comprises at least one opening configured to facilitate fixation and adaptation of the device with the corneal tissue through the at least one opening, when said device is implanted within said cornea.

15. The device of any one of claims 1-14, wherein, when said intracorneal implant is implanted within said cornea, at least a portion of said anterior end is exposed through the anterior surface of said eye.

16. The device of claim 15, wherein said anterior end is configured for mounting at least one external optical element.

17. The device of claim 16, wherein said at least one external optical element is removably mounted to said anterior end.

18. A device comprising:
- a corneal implant dimensioned and adapted for implantation within the cornea of an eye, said implant defining a planar body comprising:
i. an inner platform portion located about an axis of circular symmetry of said implant, and
ii. a peripheral portion comprising one or more angularly-spaced arms that extend laterally from a peripheral edge of said inner platform;
- a wireless communication circuit configured for receiving an image data signal from an extraocular unit;
- an image projection controller configured to process said received image data signal to generate an image; and
- an image generator mounted to said inner platform portion, configured to project an image projection light beam, based on said generated image, in a posterior direction onto the retina of the eye.

19. The device of claim 18, further comprising a peripheral skirt portion which defines an outer circular perimeter of said implant, wherein said one or more angularly-spaced arms connect between said inner platform and said peripheral skirt portion;

20. The device of any one of claims 18 or 19, wherein said wireless communication circuit comprises a reception inductive coil.

21. The device of claim 20, wherein said reception inductive coil is further configured for receiving power inductively from said extraocular unit.

22. The device of any one of claims 18 or 19, wherein said wireless communication circuit comprises a photovoltaic cell configured to receive a light beam encoding said image data signal.

23. The device of claim 22, wherein said photovoltaic cell is further configured for receiving power from said light beam.

24. The device of any one of claim 18 to 23, further comprising a memory storage for storing said received image data signal.

25. The device of any one of claim 18 to 24, wherein said image generator comprises a liquid crystal display device having an illuminator element optically coupled thereto.

26. The device of any one of claim 18 to 25, wherein said image generator comprises a liquid crystal display device configured for being illuminated by an extraocular illumination source.

27. The device of any one of claim 18 to 26, wherein said peripheral skirt portion, inner platform portion, and at least one arm, define at least one opening in said planar body, and wherein said at least one opening is configured to facilitate fixation and adaptation of the device with the corneal tissue through the at least one opening, when said device is implanted within said cornea.

28. The device of any one of claim 18 to 27, further comprising an optical assembly mounted posteriorly of said image generator and optically coupled therewith, configured to focus said projected image onto said retina.

29. The device of any one of claim 18 to 28, further comprising at least one haptic that extends outwardly from said outer circular perimeter of said implant, wherein said at least one haptic comprises a proximal end which is connected to said outer circular perimeter, and a projecting segment culminating in a distal end.

30. A system comprising:
- a device comprising:
▪ a corneal implant dimensioned and adapted for implantation within the cornea of an eye, said implant defining a planar body comprising:
i. an inner platform portion located about an axis of circular symmetry of said implant, and
ii. a peripheral portion comprising one or more angularly-spaced arms that extend laterally from a peripheral edge of said inner platform,
∘ a wireless communication circuit configured for receiving an image data signal from an extraocular unit,
∘ an image projection controller configured to process said received image data signal to generate an image, and
∘ an image generator mounted to said inner platform portion, configured to project an image projection light beam, based on said generated image, in a posterior direction onto the retina of the eye; and
- an extraocular unit comprising:
∘ at least one imaging device for capturing visual images,
∘ an image processing module for processing and storing said captured visual images as digital image data, and
∘ a wireless communication module for transmitting said digital image data to said device through said wireless communication circuit.

31. The system of claim 30, wherein said device further comprises a peripheral skirt portion which defines an outer circular perimeter of said implant, wherein said one or more angularly-spaced arms connect between said inner platform and said peripheral skirt portion.

32. The system of any one of claims 30 or 31, wherein said wireless communication circuit comprises a reception inductive coil, and wherein said wireless communication module comprises a transmission inductive coil.

33. The system of claim 32, wherein said reception inductive coil is further configured for receiving power inductively from said transmission inductive coil.

34. The system of any one of claims 30 or 31, wherein said wireless communication module comprises a light beam source.

35. The system of claim 34, wherein said wireless communication circuit comprises a photovoltaic cell configured to receive a light beam encoding said image data signal, wherein said light beam is emitted from said light beam source.

36. The system of claim 35, wherein said photovoltaic cell is further configured for receiving power from said light beam.

37. The system of any one of claim 30 to 36, wherein said device comprises a memory storage for storing said received image data signal.

38. The system of any one of claim 30 to 37, wherein said image generator comprises a liquid crystal display device having an illuminator element optically coupled thereto.

39. The system of any one of claim 30 to 38, wherein said image generator comprises a liquid crystal display device, and wherein said extraocular unit comprises an illumination source configured to provide illumination to said liquid crystal display device.

40. The system of claim 39, wherein said illumination source is configured to emit sufficient illumination so as to traverse impaired or opaque layers and to reach said device, when it is implanted within said cornea.

41. The system of any one of claim 30 to 40, wherein said peripheral skirt portion, inner platform portion, and at least one arm, define at least one opening in said planar body, and wherein said at least one opening is configured to facilitate fixation and adaptation of the device with the corneal tissue through the at least one opening, when said device is implanted within said cornea.

42. The system of any one of claim 30 to 41, wherein said device comprises an optical assembly mounted posteriorly of said image generator and optically coupled therewith, configured to focus said projected image onto said retina.

43. The system of any one of claim 30 to 42, wherein said device comprises at least one haptic that extends outwardly from said outer circular perimeter of said implant, wherein said at least one haptic comprises a proximal end which is connected to said outer circular perimeter, and a projecting segment culminating in a distal end.

44. The system of any one of claim 30 to 43, wherein the components of said extraocular unit are mounted to one of: a spectacle frame, headwear, a helmet, and a headband.
